(19) Europäisches Patentamt
European Patent Office
Office européen des brevets

(11) EP 1 143 800 B1

(12) **EUROPEAN PATENT SPECIFICATION**

(45) Date of publication and mention of the grant of the patent: **02.08.2006 Bulletin 2006/31**

(21) Application number: **00901218.8**

(22) Date of filing: **24.01.2000**

(51) Int Cl.:
***A01N 63/00*** *(2006.01)* ***A01N 63/02*** *(2006.01)*
***C12N 15/31*** *(2006.01)* ***C12P 21/00*** *(2006.01)*
***C07K 14/24*** *(2006.01)* *C12P 21/00 (2006.01)*
*C12R 1/01 (2006.01)*

(86) International application number:
**PCT/GB2000/000219**

(87) International publication number:
**WO 2000/042855 (27.07.2000 Gazette 2000/30)**

(54) **BIOLOGICAL CONTROL OF NEMATODES**

BIOLOGISCHE BEKÄMPFUNG VON NEMATODEN

LUTTE BIOLOGIQUE CONTRE LES NEMATODES

(84) Designated Contracting States:
**AT BE CH CY DE DK ES FI FR GB GR IE IT LI LU MC NL PT SE**

(30) Priority: **22.01.1999 GB 9901499**

(43) Date of publication of application:
**17.10.2001 Bulletin 2001/42**

(73) Proprietor: **THE UNIVERSITY OF WARWICK**
**Coventry**
**CV4 8UW (GB)**

(72) Inventors:
- **MORGAN, James Alun Wynne**
  **Warwick,**
  **Warwickshire CV35 9EF (GB)**
- **JARRETT, Paul**
  **Warwick,**
  **Warwickshire CV35 9EF (GB)**
- **ELLIS, Debbie**
  **Warwick,**
  **Warwickshire CV35 9EF (GB)**
- **OUSLEY, Margaret Anne**
  **Warwick,**
  **Warwickshire CV35 9EF (GB)**

(74) Representative: **Lord, Hilton David et al**
**Marks & Clerk**
**90 Long Acre**
**London WC2E 9RA (GB)**

(56) References cited:
**WO-A-92/19739** **WO-A-98/08388**
**WO-A-99/22598** **WO-A-99/42589**
**WO-A1-95/00647** **WO-A1-97/17432**

- **DATABASE BIOSIS [Online] BIOSCIENCES INFORMATION SERVICE, PHILADELPHIA, PA, US AN 2000:109497, GREWAL, PARWINDER S. (1) ET AL: "Allelopathy: A possible mechanism of suppression of plant-parasitic nematodes by entomopathogenic nematodes." retrieved from STN XP002136524 & NEMATOLOGY, (NOV., 1999) VOL. 1, NO. 7-8, PP. 735-743. ,**
- **DATABASE BIOSIS [Online] BIOSCIENCES INFORMATION SERVICE, PHILADELPHIA, PA, US AN 2000:109391, HAN, RICHOU ET AL: "Trans - specific nematicidal activity of Photorhabdus luminescens." retrieved from STN XP002136525 & NEMATOLOGY, (NOV., 1999) VOL. 1, NO. 7-8, PP. 687-693. ,**
- **CHEMICAL ABSTRACTS, vol. 132, Columbus, Ohio, US; abstract no. 163304, HU, KAIJI ET AL: "Nematicidal metabolites produced by Photorhabdus luminescens (Enterobacteriaceae), bacterial symbiont of entomopathogenic nematodes" XP002136522 & NEMATOLOGY (1999), 1(5), 457-469 ,**
- **CHEMICAL ABSTRACTS, vol. 126, Columbus, Ohio, US; abstract no. 234686, GEORGIS, R. ET AL: "Novel pesticidal substances from the entomopathogenic nematode-bacterium complex" XP002136523 & ACS SYMP. SER. (1997), 658(PHYTOCHEMICALS FOR PEST CONTROL), 134-143 ,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 143 800 B1

- K. HU ET AL.: "Mortality of Plant-Parastic Nematodes Caused by Bacterial (Xenorhabdus spp. and Photorhabdus Luminescens) Culture Media" JOURNAL OF NEMATOLOGY, vol. 27, no. 4, December 1995 (1995-12), XP000905673
- PATENT ABSTRACTS OF JAPAN vol. 013, no. 286 (C-613), 29 June 1989 (1989-06-29) -& JP 01 080294 A (SUMITOMO CHEM CO LTD;OTHERS: 01), 27 March 1989 (1989-03-27)

## Description

TECHNICAL FIELD

The present invention relates to methods and materials for controlling nematodes.

PRIOR ART

Several thousand species of nematodes, sometimes called eel worms, are known. Numerous nematodes attack and parasitize humans and animals and cause disease. Additionally, several hundred species are known to feed on living plants. Certain of these are reviewed by Agrios in "Plant Pathology - 3rd Ed" Pub Academic Press Inc, see Chapter 15 therein.

Methods of controlling nematodes and their associated diseases include cultural practices; biological methods, e.g. use of resistant varieties; physical methods, e.g. heat; and use of chemical agents.

Patent application WO 92/19739 (Mycogen) relates to genes and gene fragments from *Bacillus thuringiensis* which have nematocidal activity. These generally encode crystal toxins from particular strains.

Patent application EP 0 303 426 (Mycogen) also relates to strains of *B. thuringiensis* which have nematocidal activity.

Patent application EP 0 171 381 (Monsanto) relates to particular soil bacteria which are capable of proliferating in an environment which is infested with nematodes such as pseudomonads which colonise the surface of plant roots. The basis for the controlling activity appears to stem from glycosidase enzymes which are hypothesised to directly inhibit the nematodes.

Notwithstanding these disclosures, there is an ongoing requirement for materials which have nematocidal activity, for instance for use in crop protection or nematode-mediated disease control.

Patent application PCT/WO 99/22598 (University of Reading) publisbed 14 May 1999 claims a biopesticide for the control of insect pests or plant parasitic nematodes or both, which comprises as an effective agent a species of bacteria which is a symbiont of an entomopathogenic nematode.

The American Chemical Society Symposium Series, 1997, Vol. 658 (Phytorhemicals for Pest Control), pages 134 to 143 discusses the potential for using symbiotic bacteria of nematodes such as *Xenohabdus* and/or their metabolites to control plant-parasitic nematodes. However, the major nematicidal compound of the culture filtrate, as detected by vibration, neutralisation and infrared spectroscopy of the corresponding salt, was ammonia.

The Journal of Nematology, 1995, Vol. 27(4), pages 502 to 503 has a similar disclosure to that of the above citation. Both citations, therefore, disclose that the nematicidal activity was down to the production of ammonia.

JP-A-01080294 and WO 92/19739 relate to insecticidal and nematicidal toxins derived from *Bacillus thuringibnsis,* and genes encoding for such toxins.

DISCLOSURE OF THE INVENTION

The present inventors have established that species of bacteria which in nature are associated symbiotically with entomopathogenic nematodes, can in fact be utilised to control nematodes, and in preferred forms of the invention, to kill them. The bacterial themselves can be employed, or nematode control agents can be used which are derived from such bacteria. In one aspect of the invention, the present invention employs bacteria which are engineered and thus not naturally occurring, or nematode control agents which are derived from natural or non-natural bacteria.

It has been reported that certain bacterial species such as *Xenorhabdus* and *Photorhabdus* can be used to control insects, see e.g. PCT/WO 98/08388 of MAFF, PCT/WO 97/17432 of WARF, and PCT/WO 99/42589 of Novartis. An effect against nematodes had not previously been demonstrated

WO 98/08388 discloses insecticidal methods and compositions derived from *Xenohabdus.* Nucleic acids which code for toxins in the bacterial are described, but there is no disclosure of activity against plant-parasitic nematodes.

WO 95/00647 discloses protein toxins having insecticidal properties obtained from *Xenohabdus nematophilus* and *Xenohabdus beddingii.*

The symbiotic bacteria used in the present invention are isolatable from nematodes or the insects which the nematodes attack, and differ fundamentally in terms of life-style and activity from those soil bacteria such as *B. thuringiensis* or pseudomonads which have previously been suggested as being nematocidal.

Indeed, *prima facie,* it seems highly unlikely that nematode symbiotes might possess nematocidal activity. However, in the light of the present disclosure, a number of possible explanations for the observed activity can be tentatively proposed. Firstly, in order to protect a nutrient supply from a dead insect, the bacterial might produce anti-nematocides to prevent saprophytic nematodes gaining access. Alternatively, to become a symbiont, the bacterial strains may have once been pathogens of these nematodes and evolved towards a less hostile symbiotic relationship. The nematocidal activity may be an evolutionary throwback from the original pathogenic relationship, in which case it may be expected to be widely present amongst bacterial which have evolved in this way.

A first aspect of the present invention is the use of a bacterial nematode control agent derived from a strain naturally symbiotically associated with an entomopathogenic nematode to control a parasitic nematode in plants, characterised in that the nematode control agent has functional activity against the said parasitic nematode, and is a peptide.

As discussed in more detail below, the bacterial strains may be used in the methods of the present invention *per se,* or they may be used as a source of nematode control agent. The nematode control agent can be derived directly, or be prepared and utilised through recombinant DNA techniques, optionally via a host cell.

The target nematode will generally be different to the nematode with which the bacterial strain is found symbiotically in nature.

By means of the present invention employing bacteria or a nematode control agent, it becomes possible to control nematodes, in the sense of, to prevent or retard the effect that the nematode has on other organisms such as animals or more preferably plants, or to reduce the number of nematodes or nematode eggs in an area of interest, or to alleviate or cure a disease caused by nematodes. Control may be at the level of larval nematodes or nematode eggs, or may inhibit the motion, feeding or infectivity of adult nematodes. Nematocidal control may be employed to kill the nematode target Such controlling activity can be assessed as shown in the Examples below.

PREFERRED EMBODIMENTS

The present invention provides the use of a nematode control agent derived from a bacterial strain naturally symbiotically associated with an entomopathogenic nematode to control a parasitic nematode in plants, characterised in that the nematode control agent has functional activity against the said parasitic nematode, and is a peptide.

Correspondingly, the present invention also provides the use, in the manufacture of a medicament for the control of a parasitic nematode, of a nematode control agent derived from a bacterial strain naturally symbiotically associated with an entomopathogenic nematode, characterised in that the nematode control agent has functional activity against the said parasitic nematode, and is a peptide.

The bacterial species is typically of the genera *Xenorhabdus* or *Photorhabdus,* preferably the genus *Xenorhabdus,* for instance the species *Xenorhabdus bovienii.* Examples of particularly preferred bacteria include:

> *Xenorhabdus bovienii* strain H31 deposited with NCIMB under accession number NCIMB 40985 on 20 January 1999;
> *Xenorhabdus bovienii* strain I73 deposited with NCIMB under accession number NCIMB 40986 on 05 November 1998; and
> *Xenorhabdus* strain C42 deposited with NCIMB under accession number NCIMB 41004 on 05 November 1998.

The present invention also provides a composition for the control of a parasitic nematode, wherein the bacterial species is any of the above strains, H31, I73 and/or C42.

The nematode control agent can be a peptide derived from a symbiont of an entomopathogenic nematode or an engineered bacterium has functional activity against a nematode. The peptide nematode control agent can be produced from a nucleic acid derived from a symbiont of an entomopath nematode or an engineered bacterium and which encodes such a peptide. The peptide can be an oligopeptide or a polypeptide, notably a protein. In one version, the nematode control agent is a toxin with toxic activity against nematodes, but the nematode control agent can have other activity.

The present invention also provides a host cell containing a vector, the vector comprising nucleic acid encoding a peptide.

The nucleic acids of this invention can be employed in a method of producing a peptide comprising the step of causing or allowing the expression from a nucleic acid of this invention in a suitable host cell.

The nucleic acid can comprise a natural nucleotide sequence or a degeneratively equivalent sequence, and functional variants thereof. Variants include homologous variants encoding a peptide which is a nematode control agent, the nucleic acid having 70% or more DNA sequence identity and/or the peptide having 70% or more amino acid sequence identity. Especially preferred nucleic acids in p 13-1f and p 14-2f and variants thereof.

The present invention extends to nucleic acids having a sequence which is a derivative by way of addition, insertion, deletion or substitution of one or more nucleotides. The nucleic acid can contain longer expressed sequences such that the nematode control agent is expressed as a fusion protein.

Nucleic acids complementary to the nucleic acid encoding a nematode control agent are envisaged.

The vector is preferably capable of replicating in a suitable host such as *E. coli* or in *Xenorhabdus.* The vector can be a baculoviros. In a preferred feature, the nucleic acid is operably linked to a promotor or other regulatory element for transcription in a host cell. Vectors can further comprise any one or more of the following: a terminator sequence; a polyadenylation sequence; an enhancer sequence; a marker gene; a sequence encoding pesticidal material derived from *Bacillus thuringiensis.*

The vector can be a plant vector.

The vector may be introduced into a cell. Thus, a method for transforming a plant cell comprises the step of causing or allowing recombination between the vector and the plant cell genome to introduce the nucleic acid into the genome. The nucleic acid can be incorporated into chloroplast DNA, or into mitochondrial DNA.

The host cell can be a plant cell, which may be in a plant.

To this end, a method for producing a transgenic plant comprises the step of regenerating a plant from the transformed cell. In turn, plants of this invention extend to the progeny of such plants.

Examples of plants of this invention include crop species which can be protected, notably maize, cotton, soya, rice, *Brassica* species, tomato, potato, sugar beet, barley, soybean, peanut, onion, rye, wheat, corn, banana, raspberry, bean. Decorative and other plants are also possible, e.g. rose.

A part of the propagule of the plants is also envisaged by this invention.

A method of influencing or affecting the toxicity of a cell such as a plant cell is provided where the method includes causing or allowing expression of a heterologous nucleic acid of this invention within the cells.

In a further aspect, the invention involves the use of a material selected from: an X. *bovienii* strain, a nematode control agent; a nucleic acid; a host cell; a plant; a peptide; or a composition of the invention, for the control of a pest, especially where the pest is a nematode and the material is used to control the nematode.

The present invention extends to control of helminthiasis in humans and other animals including domesticated animals such as swine, sheep, horses, cattle, goats, dogs, cats and poultry. The nematodes to be controlled include *Haemonchus, Trichostrongylus, Ostertagia, Nematodirus, Cooperia, Ascaris, Bunostomum, Oesophagosromuni, Chaberria, Trichuris, Strongylus, Trichonema, Dictyocaulus, Capillaria, Heterkis, Toxocara, Ascaridia, Oxyuris, Ancylostoma, Uncinaria, Toxascaris, Caenorhabditis* and *Parascaris.*

Target nematodes may be selected from the genera *Aphelenochoides, Anguina, Bursaphalenchus, Criconemella, Melodigyne, Ditylenchus, Globodera, Heliocotylenchus, Heterodera, Pratylenchus, Radopholus, Rotelynchus, Tylenchus, Trichodorus, Xiphenema,* and *Caenorhabditis.*

The compositions of this invention can be used in conjunction with *Bacillus thuringiensis* or pesticidal materials derived therefrom.

Some further aspects of preferred embodiments of the invention will now be discussed.

Bacterial strains

These can be derived from any entomopathogenic nematode. Preferred species are *Xenorhabdus* and *Photorhabdus.*

Potential sources of bacteria for use in the methods of the present invention may be identified by any preferred method. For instance, entomopathogenic nematodes can be isolated using an insect baiting technique such as that described by Bedding & Akhurst (1975) Nematologia 21: 215-227. Bacteria from nematodes identified as being pathogenic to the insect are isolated, cultured, and used as a source of nematocidal agent, e.g. by analogy with the methods used in the Examples below. Preferably *Xenorhabdus* or *Photorhabdus* species are used.

The preferred bacterial strains include ones which have the characteristics of strain C42, I73 or H31 isolated by the present inventors. This *Xenorhabdus* strain has the following characteristics: rod shaped; motile; non-bio luminescent; blue on NBTA; produces antibiotics; resistant to ampicillin; forms circular colonies; has convex morphology; white colour.

This strain was presumptively identified as belonging to the genera *Xenorhabdus* since it was isolated from an insect killed by an entomopathogenic nematode and had the above characteristics. The strain has been deposited at the NCIMB (23 St Machar Drive, Aberdeen, AB24 3RY, Scotland) by the applicants under accession number NCIMB 41004 on 20 January 1999.

Further preferred strains of the present invention are two strains of *X. bovienii* designated H31 and I73 which have also been deposited under the terms of the Budapest Treaty at the NCIMB under the accession numbers NCIMB 40985 and 40986 respectively. These share characteristics of C42 in that they are rod-shaped; motile; non-bioluminescent; blue on NBTA; produce antibiotics; resistant to ampicillin; form circular colonies; and have convex morphology. The strains were identified as belonging to the species *X. bovienii* when compared to the X. *bovienii* type strain T228 using Restriction Analysis of the complete 16S rRNA gene and partial sequence analysis.

Target nematodes and diseases

The group of diseases described generally as helminthiasis is due to infection of an human or other animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often at times serious infection in various species of animals. The most common genera of nematodes infecting the animals referred to above are *Haemonchus, Trichostrongylus, Ostertagia, Nematodirus, Cooperia, Ascaris, Bunostomum, Oesophagosromuni, Chaberria, Trichuris, Strongylus, Trichonema, Dictyocaulus, Capillaria, Heterakis, Toxocara, Ascaridia, Oxyuris, Ancylostoma, Uncinaria, Toxascaris, Caenorhabditis* and *Parascaris.* Certain of these, such as *Nematodirus, Cooperia,* and *Oesophagostomum,* attack primarily the intestinal tract, while others, such as *Dictyocaulus* are found in the lungs. Still other parasites may be located in other tissues and organs of the body.

The bacteria and encoded toxins of the invention may be used as nematocides for the control of the nematodes and diseases discussed above. More preferably, however, they are used to control soil and plant parasitic nematodes. Particular crop species which can be protected include tomatoes, potatoes, sugar beet, barley, soybean, peanut, onion, rye, wheat, corn, banana, raspberry, beans. Decorative and other plants may also be treated e.g. rose.

Target nematodes may be selected from the genera Aphelenochoides, *Anguina, Bursaphalenchus, Criconemella, Melodoigyne, Ditylenchus, Globodera, Helicotylenchus, Heterodera, Pratylenchus, Radopholus, Rotelynchus*, *Tylenchus, Trichodorus, Xiphenema.* A further organism used in certain of the Examples below is *Caenorhabditis elegans.* Other target organisms and plants are discussed by Agrios in "Plant Pathology - 3rd Ed" Pub Academic Press Inc, see Chapter 15 therein.

As stated above, the target nematode will generally be different to that with which the bacterial strain is found in nature.

Methods of use of bacteria

The bacteria may be used in any appropriate method which brings them into contact with the target nematode, preferably such that they, or their products, are ingested or absorbed by the target nematode.

In particular, regarding plants, the bacteria may be formulated in a variety of ways so as to enhance stability. For instance they may be employed in admixture with substrates to protect the cells.

The mixture can be spread over, ploughed into or otherwise mixed with nematode infected or potentially infected soil.

Regarding animals, bacteria intended for enteric inoculation can be mixed with carrier material that is suitable for ingestion by the intended animals.

Isolation of agent

Nematode control agents of the present invention, which may be proteinaceous, or nucleic acids encoding them, may be isolated and/or purified from the C42, I73 or H31 bacteria described above, in substantially pure or homogeneous form, or free or substantially free of other materials from the bacterial strain of origin. Where used herein, the term "isolated" encompasses all of these possibilities.

Methods of purifying proteins from heterogenous mixtures are well known in the art, e.g. selective precipitation, proteolysis, ultrafiltration with known molecular weight cut-off filters, ion-exchange chromatography, gel filtration, etc. A particularly useful initial technique in this regard is ultracentrifugation. Further methods which are known to be suitable for protein purification are disclosed in "Methods in Enzymology Vol 182 - Guide to Protein Purification" Ed. M P Deutscher, Pub. Academic Press Inc. Other references which outline techniques commonly used by those of ordinary skill in the art include "Protein Purification - principles and practice" Pub. Springer-Verlag, New York Inc (1982), and by Harris & Angal (1989) "Protein purification methods - a practical approach " Pub. O.U.P. UK.

Nematocidal activity may be assessed using a spread assay as discussed below.

The C42, I73 or H31 agent may be wholly or partially synthetic. In particular they may be recombinantly produced from nucleic acid sequences which are not found together in nature (do not run contiguously) but which have been ligated or otherwise combined artificially.

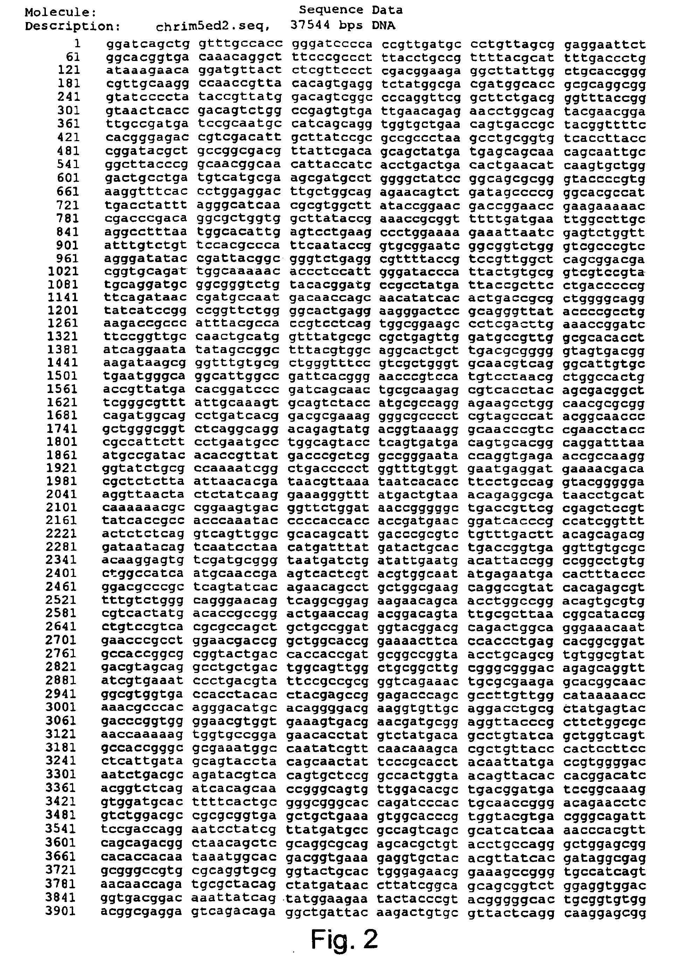

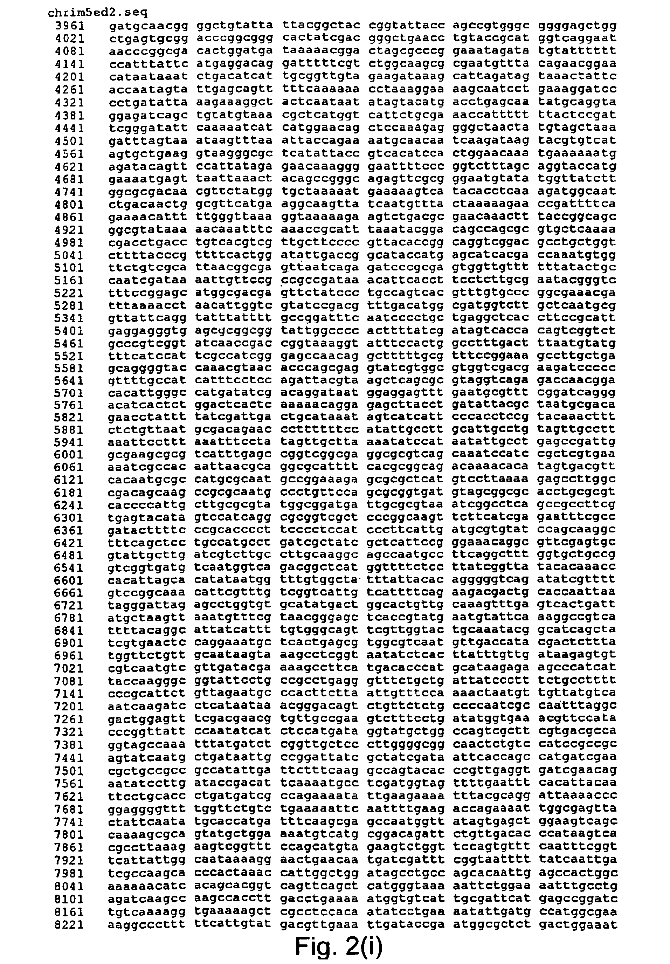

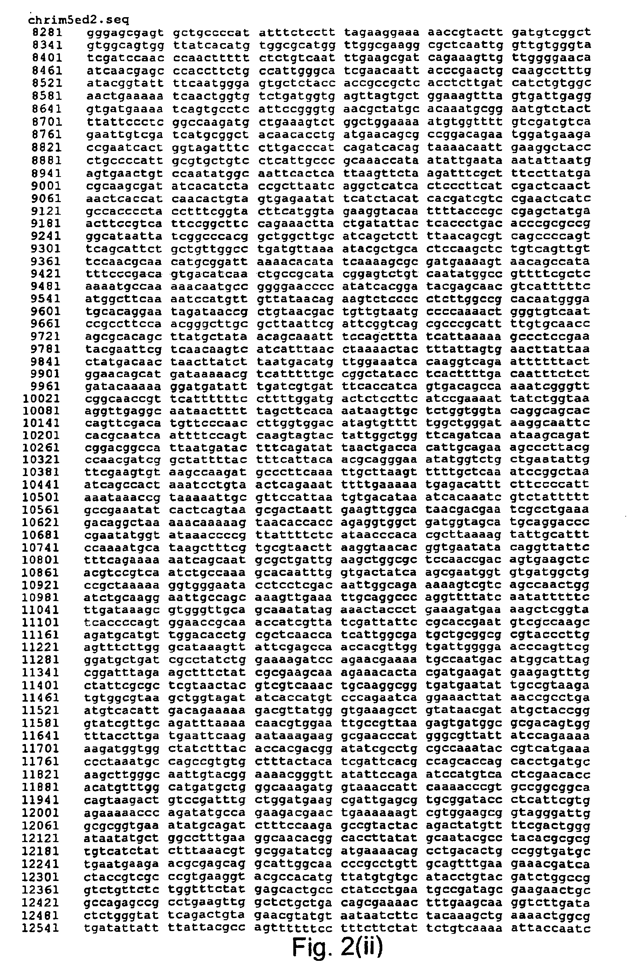

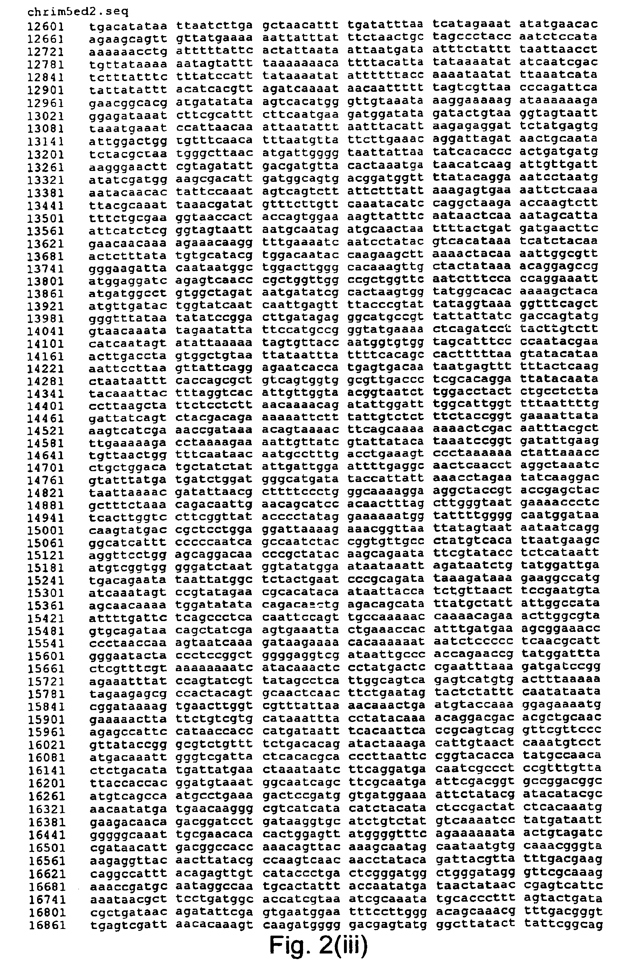

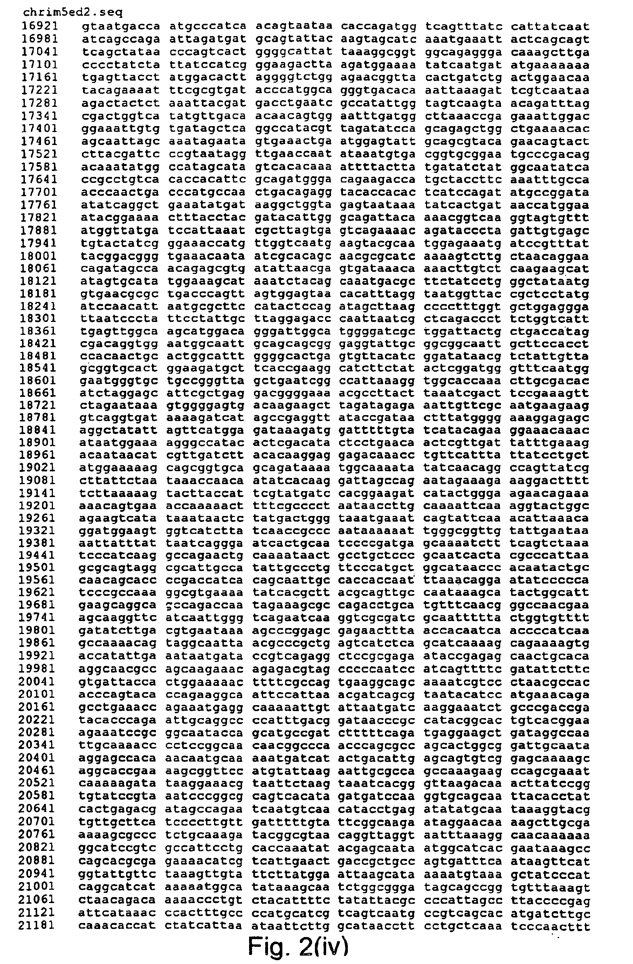

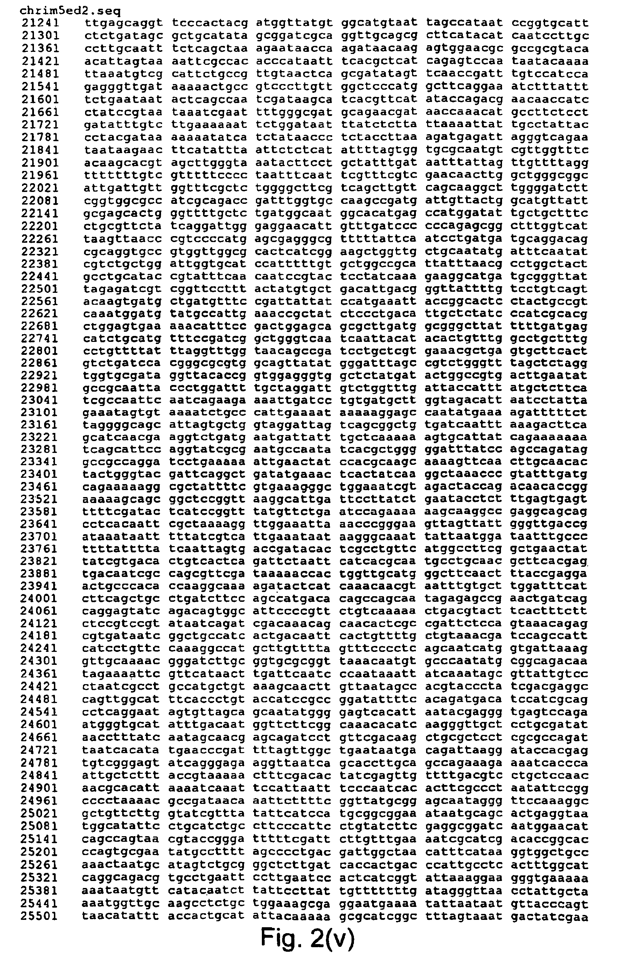

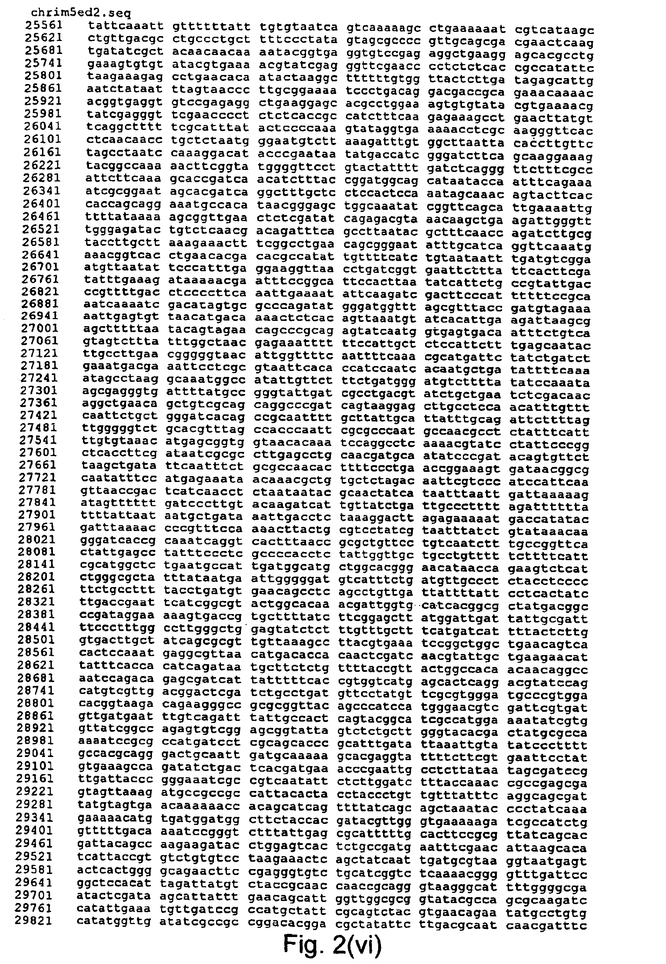

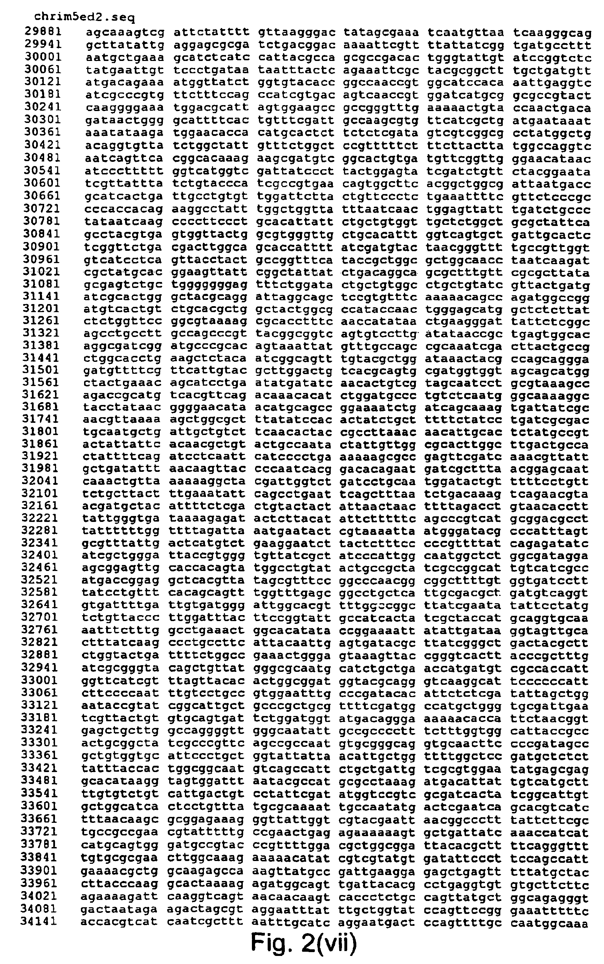

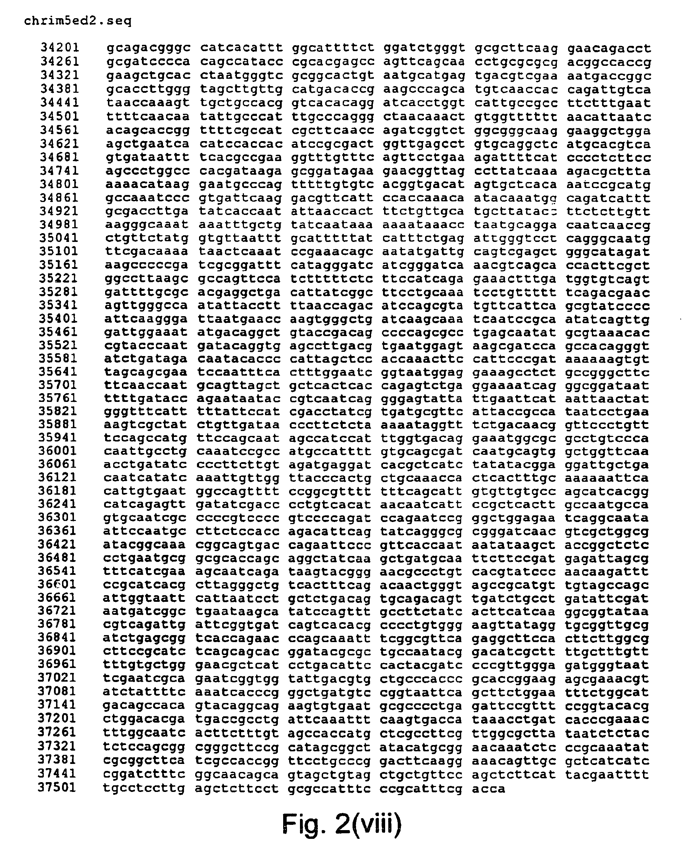

For instance, in the Examples below, nucleic acid encoding toxin(s) from I73 has been expressed in hosts cells using a vector system. Amino acid sequences of 38 different putative I73 toxin(s) are set out in sequence Annex 1. These sequences are based on the nucleic acid sequence set out in Fig 2 ('chrim5'), a cosmid clone derived from I73 genomic DNA which conferred nematocidal activity upon *E. coli* cells into which it was introduced (i.e. significantly reduced nematode larval growth and development, and feeding). As detailed below, the entire amino acid sequence as set out in each case may not be required for nematocidal activity. In particular the portion up to the first Met in each sequence may be omitted, as may other portions which may not contribute to the nematocidal activity. Thus, not all the proteins or genes may be required for nematocidal activity, and usually there will be one or more principal proteins, though others may play supporting roles such as in enhancing the activity or encoding other nematocidal activities.

Thus isolated nematocidal agents comprising a polypeptide containing all, or a nematocidal fragment, of any of the depicted I73 sequences, form one aspect of the present invention. Preferred agents include those encoded by p14-sf and p13-1f. Other active variants of these sequences are also encompassed as described below.

Candidate agents fur use in this invention to control nematodes extend to those from the bacteria described in PCT/WO 99/22598, as well as the insecticidal toxins and bacteria of PCT/WO 99/42589, PCT/WO 98/08388 and PCT WO 97/17432.

Nucleic acids and variants

In one aspect of the present invention there is provided a nucleic acid molecule encoding a nematode control agent of the present invention, for example a toxin, as described above, the nucleic acid being comprised within a vector contained in a host cell.

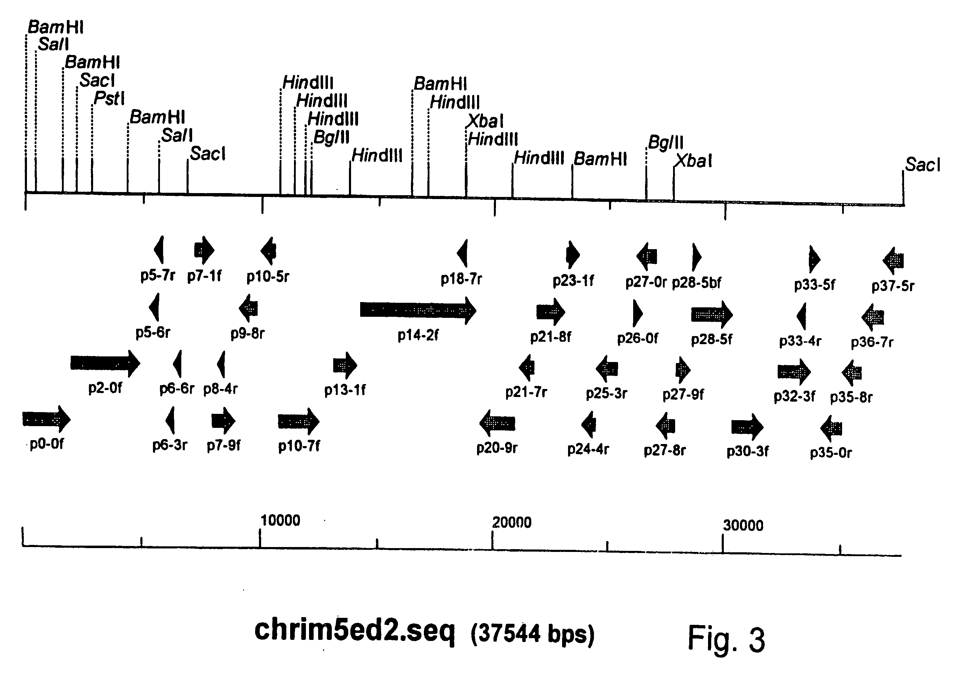

The nucleic acid may be derived from the sequence shown in Fig 2 or the complement (or degenerate equivalent) thereof. This sequence (cHRIM5) was itself derived from I73 and identified by its unexpected nematocidal activity. Regions of this sequence believed to correspond to genes of the present invention are described in Fig 3. Isolated nucleic acids comprising one or more of these regions which encode a nematocidal activity are particularly preferred.

In the light of the present disclosure, further nucleic acids of the present invention may be isolated using PCR or southern blotting or other techniques well known to those skilled in the art. This requires the use of two primers to specifically amplify target nucleic acid, so preferably two nucleic acid molecules with sequences characteristic of the C42, H31 or most preferably an I73 toxin isolated as above are employed. Using RACE PCR, only one such primer may be needed (see "PCR protocols; A Guide to Methods and Applications", Eds. Innis et al, Academic Press, New York, (1990)).

Thus a method involving use of PCR in obtaining nucleic acid according to the present invention may include:

(a) providing a preparation of bacterial nucleic acid,
(b) providing a pair of nucleic acid molecule primers suitable for PCR, at least one of said primers being a primer based on a toxin from C42, H31 or I73,
(c) contacting nucleic acid in said preparation with said primers under conditions for performance of PCR,
(d) performing PCR and determining the presence or absence of an amplified PCR product. The presence of an amplified PCR product may indicate identification of a variant.

In a further aspect of the present invention there are disclosed nucleic acids which are variants of the C42, I73 or H31 toxin. A variant nucleic acid molecule shares homology (or identity) with all or part of the C42, H31, or most preferably I73 sequence discussed above.

Preferably sequence comparisons are made using FASTA and FASTP (see Pearson & Lipman, 1988. Methods in Enzymology 183: 63-98). Parameters are set, using the default matrix blosum62, as follows:

Gapopen (penalty for the first residue in a gap): -12 for proteins / -16 for DNA
Gapext (penalty for additional residues in a gap): -2 for proteins / -4 for DNA KTUP word length: 2 for proteins / 6 for DNA.

Homology (similarity or identity) may be at the nucleotide sequence and/or encoded amino acid sequence level. Preferably, the nucleic acid and/or amino acid sequence shares at least about 70%, 75%, 80%, or 85% homology, most preferably at least about 90%, 95%, 96%, 97%, 98% or 99% homology.

Another method for assessing homology at the nucleic acid level is by hybridization screening. One common formula for calculating the stringency conditions required to achieve hybridisation between nucleic acid molecules of a specified sequence homology is shown in Molecular Cloning: a Laboratory Manual: 2nd edition, Sambrook et al, 1989, Cold Spring Harbor Laboratory Press:

$$T_m = 81.5°C + 16.6 \text{Log} [Na+] + 0.41 (\% G+C) - 0.63 (\% \text{formamide}) - 600/\#\text{bp in duplex}$$

As an illustration of the above formula, using [Na+] = [0.368] and 50-% formamide, with GC content of 42% and an average probe size of 200 bases, the Tm is 57°C. The Tm of a DNA duplex decreases by 1 - 1.5°C with every 1% decrease in homology. Thus, targets with greater than about 75% sequence identity would be observed using a hybridization temperature of 42°C. Such a sequence would be considered substantially homologous to the nucleic acid sequence of the present invention.

Variants of the present invention can be artificial nucleic acids.

Alternatively they may be novel, naturally occurring, nucleic acids, isolatable using the information disclosed herein. Thus a variant may be a distinctive part or fragment (however produced) corresponding to a portion of the C42, I73 or H31 toxin. The fragments may encode particular functional parts of the agent or they may be used for probing for, or amplifying, sequences corresponding to C42, I73 or H31 toxin. Sequence variants which occur naturally may include homologs of the C42, I73 or H31 toxin from other bacteria, including nematode-symbionts. Artificial variants (derivatives) may be prepared by those skilled in the art, for instance by site directed or random mutagenesis (i.e. nucleotide addition, deletion or substitution, optionally to lead to amino acid addition, deletion or substitution) or by direct synthesis. Preferably the variant nucleic acid is generated either directly or indirectly from an original nucleic acid encoding the C42, I73 or H31 toxin.

Changes may be desirable for a number of reasons, including introducing or removing the following features. Sites which are required for pre- or post-translation modification. Changes for codon usage preferences to enhance gene expression in different organisms. Leader or other targeting sequences (e.g. membrane or golgi locating sequences) may be added to the expressed protein to determine its location following expression. All of these may assist in efficiently cloning and expressing an active polypeptide in recombinant form. Other desirable mutation may be random or site directed mutagenesis in order to alter the activity (e.g. host specificity) or stability of the encoded polypeptide. Changes may be by way of conservative variation, i.e. substitution of one hydrophobic residue such as isoleucine, valine, leucine or methionine for another, or the substitution of one polar residue for another, such as arginine for lysine, glutamic for aspartic acid, or glutamine for asparagine. Also included are active (nematocidal) variants having non-conservative substitutions.

Variant nucleic acids encompass all of these possibilities. When used in the context of polypeptides or proteins they indicate the encoded expression product of the variant nucleic acid i.e. variants of C42, I73 or H31 toxin e.g. variants of the I73 toxin sequences disclosed hereinafter.

Vectors and production of host cells

The nucleic acid encoding the nematode control agent maybe provided in the form of a recombinant and preferably replicable vector.

Generally speaking, those skilled in the art are well able to construct vectors and design protocols for recombinant gene expression. Suitable vectors can be chosen or constructed, containing appropriate regulatory sequences, including promoter sequences, terminator fragments, polyadenylation sequences, enhancer sequences, marker genes and other sequences as appropriate. For further details see, for example, Sambrook et al (1989) *supra.*

The permitted vectors include, *inter alia,* any plasmid, cosmid, phage or *Agrobacterium* binary vector in double or single stranded linear or circular form which may or may not be self transmissible or mobilizable, and which can transform a prokaryotic or eukaryotic host either by integration into the cellular genome or exist extrachromosomally, e.g. an autonomous replicating plasmid with an origin of replication. Illustratively integration can occur into chloroplast DNA or into mitochondrial DNA.

Preferably the nucleic acid in the vector is under the control of, and operably linked to, an appropriate optionally inducible promoter or other regulatory elements for transcription in a host cell such as a microbial, e.g. bacterial, yeast, filamentous fungal or plant cell. The vector may be a bi-functional expression vector which functions in multiple hosts. In the case of genomic DNA, this may contain its own promoter or other regulatory elements and in the case of cDNA this may be under the control of an appropriate promoter or other regulatory elements for expression in the host cell. The vectors and host cells into which they are introduced may be used to clone or otherwise identify nucleic acids according to the invention.

The agent may be used as part of a viral vector which is itself pathogenic to nematodes.

Also of interest in the present context are nucleic acid constructs which operate as plant vectors. Specific procedures and vectors previously used with wide success upon plants are described by Guerineau and Mullineaux (1993) (Plant transformation and expression vectors. In: Plant Molecular Biology Labfax (Croy RRD ed) Oxford, BIOS Scientific Publishers, pp 121-148). Suitable vectors may include plant viral-derived vectors (see e.g. EP-A-194809). Suitable promoters which operate in plants include the Cauliflower Mosaic Virus 35S (CaMV 35S). Other examples are disclosed at page 120 of Lindsey & Jones (1989) "Plant Biotechnology in Agriculture" Pub. OU Press, Milton Keynes, UK.

Host cells

The toxin genes or gene fragments encoding the nematocidal agents of the subject invention may be introduced into a host cell, microbial, animal or plant. Expression of the toxin gene in the host cell results, directly or indirectly, in the intracellular production and maintenance of the nematocide.

Thus the present invention also provides methods comprising introduction of such a construct into a plant cell or a microbial cell and/or induction of expression of a construct within a cell, by application of a suitable stimulus e.g.

an effective exogenous inducer.

Hosts may be used to assay the activity of particular sequences or fragments. Hosts can also be used to generate quantities of toxin which can be employed in situ in suitable treated cells, or alternatively with suitable hosts, e.g., *Pseudomonas* viable microbes can be applied to the sites of nematodes where they will proliferate and where they or their products can be ingested by the nematodes. Higher organisms, preferably plants, can also be engineered with the toxin. The result in each case is a control of the nematodes. A host may be selected that can tolerate harsh environmental conditions and then grow when they improve, as illustrated by *Bacillus* species where the spores can exist under environmental extremes.

Characteristics of interest for use as a nematocide microcapsule i.e. a vehicle for the active agent include protective qualities for the nematocide, such as thick cell walls, pigmentation, and intracellular packaging or formation of inclusion bodies; leaf affinity; lack of mammalian toxicity; attractiveness to nematodes for ingestion; ease of killing and fixing without damage to the toxin; and the like.

Treated host cells

Where the cell is treated, the cell will usually be intact and be substantially proliferative form when treated, rather than in a spore form, although in some instances spores may be employed. Treatment of the microbial cell, e.g. a microbe containing the bacterial toxin gene or gene fragment, can be by chemical or physical means, or by a combination of chemical and/or physical means, so long as the technique does not deleteriously affect the properties of the toxin, nor diminish the cellular capability in protecting the toxin.

Viable hosts

Where the toxin gene or gene fragment is introduced via a suitable vector into a microbial host, and said host is applied to the environment in a living state, it is preferable that microorganism hosts are selected which are known to occupy the phytosphere (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the nematocide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, e.g., genera *Pseudomonas, Erwinia,* Serratia, *Klebsiella,* Xanthomonas, *Streptomyces, Rhizobium, Rhodopseudomonas,* Methylophilius, *Agrobacterium , Acetobacter, Lactobacillus, Arthrobacter, Azotobacter, Leuconosroc,* and *Alcaligenes;* fungi, particularly yeast, e.g., genera *Saccharomyces*, *Cryprococcus, Kluyveromyces, Sporobolomyces, Rhodororula,* and *Aureobasidium.*

Plants as hosts

Nucleic acid encoding the nematocides of the present invention can be introduced into plant cells using any suitable technology, such as a disarmed Ti-plasmid vector carried by *Agrobacterium* exploiting its natural gene transfer ability (EP-A-270355, EP-A-0116718, NAR 12(22) 8711 - 87215 1984), particle or microprojectile bombardment (US 5100792, EP-A-444882, EP-A-434616) microinjection (WO 92/09696, WO 94/00583, EP 331083, EP 175966, Green et al. (1987) Plant Tissue and Cell Culture, Academic Press), electroporation (EP 290395, WO 8706614 Gelvin Debeyser) other forms of direct DNA uptake (DE 4005152, WO 9012096, US 4684611), liposome mediated DNA uptake (e.g. Freeman et al. Plant Cell Physiol. 29: 1353 (1984)), or the vortexing method (e.g. Kindle, PNAS U.S.A. 87: 1228 (1990d). Physical methods for the transformation of plant cells are reviewed in Oard, 1991, Biotech. Adv. 9: 1-11.

*Agrobacterium* transformation is widely used by those skilled in the art to transform dicotyledonous species. It has also been used with filamentous fungi (see de Groot et al, 1998, Nature Biotechnology 16: 839-842).

Recently, there has also been substantial progress towards the routine production of stable, fertile transgenic plants in almost all economically relevant monocot plants (see e.g. Hiei et al. (1994) The Plant Journal 6, 271-282)). Microprojectile bombardment, electroporation and direct DNA uptake are preferred where *Agrobacterium* alone is inefficient or ineffective. Alternatively, a combination of different techniques may be employed to enhance the efficiency of the transformation process, e.g. bombardment with *Agrobacterium* coated microparticles (EP-A-486234) or microprojectile bombardment to induce wounding followed by co-cultivation with *Agrobacterium* (EP-A-486233).

Generally speaking, following transformation, a plant may be regenerated, e.g. from single cells, callus tissue or leaf discs, as is standard in the art. Almost any plant can be entirely regenerated from cells, tissues and organs of the plant. Available techniques are reviewed in Vasil et al., Cell Culture and Somatic Cell Genetics of Plants, Vol I, II and III, Laboratory Procedures and Their Applications, Academic Press, 1984, and Weissbach and Weissbach, Methods for Plant Molecular Biology, Academic Press, 1989.

The generation of fertile transgenic plants has been achieved in the cereals rice, maize, wheat, oat, and barley (reviewed in Shimamoto, K. (1994) Current Opinion in Biotechnology 5, 158-162.; Vasil, et al. (1992) Bio/Technology 10, 667-674; Vain et al., 1995, Biotechnology Advances 13 (4): 653-671; Vasil, 1996, Nature Biotechnology 14 page 702).

Combination nematocides

In further embodiments of the invention, bacteria associated with entomopathogenic nematodes or the toxins or products discussed above are used in conjunction with other nematocidal bacteria such as *B. thuringiensis* strains (e.g. from WO 92/19739) or pesticidal materials derived therefrom.

Materials for use in the present invention

The present invention also embraces materials for use in the methods above. These materials include the novel bacterial strains which are associated symbiotically with an entomopathogenic nematode and which are capable of controlling a target nematode. In particular the invention encompasses strain C42, I73 or H31 in isolated or substantially isolated form, or strains having the characteristics of C42, I73 or H31 (including nematocidal activity assessed as below).

Also embraced are compositions and formulations of these bacteria. These may comprise or consist of wettable powders, granules or dusts, mixed with various inert materials, such as inorganic minerals (phyllosilicates, carbonates, sulfates, phosphates, methylcellulose, xanthan gum and the like) or botanical materials (powdered corncobs, rice hulls, walnut shells, peat moss, vermiculite, soil, seeds, other plant tissue and the like). The formulations may include spreader-sticker adjutants, stabilizing agents or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

Bacteria may be mixed with other material while in freeze-dried form, encapsulated in biodegradable or water-soluble material, or otherwise treated to prolong their viability or decrease their levels of metabolic activity during handling. If desired, the carrier material may contain assimilatable nutrient sources to support proliferation of the bacteria.

Also included are purified or substantially purified nematocidal agents (particularly proteinaceous agents) isolated or isolatable from the strains or host cells discussed above.

Thus the invention further discloses nematocidal compositions comprising one or more agents as described above. Such compositions preferably further comprise other nematocidal materials from other *Xenorhabdus* species or non-*Xenorhabdus* species. These other materials may be chosen such as to have complementary properties to the agents described above, or act synergistically with it.

Toxins of the invention for use with animals can be adapted to be administered orally in a unit dosage form such as a capsule, bolus or tablet, or as a liquid drench when used as an anthelmintic in mammals, and in the soil to control plant nematodes. The drench is normally a solution, suspension or dispersion of the active ingredient, usually in water, together with a suspending agent such as bentonite and a wetting agent or like excipient. Generally, the drenches also contain an antifoaming agent. Drench formulations generally contain from about 0.001 to 0.5% by weight of the active compound. Preferred drench formulations may contain from 0.01 to 0.1% by weight, the capsules and boluses comprise the active ingredient admixed with a carrier vehicle such as starch, talc, magnesium stearate, or dicalcium phosphate. Where it is desired to administer the toxin compounds in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate, vegetable gums and the like. Such unit dosage formulations may be varied widely with respect to their total weight and content of the antiparasitic agent, depending upon the factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or, optionally, fed separately. Preferably, a carrier for feed administration is one that is, or may be, an ingredient of the animal ration. Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, crushed limestone and the like.

Alternatively, the antiparasitic compounds may be administered to animals parenterally, for example, by intraluminal, intramuscular, intratracheal, or subcutaneous injection, in which event the active ingredient is dissolved or dispersed in a liquid carrier vehicle. For parenteral administration, the active material is suitably admixed with an acceptable vehicle, preferably of the vegetable oil variety, such as peanut oil, cotton seed oil and the like. Other parenteral vehicles, such as organic preparations using solketal, glycerol, formal and aqueous parenteral formulations, are also used. The active compound or compounds are dissolved or suspended in the parenteral formulation for administration; such formulations generally contain from 0.005 to 5% by weight of the active compound.

Further aspects of the invention include nucleic acids, vectors and host cells containing a heterologous construct according to the present invention, especially a plant or a microbial cell.

Such microbial cells may be treated as described in the methods above. Examples of chemical reagents are halogenating agents. Other suitable techniques include treatment with aldehydes, such as formaldehyde and glutaraldehyde; anti-invectives, such as zephiran chloride and cetylpyridinium chloride; alcohols, such as isopropyl and ethanol; various histologic fixatives, such as Bouin's fixative and Helly's fixative (See: Humason, Gretchen L., Animal Tissue Techniques, W.H. Freeman and Company, 1967); or a combination of physical (heat) and chemical agents that preserve and prolong the activity of the toxin produced in the cell when the cell is administered to the host animal. The method of inactivation or killing retains at least a substantial portion of the bio-availability or bioactivity of the nematode control agent.

In all of the compositions discussed above, the nematocide concentration may vary widely depending upon the nature of the particular formulation, particularly whether it is a concentrate or to be used directly. The nematocide will be present in at least 1% by weight and may be 100% by weight. The dry formulations will have from about 1-95% by weight of the nematocide while the liquid formulations will generally be from about 16% by weight of the solids in the liquid phase. The formulations will generally have from about $10^2$ to about $10^{10}$ cells/mg, more preferably $10^7$ to about 109 cells/mg. These formulations will be administered at about 50 mg (liquid or dry) to 1 kg or more per hectare. The formulations can be applied to the environment of the nematodes, e.g., plants, soil or water, by spraying, dusting, sprinkling, or the like.

In addition to the above the invention includes plant cells which have been transformed with the genes of the present invention, and plants which include such plant cells.

EXAMPLES OF THE INVENTION

The invention will now be further described with reference to the following non-limiting Figures and Examples. Other embodiments of the invention will occur to those skilled in the art in the light of these.

FIGURES

Fig 1 shows the cHRIM5 cosmid vector and subclones used for sequencing, as described in Example 6.

Fig 2 shows the sequence of cHRIM5 (1-37544 bps).

Fig 3 shows the position and orientation of ORFs in the cHRIM5 sequence.

Fig 4 shows deletions of cHRIM5 tested for nematocidal activity.

Fig 5 illustrates cloning of nematocidal activity in PLEX.

<u>Example 1 - Source of strains C42, I73 and H31</u>

Strain C42 was obtained using an insect entrapment method. Insects which were killed on the surface of a soil sample were observed under a microscope at high magnification. Any that contained high numbers of bacteria and not fungal hyphae were presumed to have been killed by insect parasitic nematodes. The identified presence of nematodes also aids this identification step, but it is not essential. These samples were plated on to NBTA media (see Poinar & Thomas, 1984 Nematodes p238-280 in "Laboratory guide to insect pathogens and parasites" Eds. Poiner & Thomas, Pub. Plenum Press, New York). Any colonies that developed that had characteristic features (e.g. morphology, size, colour) of *Xenorhabdus* or *Photorhabdus* strains were selected. Non-luminescent colonies were presumptively identified as *Xenorhabdus.* The identity of those having nematocidal activity as assessed in Example 3, is further confirmed using 16s rRNA sequence data (see Brunel et al 1997, Applied and Environmental Microbiology 63,2: 574-580).

I73 and H31 strains were obtained in a similar way to strain C42 but they were identified as belonging to the species X. *bovienii* when compared to the *X. bovienii* type strain T228 using Restriction Analysis of the complete 16S rRNA gene (see Brunel et al, 1997 Applied and Environmental Microbiology: 574-580), and partial 16s ribosomal RNA sequence analysis.

Example 2 - Cell growth and preservation

Subcultures of the *Xenorhabdus* species C42, I73 and H31 were used to inoculate three 9 cm diameter petri dishes containing L agar (10g tryptone, 5 g Yeast Extract, 5 g NaCl and 15 g agar per 1t). Plates were incubated for 48 hrs at 26°C and the resulting growth harvested by scraping off bacterial cells and thoroughly resuspending in 40 mls of 5% w/v lactose. The cells were washed once by centrifugation (5000 x *g* for 10 mins), resuspended in 10 mls of 5% w/v lactose, dispensed into 1 ml aliquots and freeze dried (-60°C for 48 hrs) for medium term storage at 2°C. Other stocks were re-suspended in nutrient broth containing 10% w/v glycerol (Protect) and frozen at -70°C.

Example 3- Activity of cells against *Caenorhabditis elegans*

The bioassays were performed by allowing *C. elegans* to feed on live bacterial cell suspensions spread over the surface of Luria broth agar (Luria broth containing 1.2%w/v agar) in segmented square petri dishes (2.0 x 2.0 cm per test well). A minimum of three test wells, each containing 50-100 nematodes were used for each test. Mortalities were recorded after 3 days at 18°C.

*C. elegans* was cultured on *Escherichia coli* at 18°C on 9 cm diameter LB agar plates. Once the nematodes had colonised the complete plate they were re-subbed on a fresh plate to maintain stocks and the remainder re-suspended in 40 ml LB. The tube was allowed to stand for 15 min and the nematodes settled to the bottom. The concentrated nematodes were removed using a sterile pipette and placed in 40 mls of fresh LB. The process was repeated 5 more times to wash the nematodes away from the *E. coli* cells. The nematodes were then diluted so that approximately 50 nematodes were present in 50 μl of LB.

The *Xenorhabdus* cells used were cultured in LB at 30°C/ 100 rpm for 24 hours and 50 μl spread on to the surface of each test well. The control *E. coli* cells were treated in a similar way but incubated at 37°C for growth. After application the wells were air dried for 30 min, and 50 μl of the nematode suspension placed in each well. Again the wells were air dried for 30 min. Plates were incubated at 18°C with 80% relative humidity for 3 days.

*Xenorhabdus* spp. C42, H31 and I73 gave 95% mortality, as compared with no significant effect for certain other *Xenorhabdus* bacterial strains and *E. coli.* Thus these results clearly show that cells from *Xenorhabdus* C42, H31 and I73 are an effective nematocide.

Example 4 - Cloning of nematode active gene from I73

Total DNA was isolated from I73 using a Quiagen genomic DNA purification kit (cat no. 10243). To isolate DNA, cells were grown in Luria broth (10g tryptone, 5g yeast and 5g NaCl per 1t) at 26°C with shaking at 200 rpm to an optical density of 1.5 A600. Cells were harvested by centrifugation at 4000 x *g* and the DNA isolated using Quiagen 100/G tips, as per manufacturer's instructions. The purified DNA was stored at -20°C in TE buffer (10 mM Tris, 1 mM EDTA, pH 8.0).

To obtain a representative I73 library, total DNA was partially digested with *Sau*3A. Approximately 25 μg of DNA was incubated at 37°C with 0.25 units of enzyme. At intervals of 5, 15, 30, 45 and 60 minutes, samples were removed and heated at 65°C for 10 minutes. To determine the size of the resulting DNA fragments, the samples were separated on a 0.5% (w/v) agarose gel. The samples containing a dominant DNA fragment size of between 30 and 50 Kb were combined and treated with shrimp alkaline phosphatase (Boehringer) for 20 minutes at 37°C. The DNA was ligated into the *Bam*HI site of the Stratagene cosmid vector Supercos 1 (scos) and packaged into the *Escherichia coli* strain XL Blue 1, using a Gigapack II packaging kit (Stratagene) following the manufacturer's instructions.

To identify individual cosmid clones with activity to *C. elegans,* single colonies were grown in individual wells of segmented square petri dishes on Luria agar, containing 50 μg/ ml ampicillin at 30°C for 24 hours. To each well, approximately 50, mainly L4 and adult *C. elegans* larvae were added in 50 μl of Luria broth. The dishes were incubated at 18°C and examined after 6 days for nematode development.

A total of 600 clones were examined and one coded cHRIM5 was found, which caused significant reduction in larval numbers, with no live L4 and adult larvae observed compared to on average, greater than 40 in all other clones tested.

Example 5 - Activity of cHRIM5, C42, H31 and I73 against C. *elegans*

Clone cHRIM5 was grown in 50 mls LB containing 50 μg of ampicillin per ml at 30°C/200 rpm for 40 hours. C42, H31 and I73 were grown in 50 mls LB at 26°C for 48 hours/200 rpm. Cultures were centrifuged at 4000 x *g* for 10 minutes, washed once and resuspended in 5 mls of PBS (0.05 mM phosphate buffer, 0.125M NaCl). To determine

activity, 300 μl of cells were added in triplicate, to 1.2 ml of PBS containing 25, mainly L4 and adult *C. elegans* larvae in multi well square dishes. As a control, an equivalent amount of XL 1 Blue *E. coli* cells containing Supercos 1 were used to determine nematode survival. The assays were incubated at 18 °C for 7 days before approximate nematode counts and observations were made.

Activity of cells on *C. elegans*

| Cell line | No. and size of larvae/ square | Cell turbidity |
|---|---|---|
| XL 1 Blue/Supercos 1 | >100 (all stages) | Clear |
| XL 1 Blue/cHRIM5 | <20(mainly small, L1,2 &3) | Cloudy |
| C42 | <10 | Cloudy |
| H31 | < 10 | Cloudy |
| I73 | < 10 | Cloudy |

Thus cHRIM5, C42, H31 and I73 all gave a reduction in nematode numbers, and in particular cHRIM5 cells significantly reduced larval growth and development. All four strains caused a reduction in feeding (as indicated by the cloudy cell suspensions).

Example 6 - DNA and protein sequences

Plasmid and cosmid DNA for cloning was prepared using the QIAGEN midi system (tip 100, cat. No 12143). Cells were grown in Luria broth (Merck) at 37°C with shaking at 200 rpm for 18 hours. Cells were harvested by centrifugation at 6,000 x *g* and the DNA isolated as per manufacturers instructions. Restriction digestion (Roche, Life Technologies), dephosphorylation (Roche) and ligation (Life Technologies) were carried out using manufacturer's recommended conditions and as outlined by Sambrook et al. Transformation was accomplished using electrocompetant cells and a BIO-RAD Gene pulser set at 12.5V $cm^{-2}$. Two μl of DNA was used to electroporate 80 μl of early log phase *E. coli* DH5 alpha cells washed 3 times in sterile water (centrifugation at 6000 x g for 5 mins) and resuspended in 1 /100th the original volume in 10% (v/v) glycerol. Luria agar containing either kanamycin or ampicillin at 50 μg $ml^{-1}$ were used to select clones where appropriate.

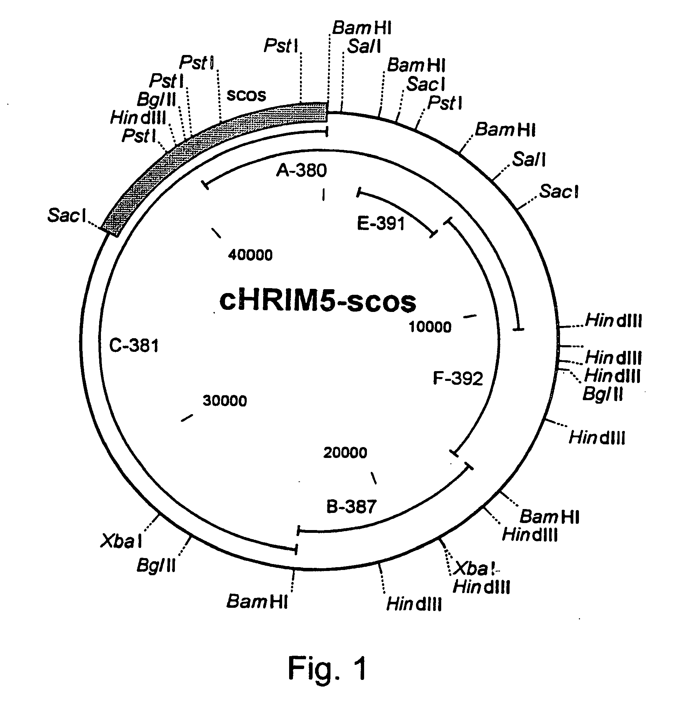

DNA sequence analysis of cHRIM5 was completed by sequencing a number of sub clones and primer walking, see figure 1 for the supercos vector, where the numbers are kBp. The sub clones used are as follows:

| code | cHRIM5 treatment | vector used or remaining |
|---|---|---|
| A-380 | *Hin*dIII digestion and self-ligation | deleted scos |
| B-387 | *Bam*HI digestion and self-ligation | pUC 19-*Bam*H1 digestion |
| C-381 | *Sal*I-*Bam*HI digestion | scos |
| E-391 | *Sal*I-*Bam*HI digestion | pUC 19-*Sal*I *Bam*H1 digestion |
| F-392 | *Sal*I-*Bam*HI digestion | pUC 19-*Sal*I *Bam*H1 digestion |

Sub clone A-380 was constructed by digesting cHRIM5 DNA with the restriction enzyme *Hind*III and re-ligating fragments, this clone contains a deletion of the insert and scos cosmid DNA as the vector. Sub clone B-387 is a *Bam*HI digestion of cHRIM5 cloned into the plasmid pUC 19 also cut with *Bam*HI and dephosphorylated. Sub clone C-381 was obtained by digesting CHRIM5 DNA with *Bam*HI and re-ligating the fragments, this clone contains the scos cosmid as the vector. Clones E-391 and F-392 were obtained by cutting cHRIM5 DNA with *Sal*I and *Bam*HI and ligating these fragments into the vector pUC19 also cut with these enzymes.

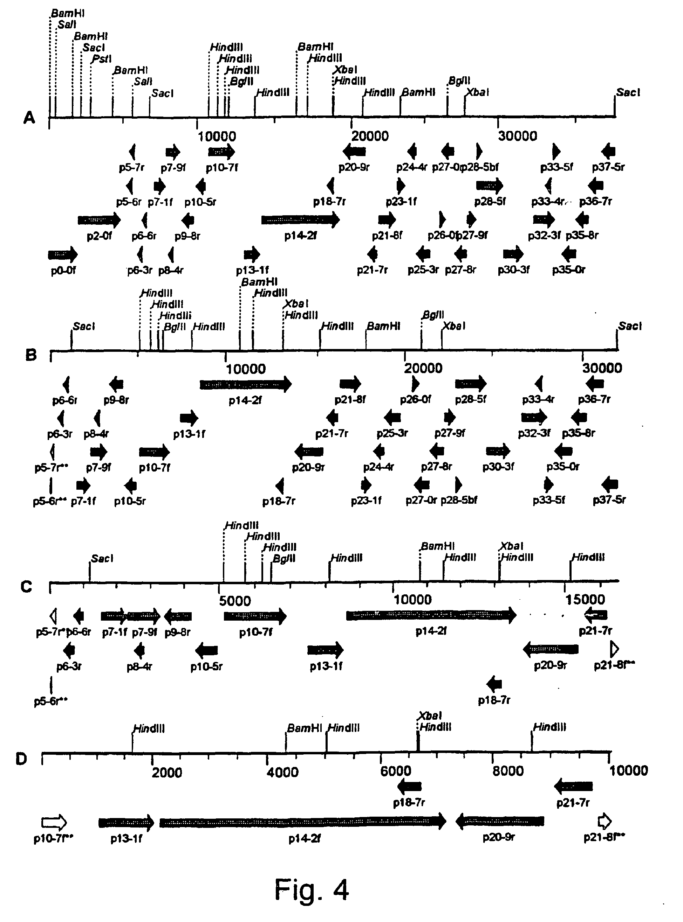

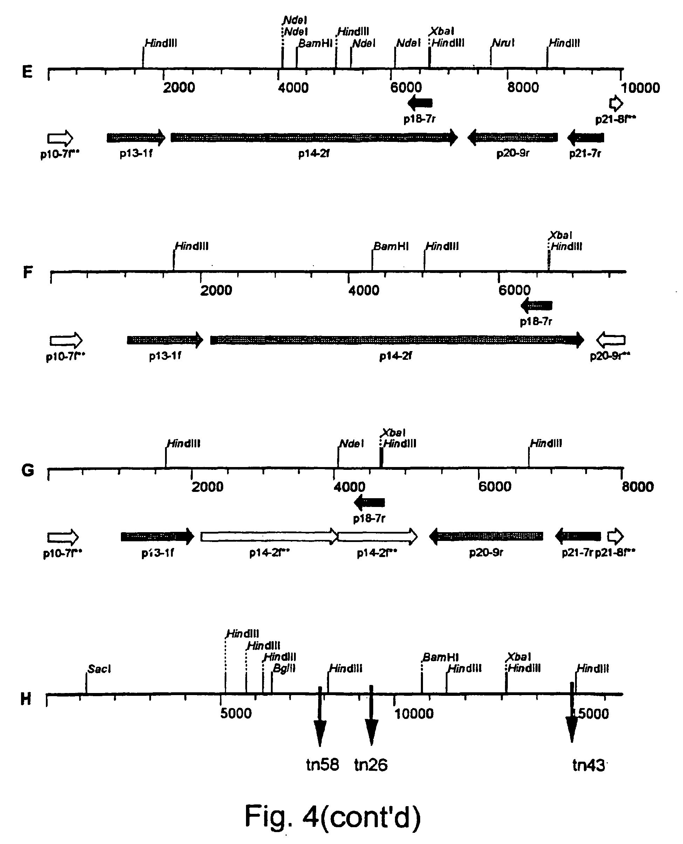

Sequencing was conducted using the artificial transposon AT2 (supplied by Perkin-Elmer-Applied Biosystems, Primer Island Transposition kit, cat No. 403015) using the cosmid cHRIM5 and all sub-clones as target DNA. One μg of cHRIM5 DNA was incubated with the transposon AT2 for 1 hour at 30°C in a final volume of 20 μl. After incubation the reaction was stopped by adding 5 μl of 0.25M EDTA, 1% (w/v) SDS, and heat treatment at 65°C for 30 mins. The DNA was desalted by dialysis against water. One μl of the reaction mix was used to electroporate 80 μl of early log phase *E. coli* DH5 alpha cells. Colonies were selected on LB media containing 50 μg/ml trimethoprim. Once inserted the transposon mutants were used to provide a range of positions of primer sites at random intervals throughout the clones. The two primers PI+ and PI- near the end of the transposon were used to generate sequence data. In addition standard primers for the pUC19 and scos vectors were used to generate sequence data at the ends of each clone. DNA for sequencing was prepared using the QIAGEN ion exchange media (qiawell8, cat. No. 17122). Clones were grown in 1 ml of Luria broth containing trimethoprin (50 μg $ml^{-1}$) for 18 hours. Cells were centrifuged at 13,000 x *g* for 5 mins and

resuspended in 350 μl of buffer P1. After 5 mins 350 μl of buffer P2 was added and the samples incubated for 5 mins at room temperature. To this 350 μl of buffer P3 was added and the samples left on ice for 15 mins. After centrifugation at 13,000 x *g* for 15 mins the samples were loaded on the Qiagen column under vacuum, and washed with buffer QC. DNA was eluted with buffer QF (500 μl) at 50°C and isopropanol precipitated (0.8 vol). After centrifugation at 13,000 x *g* for 30 min, DNA was washed with 70% (v/v) ethanol and air dried for 10 mins. The final pellet was resuspended in 10 μl of water. Cycle sequencing reactions using the Perkin-Elmer Applied Biosystems division Big Dye reaction kit (cat No. 4303149) were prepared using standard conditions for plasmid and cosmid sequencing. Samples were analysed on ABI Automated Sequencers. DNA sequences were assembled using the DNA* software. The complete sequence of cHRIM5 was obtained by primer walking to join the final DNA contigs together. The final sequence of cHRIM5ed2 is shown in Figure 2. Analysis of the DNA using the software Clone indicated a number of ORF illustrated in Figure 3 and 4. Corresponding protein sequences are also presented at Annex I.

Example 7 - Fragments that encode nematocidal activity

To identify smaller fragments that encoded nematocidal activity, a series of sub-cloning experiments were performed using *E. coli* DH5 alpha. Qiagen midi and miniprep methods, restriction and ligations were used as for previous examples. Nematicidal activity of all constructs was determined as described in Example 4. In Figure 4, we show the deletions of cHRIM5 tested for nematicidal activity. Restriction sites and genes are indicated. Size in base pairs indicated on the map line. A cHRIM5, B cHRIM6, C cHRIM7, D cHRIM8, E cHRIM8, F cHRIM10, G *Nde*I deletion of cHRIM8, H Approximate positions (arrows) of three AT2 transposon insertions (tn58, tn26, tn43) in cHRIM9.

The cosmid cHRIM5 (figure 4A) was digested with the enzyme *Sal*I and religated. The resulting sub clone cHRIM6, illustrated in Figure 4B showed nematicidal activity. cHRIM6 was digested with the enzyme *Sma*I and religated, producing sub-clone cHRIM7 (Figure 4C). CHRIM7 was digested with *Bgl*II and the kanamycin resistance gene block (*nptII*, Pharmacia) cut with *Bam*HI was ligated into it. After selection on LB containing kanamycin (50μg $ml^{-1}$) and ampicillin (50μg $ml^{-1}$) the clone was digested with *Sal*I and religated, in effect creating a deletion from the *Sal*I site to the *Bgl*II site of cHRIM6 to generate CHRIM8 (figure 4D). By cutting cHRIM8 with *Nru*I a further deletion was made to create cHRIM10 (figure 4F). All the above clones maintained nematicidal activity.

Deletion of cHRIM8 with *Nde*I, removed a portion of the p14-2f gene (figure 4G), this reduced nematicidal activity. This indicates that the p14-2f gene or protein are important for nematocidal activity. Transposon mutagenesis of cHRIM9 (a clone very similar to cHRIM7 but deleted with *Nar*I rather than *Sma*I) with the artificial transposon AT2 (Perkin Elmer Applied Biosystems) resulted in a number of inserts within this clone (figure 4H). Insert cHRIM9-tn43 was restriction mapped to an approximate position of bp 20,700 (on cHRIM5) within the p20-9r gene, this mutant retained nematicidal activity. This indicates that this gene is not essential for activity. Insert cHRIM9-tn58 mapped to an approximate position of bp 13,400 (on cHRIM5), within the p13-1f gene, nematicidal activity was reduced. This indicates that this gene, region of DNA or the blocking effect of the transposon in this position is important for activity. Insert cHRIM9-tn26 was restriction mapped to approximate position of bp 15,000 (on cHRIM5) within the p14-2f gene, nematocidal activity was reduced. This indicates that this gene, region of DNA or the blocking effect of the transposon in this position is important for activity.

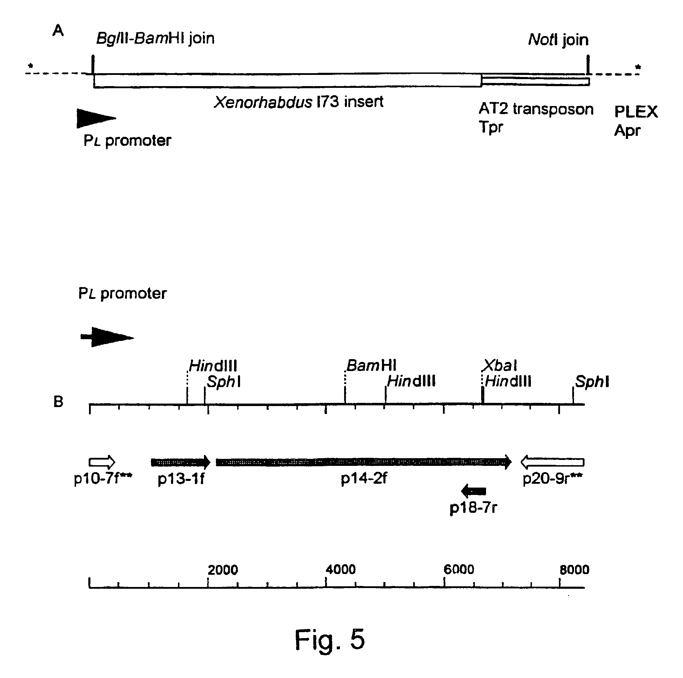

Clone cHRIM6-tn43 was digested with *Bgl*II and *Not*I and cloned into the vector PLEX (Invitrogen cat. No. K450-01) cut with *Bam*HI and *Not*I. The *E. coli* strain used was GI742 supplied by Invitrogen. The resulting plasmid insert (PLEX-*Bgl*II/tn43, Figure 5) places the p14-2f and p13-1f genes under the control of the bacteriophage Lambda $P_L$ promoter. Figure 5 illustrates the cloning of DNA encoding nematocidal activity in the expression vector PLEX, where: A, plasmid clone; B, insert and gene locations; Tpr, trimethoprim resistance; Apr, ampicillin resistance; $P_L$, bacteriophage lambda PL promoter; *, plasmid joins to form a circular molecule; **, incomplete genes. Selection of colonies on RMG media (described in the Invitrogen manual) containing ampicillin (50 μg $ml^{-1}$) and trimethoprim (50 μg $ml^{-1}$) prevents expression from the $P_L$ promoter. Colonies were then cultured on LB containing Trimethoprim (50μg $ml^{-1}$) in 2.0 $cm^2$ wells for nematocidal tests. The clone was active. This indicates that genes within this fragment have nematocidal activity. The clone PLEX-*Bgl*II/tn43 was digested with *Cla*I and religated, this resulted in a deletion of part of the p13-1f gene, this clone had reduced nematocidal activity indicating the importance of this gene.

All these results indicate that the genes and gene products of p13-1f and p14-2f are important for nematocidal activity. Other smaller genes within the *Bgl*II to *Nru*I sites of cHRIM10 and PLEX-*Bgl*II/tn43 may also be essential. In addition genes outside this region within the remaining cosmid clone (cHRIM5) may also encode products with nematocidal activity, or may enhance the nematocidal activity of genes in the smaller region (*Bgl*II-*Nru*I of cHRIM10 and PLEX-*Bgl*II/tn43).

Example 8 - Field trials

Activity of strains selected in accordance with the above methods, or from depositary institutions which include bacteria which in nature are associated symbiotically with entomopathogenic nematodes, may be further assessed in field trials as follows.

Symbiotic bacteria in the absence of their nematode host can be inoculated into one or more portions of a field which is infested with nematodes, or into containers containing unsterilised soil from such a field. The bacteria can be inoculated onto the roots of plants, or into seeds. Periodically treated and untreated areas or containers can be assayed for nematode larva, egg, or cyst counts and for the presence of the inoculated bacteria by methods well known to those skilled in the art. A reduction in the number of nematode counts in areas in which the symbiote bacteria are present indicates control of the nematodes otherwise found in the untreated areas or samples.

Annex I - amino acid sequences

SEQ ID NO:1

P0-0f

ISWFATGIPTVDALLAEEFWHGDKQAFPPFTCRFTHFDPDKEQDVTLVPSTEEAYWLHRA
LQGQPLHSEVYGDDGTAQAGIPYTVMDSRPQVRLLTGLPGNSPTVWPSVIEQRTWQYERI
ADDPQCHQQVVLNSDRYGFPRETVDIAYPRRPKPAVSPYPDTLPATLFDSSYDEQQQQLR
LTRQRQHYHHLTDTEHQVLGLPDVMRSDAWGYPAARVPREGFTLEDLLAENSLIAPGTPL
TYLGHQRVAYTGTTGTEEKPTRQALVAYTETAVFDELALQAFNGTLSPEALEKKLIESGY
LSVPRPFNTGAESAVWVARQGYTDYGGSEAFYRPLAQRTTVQIGKNTLHWDTHYCAVVRM
QDAAGLYTDAAYDYRFLTPVQITDANDNQQHITLTALGQVSSGRFWGTEEGTPQGYTPPE
DRPFTPPSSVAEALDLKPDLPVANCMVYAPLSWMPLAHTYQEYIAGFTWQALLDAGVVTE
DKRVCALGFRRWVQRQGIVLNGQALADSREPVHVLTLATDRYDTDPDQQLRKSVTYSDGF
GRLLQSAVYHAPGEAWQRAADGSLITDAKGAPLVAHTATRWAVSGRTEYDGKGQPVRTYP
PFFLNAWQYLSDDSARQDLNADTHRYDPLGREYQVRTAKGYLRQNRLTPWFVVNEDENDT
LS

SEQ ID NO:2

P1-2r

YLPQRGQCDMLLVVIGIGYLNGGQEAVIIGGIRVQTRRILHTDDRTVMGIPMEGVFANLH
RRPLSQRTVKRLRPAVIGISLTGDPDRRFRTGIEWAWNRQITRLD

SEQ ID NO:3

P2-0f

SHLPARYGGRLTTLSRKGFMTVNRGDNLHQKTPEVTVLDNRGLTVRELRYHRHPNTPTTT
DERITRHRFTLSGQLAHSIDPRLFDLQQTDNTVNPNMIYDTALTGEVVRTRSVDAGNDLI
LNDITGRPVLAINATEVTRTWQYENDTLPGRPLSITEQPAGEAGRITERFVWAGNSQAEK
NSNLAGQCVRHYDTAGLNQTDSIALNGIPLSVTRQLLPDGTDADWQGNNEPAWNDRLAPE
NFTTLSTADATGAVLTTTDAAGNLQRVAYDVAGLLTGSWLRLAGGTEQVIVKSLTYSAAG
QKLREEHGNGVVTTYTYEPETQRLVGIKTKRPQGHAQGTKVLQDLRYEYDPVGNVVKVTN
DAEVTRFWRNQKVVPENTYVYDSLYQLVSATGREMANIVQQSTLLPTPSLIDSSTYSNYS
RTYNYDRGDNLTQIRHSAPATGNSYTTDITVSDHSNRAVLDTLTDDPAKVDALFTAGGHQ
IPLQPGQNLVWTPRGELLKVAPVVRDGQISDQESYRYDAASQRIIKTHVQQTANSSQAQS
TLYLPGLERHTTINGTTVKEVLHVITIGEAGRAQVRVLHWENGKPGAISNNQMRYSYDNL
IGSSGLEVDGDGQIISMEEYYPYGGTAVWTARSQTEADYKTVRYSGKERDATGLYYYGYR
YYQPWAGSWLSADPAGTIDGLNLYRMVRNNPATLDDKNGLAPGNRYVFFPFIHEDRIFRL
ASANVYRTEHNKSDIIAVVEDKALDSKLFTNSIEQFFKKPKGKAILKGSPDIKERLLNNI
VHDLSNMQVGDQLYVNAHGHSAKPFFYSDSGYSKIIMEQLQRGANYVAKDLVNKFKLPEN
ATIKISTCHSAEGKGAHITVTSTGTNEKMRYSSIIENKGEFSRSLAGTMENELIKLQPGR

VRGNVYGYLGATTFYGAKNEKVIHLKDGNLTTGVHEGKLSMFTKKNRFSENIFGLKVKRS
LTRTNFTGSGV

SEQ ID NO:4

p-2-9r

PAAEYVRDFTITCSVPPASRSQLPVSRPATSYATRCRLPAASVVVSTAPVASAVLRVVKF
SGASRSFQAGSLFPCQSASVPSGSSWRVTDSGMPLSAILSVWFSPAVS

SEQ ID NO:5

P3-2r

QRALLNDIGHFAPGGTDQLIQAVIDIGVLRHHFLVAPEAGNLRIVRHFHHVPHRVVLIAQ
VLQHLRPLCMSLWAFGFYANKALGLRLVGVGGHHAVAVLFAQFLTRGGIRQGFHDNLLCP
ARKPQPTASQQACYVIRHTLQVTGRIGGGQYRAGGIRRAQGGEVFRCQPVVPGGFIVSLP
VCVRTIRQQLARDGQRYAVKRNTVRLVQSGGVIVTHALSGQVAVLLRLTVPCPDKTLCDT
ACFASRLFCDTERASG

SEQ ID NO:6

P3-6r

SDRRQTGYAYSADHYRISGRSTVCTVRAGLMNYQCWLQHAATQLSESDSPKRDAEILLGY
VTGRSRTYLIAFDETLISSEELHQLDSLLVRRIQGEPVAYIIGEREFWSLPFAVSPATLI
PRPDTECLVEKALELLPDSPARILDLGTGTGAIALALASERNDCYVTGVDINSDAVMLAQ
HNAEKNAGKLAIHNVNFLQSEWFAAVGNQQFDMIVSNPPYIDERDPHLQEGDIRFEPATA
LIAAQNGMADLQAIVGQARHFLSPNGWLLLEHGWKQGTVVRNLFLEKGYQQIATFQDYGG
NERITIGRWNKNETHS

SEQ ID NO:7

P3-7f

ARRAVRRCGYCTGRTESRVPSVTTRCATAMITLSAAAVWRWTVTDKLSVWKNTTRTGALR
CGRRGVRQRLITRLCVTQARSGMQRGCIITATGITSRGRGAG

SEQ ID NO:8

P5-6r

WQNGGSSSTTPRYLAGCYVWYPCSARLSGNAKSLLAPDGEWMKHTLKSKASGNTFTGRLI
PTGRPTVVTIDKSGANTAALTLLNAEGEPQQGIEIRQNKYLNNRIEQDHRHVKRRIRPML
GFKSFRRAQT

SEQ ID NO:9

P5-7r

ALLFLSESRVMSLIRNAFKLLHYPVDIMAQCVRWSLTYALSLRNLEEMMAKRGIFVDHAT
IPRWVLRLVPLLSKAFRKRKKPVGSRWRMDETYIKVKGQWKYLYRSVDTDGQTDCGDYR

SEQ ID NO:10

P6-3f

VHSPSGAVAPGKFFIENFADTFPAPLPLHPFIDACIQQGFQLLPCLIAIAHSGKQAFECV
LLDRLALQGSQCLQALVLPVGDVNGQTAHGFLLIGYTQTHISTYNGLWLFITQGVRYRFV
RQTFVCRSLSFSEDDCTN

SEQ ID NO:11

P6-3r

RTCRERPRLMDYVLTKAAEADLRAIIRHTRKQWGDAQVRRYITALEQGIARLAVGQGSFK
DMSALFPALRMAHCERHYVFCLPRENAPALIVAIFHERMDLLTRLADRLK

SEQ ID NO:12

P6-6r

PQTIICANVGLCITDKEKTMSRLTIDITDRQHQSLKALAALQGKTIKQYALERLFPGMSD
SDQAWQELKALLDTRINEGMEGKGCGKSIGEILDEELAGSDRA

SEQ ID NO:13

P7-1f

NAHFLIVSKTNVVMSNQDPHNKRDSLFSAPIANLGDWSFDERVAEVFPDMVKRSIPGYSN
IISMIGMLASRFVTPGSQIYDLGCSLGAATLSIRRSINADNCRIIAIDNSPAMIERCRRH
IDSFKASTPVEVIEQNILDTDIQNASMVVLNFTLQFLHPDDRQKILKKIYAGLKPGGVLV
LSEKFNFEDQKIGELLFNMHHDFKRANGYSELEVSQKRSMLENVMRTDSVDTHKSRLKEV
GFQHVEVWFQCFNFGSLLAIKGTEQ

SEQ ID NO:14

P7-9f

TMIDFGNFYQLIAKHPLNHWLDSLPAQLSHWQKTSQHGQFSSWVKILENLPEIKPSHLDL
KNGVIAIHEPDLSKGEKARLHNILKILMPWRKGPFSLYDVEIDTEWRSDWKWERVLPHIS
PLEGKTVLDVGCGSGYHMWRMVGEGAQLVVGIDPTQLFLCQFEAIRKLLGNNQRAHLLPL
GIEQLPELQAFDTVFSMGVLYHRRSPLDHLWQLKNQLVSDGELVLESLVIEGDENQCLIP
GERYAQMRNVYFIPSAKMLKVWLEKCGFVDVRIVDHAATTPDEQRRTEWMKTESLVDFLD
PSDHSKTIEGYPAPLRAVLIARKP

SEQ ID NO:15

P8-4r

SLQIDREKVGLDRYPQPIERLRQPCATCDNHCHSRHQVRFFLLKEKYGAALAPISSQSAI
RYQFQRHTMKKGLFAMASIFSGYCGGELFHLLTDPAHESQ

SEQ ID NO:16

**P9-8r**

SSFRLNDDLLTNSYSEGFLMIKLEICCYSISCALVAQNAGADRIELSASPLEGGLTPSFG
ALQQSLQRLSIPVHPIVRPRGGDFCYNNMDFEAMKNDVARIRDMGFPGIVFGILSENGHI
DRLRMRQLMSLSGNMAVTFHRAFDMCFNPHVALEQLTELGVQRILTSGQQQNAELGLTLL
KELMQASRGPIIMPGAGVRVSNISKFLEAGMTEVHSSAGKIVPSTMKYRKVGVAMSSDDR
DVDEYSHYSVDGELVESMKGVMSLIKR

SEQ ID NO:17

**P10-5r**

YFGKNRRFVIYVTLMERNFYGLFNGEEMSHFSKISELQDLVADLAGFEQKLKQFEGHLGL
HFEQYSADHISLRCNESKIADRWRKGFLQCGQLISESIINGRPICLFDLNQPIVLLDWKI
DCVELPYPSQKHYVHQGWEHVELVLPVPPEQLICEAKKLLPQPLPDNFRMKESHPKGKNE
RLPNPILAV

SEQ ID NO:18

**P10-7f**

GNTVNIQVILSEKISNALIEAGAPTDSEAHVRQSAKAQFGDYQANGVMAAAKKVGIPPRQ
LAEKVVSQLDLQGIASKVEIAGPGFINIFLDKAWVAANIETTLKDEKLGITPVEPQTIVI
DYSAPNVAKQMHVGHLRSTIIGDAAARTLEFLGHKVIRANHVGDWGTQFGMLIAYLEKIQ
NENANDMALADLEAFYREAKKHYDEDEEFAIRARNYVVKLQGGDEYCRKMWRKLVDITMS
QNQETYNRLNVTLTEKDVMGESLYNDMLPGIVADLKQRGIAVKSDGATVVYLDEFKNKEG
EPMGVIIQKKDGGYLYTTTDIACAKYRHETLNASRVLYYIDSRQHQHLMQAWAIVRKTGY
IPESMSLEHHMFGMMLGKDGKPFKTRAGGTVRLSDLLDEAIERADTLIREKNPDMPEDEL
KKVVEAVGIGAVKYADLSKSRTTDYVFDWDNMLAFEGNTAPYMQYAYTRVSSIFKRADID
ENSLTLPVMLNEEREQALATRLLQFEETITTVAREGTPHVMCAYLYDLAGLFSGFYEHCP
ILNADSEELRQSRLKLALLTAKTLKQGLDTLGIQTVERM

SEQ ID NO:19

**P11-1r**

AQVSNMHLLGDIRCGIIDNDGLRFHWGDTELFIFQGSFYICCNPRFIKKNIDKTWACNFN
FAGNSLQIQLADDFFCQLSRRYSHLFSGSHHTIRLIVTKLCFGRLTDVSFTVGWSASFNQ
RIADFF

SEQ ID NO:20

P12-1r

HARVGVLHIRCRVAFKGQHIIPVENIVCSTALGKICIFHRANPYRFHDFFQFVFWHIWVF
LTNEGIRTLNRFIQQIGQSYCAAGTGFEWFTIFAQHHAKHVVFE

SEQ ID NO:21

P12-5r

YHASFQLCRRLLHTFYSLNTQSIKTLLQSFRCQQSQLQAALAQFFAIGIQDRAVLIETRE
QTGQIVQVCTHNMWRTFTGDGSDRFFKLQQAGCQCLLAFFIQHHRQCQAVFIDIRTFKDR

SEQ ID NO:22

P13-1f

FTLREDSMSDWTGVSTFNVILETGLDNCNIYANGLNMIGVIINITPTDDEGNFVDIDDVT
LNDNIKIVDYIDGSDIDGSDGWFYTGNPNEYNTIPNSQSYSLLKSENSQITQIKRYVSCS
NTSRLRTKSFSAKVTTTSGKVISITQNSINSSRVVINAIDATNFTDDELRTTKETRFENQ
SYTSHKSSTNSLYVHTWTIPRSLKLQNWRWEDYNNGWTWAQSCYYKTGADGGSESTRWLA
AGSIFPPGNYDGLWLDNDIALSGMAHKSYNVDTGINQLSFTRIIGKGFSWVYNISGLDRG
HAVIIIDQYGNKYRILFHAGYENSDPYLSSSIVY

SEQ ID NO:23

P14-2f

VYIKFLKLFRRITMSDNNEFFTQANNFTSAVSGGVDPRTGLYNIQITLGHIVGNGNLGPT
LPLTLSYSPLNKTDIGFGIGFNFGLSVYDRKNSLLSLSTGENYKVIETDKTVKLQQKKLD
NLRFEKDLKENCYRIIHKSGDIEVLTGFNNNAFDLKVPKKLLNPAGHAIYIDWNFEATQP
RLNRIYDDLDGHDIPLLNLEYQGLIKTILTLFPGQKEGYRTELRFLNRQLNSIHNFSLGN
ENPLTWSFGYTPIGKNGILGQWITSMTAPGGLKETVNYSNNNQGHHFPQSANLPVLPYVT
LMKQVPGAGQPAIQAEYSYTSHNYVGGGSNGIWNNKLDNLYGLMTEYNYGSTESRRYKDK
EGHDQIVRIERTYNNYHLLTSECKQQNGYIQTTETAYYAIIGHNFDSQPSQFQLPKTKTE
TWRSADNSYRSEITETTFDESGNPLTKVIKDKKTQKIISPSTHWEYYPPAGEVDNCPPEP
YGFTRFVKKIIQTPYDSEFKDDPEKFIQYRYSLIGSQSHVTLKIEERHYSATQLLNSTLF
QYNTDKSELGRLLKQTECTKGENGKTYSVVHKFTYTKQDDTLQQSHSITTHDNFTIHRSQ
VRSRYTGRLFSDTDTKDIVTQMSYDKLGRLLTRTLNSGTPYANTLTYDYELNNLQDDNRP
PFVITTTDVNGNQLRNEFDGAGRHVSQCLKDSDGDGKFYTIHTQQYDEQGRHHTSTYSDY
LTNGRQQTDPDKVHLSMSKSYDNWGQIANTHWSYGVSEKITVDPITLTATKQLQSNSNNV
QTGKEVTTYTPSQQPIQITLFDEAGHLQSCHTLTRDGWDRVRKETDAIGQCTIYQYDNYN
RVIQITLPDGTIVNRKYAPFSTDTLITDIRVNGISLGQQTFDGLSRLTQSQDGGRVWAYT
YSAGNDQCPSTVITPDGQFIHYQYQPELDDAVLQVASNEITQQFSYNPVTGALLKAVAEG
QSLTPIYYPSGRLKMENINDMKKMSYLWTLRGLENGYTDLTGTIQKISRDTHGRVTQIKD

SSIKTTLNYDDLNRHIGSQVTDLATGHMLTTTVEFDGLNREIGRKLCDSSGHTLDIQQSW
LKTQQLANRIVKLNGVLQRTEQYSYDSRNRLNQYKCDGAECPTDKYGHSIVTQNFTYDIY
GNITACHTTFADGTEDHATFKFANPTDPCQLTEVHHTHPDMPDNIRLKYDKAGRVINITD
NHGNTENFTYDTLGRLQNGQGSVYGYDPLNRLVSQKTDTLDCELYYRETMLVNEVRNGEM
IRLLRTGETIIAQQRASKVLLTGTDSQQSVILTSDKQNLSQEAYSAYGKHKSTANDASIL
GYNGERADPVSGVTHLGNGYRSYDPTLMRFHTPDSLSPFGAGGINPYSYCLGDPINRSDP
SGHLSWQAWTGIGMGIAGLLLTIATGGMAIAAAGGIAAAIASTSTTALAFGALSVTSDIT
SIVSGALEDASPKASSILGWVSMGMGAAGLAESAIKGGTKLATHLGAFAEDGENALLKST
SESSRIKWGVTRSLDREIVRNEEGQVIKDHSRGYTDNFMGKGEQAILVHGDKDGFLYHTE
GNKHNGKGPYTRHTPEQLVDYLKDNNIVDLTQGGDKPVHLLSCYGKSSGAADKMAKYINR
PVIAYSNKPTISQGLARIERKDFFLKSTYHSYDPRKIILGRTEKTVKPKTFRP

SEQ ID NO:24

P17-6r

LCYGHICLSGIPHRHIYIGSTYYGNRKSTVLYAAILHSVSLFYLLIAVFSASSAGYLTYG
LSYHTISVQFLGLSHQIPLLLSTYDQSLNLLLDYQYGDSGHRNLE

SEQ ID NO:25

P17-8r

SAQCIVGKVFRISMVISDIYYSTSLIIFQPDIIRHIWMSVVYLCQLAWVSWVGKFEGSMV
FCPICECGVTGGDIAIDIISKILCDYAMAIFVCRAFRTVTFILVQPITGIVRVLFCTLQY
SIQFHYSIC

SEQ ID NO:26

P18-7r

PSSLRTISLSKLLVTPHFILELSEVDLSKAFSPSSANAPRCVASLVPPLMADSANPAAPI
PIETHPSIEDAFGEASSSAPLTIDVISDVTLSAPNASAVVEVEAIAAAIPPAAAIAIPPV
AMVSSNPAIPMPIPVHACQLK

SEQ ID NO:27

P19-5f

AHCHIALFPCWHNPQYCQQHPDHHSNCHHQFKQEYPPSRQRRENITLTQLPIKHTGIEAG
SQTNRKRQTCMFQRANESKVHQLGQNQGRDRNFYWCFDILT

SEQ ID NO:28

P19-8f

PQSTPSSQNSRQLTPAESSQHQKQKSDHIEIMIPSEAPREYREQLHKATPARNRDVAPNP
SVFDILRDYHWKNFSPVKAAKSSLTPHPVHQKAIPLNDQRNTSMKQSLKPEMRQKLY

SEQ ID NO:29

P20-1r

GKNCINDQGNLPDRYTQNCRPHLTDNPPYGTVTERNPRQYQHADLFQMRKLIGQLQNPSG
NNGPTQRQHWRIAIRSHKQCKNDHTDIEQCRSKSRHRKAVPCIKNCASQRSQRNQKDIRK
RNSK

SEQ ID NO:30

**P20-9r**

NNTMNLLKSLAAVSSMTMFSRVLGFIRDAIIARIFGAGMATDAFFVAFKLPNLLRRIFAE
GAFSQAFVPILAEYKNQQGDEATRTFIAYISGMLTLILAIVSVIGVIAAPWIIYVTAPGF
TDTPDKFVLTRDLLRITFPYIFLISLASLAGAILNTWNRFSVPAFAPTLLNVSMIIFALF
VAPYCNPPVLALGWAVVAGGVLQLAYQLPHLKKIGMLVLPRISFRDSAVWRVIRQMGPAI
LGVSVGQISLIINTIFASFLVSGSVSWMYYADRLMELPSGVLGVALGTILLPSLAKSFSS
GNHEEYRKLMDWGLRLCFLLALPCAVALGILAEPLTVSLFQYGHFSAFDAEMTQRALIAY
CFGLMGLIVVKVLAPGFYSRQDIKTPVKIAIATLILTQLMNLAFVGPLKHAGLALSIGLA
ACFNASMLYWQLRKRDIFTPLAGWGIFLFKLVVAIAVMVGVLLAVLWVMPAWEQGNMAMR
LLRLMGVVIAGAGSYFAVLALMGFRLKDFAHRGLQ

SEQ ID NO:31

**P21-7r**

AIILIRDKLSRIFSRQISGEGMFGYRSASPKIRFITDRMVVRLVYERDAYRLAEYYSENK
DFLKPWEPTRDGSFYQPSGWTNRLNYIAELQRQNATFNFVLLDSDEREIMGVANFTNVVR
GAFHSCYLGYSLAEKLQGQGLMYEALQPAIRYMQRYQRMHRIMANYMPHNHRSGNLLKKL
GFEQEGYAKNYLMIDGVWQDHVLTALTDDAWGKVGL

SEQ ID NO:32

**P21-8f**

WCAMSLVSQARSLGKYFLLFDNLLVVLGFFVVFPLISIRFVEQLGWAALIVGFALGLRQL
VQQGLGIFGGAIADRFGAKPMIVTGMLLRALGFALMAMAHEPWILLLSCVLSGLGGTLFD
PPRAALVIKLTRPHERGRFYSILMMQDSAGAVVGALIGSWLLQYDFNIVCWIGASIFVLA
ALFNAWLLPAYRISTIRTPIKEGMMRVIRDRRFLYYVLTLTGYFVLSVQVMLMFPIIIHE
ITGTPTAVKWMYAIETAISLTLLYPIARWSEKHFRLEQRLMAGLFLMSICMFPIGWVNQL
HTLFGLLCLFYLGLVTADPARETLSASLSDPRARGSYMGFSRLGLALGGAIGYTGGGWLY
DTGRDLNMPQLPWILLGLSGLITIYALHRQFNQKKIDPVMLGRH

SEQ ID NO:33

**P23-1f**

KGANMKRFFLGAALVLVGLVSGCDQFKDFSINEGLMNDYLLKKVHYQKKISIPGIANANI
TLGDLSSQIGRQDPEKIELSTQAKVQLATLLGTIQADMKLTIKAKPVFDAEKGAIFVKGL
EIVDYQTTPEKAAAPVKALIPYLNTSLSEFFDTHPVYVLNPEKSKAEAAASQFAKRLEIK
PGKLVIGLTDK

SEQ ID NO:34

P24-4r

QVALQHGRRLGTITLFDNLLGLNQVMNEFSIVCRILGTLFNRAPQDPVLQPLITMIAEGK
LKQAWPLEQDEWLDRLQQNSELSVMAADYHALFTGESASVAVCRSDYTDGEESEVRQFLT
ERGMPLSDTPADQFGSLLLAVSWLEDQAAEDEIQAQITLFDEYLLPWCGQFLGKVEAHAT
SGFYRTLAIVTREALQALRDELESE

SEQ ID NO:35

P25-3r

DCMNIIFFHPSFNTDEWIQGIQARLPDAKVRQWVSGDQEPADYALVWQPPYEMLANRQGL
KGIFALGAGVDAIFKQESKNPGTLLADVPLIRLEDTGMGRQMQEYAITSVLHYFRRMDEY
KRYQEQRLWNPIAPHNRKEFVIGVLGAGILGRSVIGKLMEFDFNVRCWSRTSKQLDSVES
FYGKEQLGDFLSGCKVLINLLPDTPDTRGILNLSLFSQLKSGSYVINLARGAQLVEQDLL
VAIDKGYIAGATLDVFAEEPLSNMHPFWTHPRINVTPHIAANTIPEAAMDVICENIRRMV
QGEMPTGLVDRVRGY

SEQ ID NO:36

P26-0f

KTSQGFTSTTCSNGNVLKICGLITPCSSLIQRTYPNNMTIGIFSKESTAKNFGMGFLYYF
DLRVLSPFFKAPINIFTGWQHNTNFRKSRNSTIRLCSSTPNSKQYFTTSRKCHITGAGKY
RFSIENCFIKSG

SEQ ID NO:37

P27-0r

YSAGCSTVLKSSLNLQCDTFNCESFVMLTLNFSTSVNAKPSHIWAHYVDFDLRKKWEVDL
EYFQFEGEVKTGQYGRMILSGMPEIRFYLSNIEVNKEFTDQVNLPQMGILTFRHQIITDE
NNMACRVQVTVSFEPDANIPAVQAESFFKQGTQDLVESVLRLKSVVETVSPKPNLQLVYV
SDIESSTAFYKTIFNAEPIFASSRYVAFPAGGEVLFAIWSGGAKPDRAIPRFSEIGIMLP
SGKDVDRCFEEWRKNPEIKIVQEPHTEVFGRTFLAEDPDGHIIRVCPLD

SEQ ID NO:38

P27-8r

KGNQITMILYKGSKNYLFNQLNYDSCVLLEVDESVNLNGWDELSRAQRLLFLMEILRRYH

FPVQGKVLAQKLNISLRTLYRDIASLQAQGAIIEGEPGIGYVLRPGFVLPPLMFTQNEIE
ALALGANWVAKRADPQLKESANNAISKIAAVIPAELKQMLEASSLLIGPAATAVQPVVEI
QQIRQAINTRHKITLAYLDIKDIPSERTIWPFALGYFENISIVIGWCELREEFRHFRSDR
IMRLKIENQCYPRSRQVLLKEWRAMEKISR

SEQ ID NO:39

P27-9f

RKMTIYDLKPRFQNLLRPIVIYLYKQGITANQVTLTALFLSIFAGSLLSLFPSPHLYWLL
PVFLFIRMALNAIDGMLAREHNQKSHLGAIYNELGDVISDVALYLPFCLLPDVNSLSLLI
ILFLTILTEFIGVLAQTIGASRRYDGPIGKSDRAFIFGAYGLIIAIFPLALGWSISLFAF
MIILLLVTCYQRVVKALREIRLAEQSHSK

SEQ ID NO:40

P28-5f

GVNMTPQLDQRIAEEHYFTTSDNASLFYRYWPQQQANPDRAIIIFHRGHEHSGRIQHVVD
GLDLPDVPMFAWDARGHGKTEGPRGYSPSMGTSIRDVDEFVRFIATQYGIAMENIVVIGQ
SVGAVLVSAWVHDYAPKIRAMILAAPAFDIKLYIPFATQGLQLMQKARGIFFVNSYVKAR
YLTHDETRIASYNSDPLITREIAVNILLDLYQTAERVVKDAAAITLPTLLFISGSDYVVN
KKPQHQFYQQLNTPIKEKHVMDGFYHDTLGEKDRHLVFDKIRVFIERIFALPRYQHDYSQ
EDTWSHSADEFRTLSTSLPCLCPKKLSYQLMRKVMSTHWGRTSEGVCIGLKTGFDSGSTL
DYVYRNQPQGKGILGRILDKHYLNSIGWRGIRQRKIHIEMLIRHAIRSLREQNMPVHMVD
IAAGHGRYILDAINDFSKVDSILLRDYSEINVNQGQAYIEERDLTDKIRFIIGDAFNAES
ISSITPAPTLGIVSGLYELFPDNNLLRNSLRGFADVMTENGYLVYTGQPWHPQIEVIARV
LSSHRDSQPWIMRRRTQGEMDALVEAAGFEKLYQLTDNWGIFTVSIAKRVHR

SEQ ID NO:41

P28-5bf

HHNSINVLLKNIISPHQIMLLCFTVTGHNNRPIQTERSLFFTVVMSTQDVSSMSLTDSIC
LMFLCSRGMPVDTVRQKGRAVTAHPWERRFVMLMNLSDLLPLSTASPWKISWLSARVSER
Y

SEQ ID NO:42

P30-3f

INKYKMEHHMHSSLDSRRRLWLTGVIWLLFLAPFFFLTYGQVNQFTAQRSDVGTVMFGWE
HNIPFWSWSIIPYWSIDLFYGISLFICTHRREQWLHGWRLMTASLIACVGFLLFPLKFSF
SRPTTEGLFGWLFNQLELFDLPYNQAPSLHIILLWLLWLRYSAYVSGYWRGLLHIWSVLI
ALSVLTTWQHHFIDVLTGFAVGVILSYLLPVSYRWRWQPNQDRYARKLFGYYLTGSALFA
LIASLLGGSFWILLWPAVSLLMIALGYAGLGSSVFQKQPDGRMSLSARWLLAPYQLGAWL
SYLWFRRKSAPFNHITEGIILGSLPCQPVTAVSVLDITAEWHRRSDARTVNYVCQPQIDL

LPLAPEALQSAVCTLDKLRQQGDVFVHCTLGLSRSAMVVAAWLLKQHPEYDINTVVAILR
KARPHVTFRQTHLDALSQWAKGYL

SEQ ID NO:43

P31-6f

QSCVKPDRMSRSDKHIWMPCLNGQKATYNGEHNMQPENLISKVIIATLKSWRFISTLSAF
SILIATAMLIAVFNTTALNNIALYAVLLFTTLYCQYYCWRTWLDCHYFQILNSSPEKSAE
FDQTLLLIFNKLPQSRTQNDRFNGAIKLLKKATIGLILQWILFFLFLLTLKYSA

SEQ ID NO:44

P32-3f

MNTRKINGIRPFSAFIDSCLKESYSFPRFIRDIIAGITVGVIAIPLAMALAIGSGVAPQY
GLYTAAIAGIVIAMTGGSRYSVSGPTAAFVVILYPVSQQFGLSGLLIATLMSGVILIVMG
LARFGRLIEYIPMSVTLGFTSGIAITIATMQVQNFFGLKLAHIPENYIDKVVALYQALPS
LQLSDTLIGLTTLLVLIFWPKLGVKLPGHLPALIAGTAVMGAMHLLNHDVATIGSSFSYT
LADGTQGQGIPPILPQFVLPWNLPDTHSLDISWNTVSALLPAAFSMAMLGAIESLLCAVI
LDGMTGKKHHSNGELLGQGLGNIAAPFFGGITATAAIARSAANVRAGATSPIAAVVHSLL
VLLTLLVLAPMLSYLPLAAMSAILLIVAWNMSEAHKVVDLIRHAPKDDIIVMLLCLSLTV
LFDMVRRDHYRHCAGITPVYAQNCQYDSNQHVIFNKRGERVIGRTN

SEQ ID NO:45

P33-4r

ESIGAKTSNVNNTSRECTTAAIGEVAPARTLAAERAIAAVAVMPPKKGAAILPNPWPSSS
PLEWCFFPVIPSRITAHSNDSIAPSMAIENAAGSNADTVFQLISRECVSGKFHGRTNWGR
MGGMP

SEQ ID NO:46

P33-5f

LSYSIWSVAITIGIVLASLLFMRKIANMTRISTSSLTSAEKGLLVVRINGPLFFAAAERI
FAELREKSADYQTIIMQWDAVPVLDAGGLHAFQGFVRELGKEKHIVVCDIPFQPLKTLAR
AKVMPIEGELSFYATLPKALKEMAVDYTPEVCASSEKIQGQ

SEQ ID NO:47

P34-3f

CMSDVENDRRTLGSLLHDTEAQHVNHQIVITKVAATVTQDHLVIAAFFEFFNNIAHLPRA
NKLWFFNINHSTGFRHRFNQIGLAGKEGWKLNHIHHIRDWLSLCRLMHVSDNFHAEGLFQ
FLKDFHPLFQPWPTIRADRRTVSLIKRRFKNIRNAQFLCHGDIVLTNPHGQIP

SEQ ID NO:48

P35-0r

LSCIRFIFLLIQQIYLPLTREGISMQQKVVNIGDIKVANDLPFVLFGGMNVLESRDLAMR
ICEHYVTVTQKLGIPYVFKASFDKANRSSIRSYRGPGLEEGMKIFQELKQTFGVKIITDV
HEPAQAQPVADVVDVIQLPAFLARQTDLVEAMAKTGAVINVKKPQFVSPGQMGNIVEKFK
EGGNDQVILCDRGSNFGYDNLVVDMLGFGVMQQATQGAPVIFDVTHALQCRDPLGAASGG
RRAQVAELARAGMAVGIAGLFLEAHPDPENAKCDGPSALPLAKLESFLMQIKAIDDVVKN
FPELDTSK

SEQ ID NO:49

P35-8r

VDGIKMKPIVNYEFNNTPLIDGIILVSKIIRPDFPQTLVSEQLTALVEEARQRLSSITDS
KVKLDSLLTLFYREWKFGGANGVYCLSDTLWLDRLLHSRQGSPVSLGTVFTHIAQALGLS
VQPVIFPIQLILRIDLLDQPTWFINPLNGDTLNEHTLDVWLKGNIGPTVRLKKQDLQEAD
NVSLVRKITDTIKVSLMEEKKMELALKASEVVLTFDPDDPYEIRDRGLIYAQLDCNHIAV
SDLSYFVEHCPEDPISEMIKMQINTIEQRLIVLH

SEQ ID NO:50

**P36-7r**

SDRRQTGYAYSADHYRISGRSTVCTVRAGLMNYQCWLQHAATQLSESDSPKRDAEILLGY
VTGRSRTYLIAFDETLISSEELHQLDSLLVRRIQGEPVAYIIGEREFWSLPFAVSPATLI
PRPDTECLVEKALELLPDSPARILDLGTGTGAIALALASERNDCYVTGVDINSDAVMLAQ
HNAEKNAGKLAIHNVNFLQSEWFAAVGNQQFDMIVSNPPYIDERDPHLQEGDIRFEPATA
LIAAQNGMADLQAIVGQARHFLSPNGWLLLEHGWKQGTVVRNLFLEKGYQQIATFQDYGG
NERITIGRWNKNETHS

SEQ ID NO:51

**P37-5r**

VEMREMAQEELKEAKIRNEELEQQLQLLLLPKDPDDERNCFLEVRAGTGGDEAAIFAGDL
FRMYSRYAEARRWRVEIISANEGEHGGYKEVIAKVSGDQVYGHLKFESGGHRVQRVPETE
SQGRIHTSACTVAVMPEIPEAELPDISPGDLKIDTFRSSGAGGQHVNTTDSAIRITHLPT
GIVVECQDERSQHKNKAKAMSVLAARIRAAEMRKRQEVEASERRNLLGSGDRSDRNRTYN
FPQGRVTDHRINLTLYRLDEVIEGKLDMLIQPIIIEYQADQLSALSEQD

## Claims

1. The use of a nematode control agent derived from a bacterial strain naturally symbiotically associated with an entomopathogenic nematode to control a parasitic nematode in plants, **characterised in that** the nematode control agent has functional activity against the said parasitic nematode, and is a peptide.

2. Use, in the manufacture of a medicament for the control of a parasitic nematode, of a nematode control agent derived from a bacterial strain naturally symbiotically associated with an entomopathogenic nematode, **characterised in that** the nematode control agent has functional activity against the said parasitic nematode, and is a peptide.

3. The use according to claim 1 or 2, wherein the target nematode is not the same as the nematode with which the bacterial strain is found symbiotically in nature.

4. The use according to any preceding claim wherein the nematode to be controlled comprises one or more of *Haemonchus, Trichostrongylus, Ostertagia, Nematodirus, Cooperia, Ascaris, Bunostomum, Oesophagosromuni, Chaberria. Trichuris, Strongylus. Trichonema, Dicryocaulus, Capillaria, Heterkis, Toxocara, Ascaridia, Oxyuris, Ancylostoma, Uncinaria, Toxascaris, Parascaris, Aphelenochoides, Anguina, Bursaphatenchus, Criconemella, Melodigyne, Ditylenchus, Globodera, Heliocotylenchus, Heterodera, Pratylenchus, Radopholus, Rotelynchus, Tylenchus, Trichodorus, Xiphenema,* and *Caenorhabditis.*

5. A composition for the control of parasitic nematodes which comprises as an effective agent a nematode control agent as defined in any preceding claim, wherein the bacterial species is:

   *Xenorhabdus bovienii* strain H31 deposited with NCIMB under accession number NCIMB 40985;
   *Xenorhabdus bovienii* strain I73 deposited with NCIMB under accession number NCIMB 40986; and/or
   *Xenorhabdus* strain C42 deposited with NCIMB under accession number NCIMB 41004.

6. A host cell containing a vector, the vector comprising a nucleic acid encoding a peptide as defined in any of claims 1 to 4 and which has the same sequence as all or part of cosmid cHRIM5, in particular p13-1f or p 14-2f, and variants thereof having 70% or more DNA sequence identity.

7. A host cell containing a vector according to claim 6 capable of replication.

8. A host cell according to claim 6 or 7, wherein the nucleic acid comprises a natural nucleotide sequence or a degeneratively equivalent sequence, or a functional variant thereof.

9. A host cell according to claim 6, 7, or 8, wherein the variant has a sequence which is a derivative by way of addition, insertion, deletion or substitution of one or more nucleotides.

10. A host cell according to any of claims 6 to 9, wherein the DNA is part of a longer sequence and the nematode control agent is expressed as a fusion protein.

11. A method for producing a transgenic plant which comprises the step of regenerating a plant from a plant cell according to any of claims 6 to 10.

12. A plant produced according to claim 11, which is a crop species which can be maize, cotton, soya, rice, *Brassica* species, tomato, potato, sugar beet, barley, soybean, peanut, onion, rye, wheat, corn, banana, raspberry, bean, or a decorative or other plant.

**Patentansprüche**

1. Verwendung eines Nematodenbekämpfungsmittels, abgeleitet von einem Bakterienstamm, natürlicherweise symbiotisch mit einem entomopathogenen Nematoden verbunden, um einen parasitischen Nematoden in Pflanzen zu bekämpfen, **dadurch gekennzeichnet, daß** das Nematodenbekämpfungsmittel funktionelle Aktivität gegen den parasitischen Nematoden aufweist und ein Peptid ist.

2. Verwendung, bei der Herstellung eines Medikaments für die Bekämpfung eines parasitischen Nematoden, eines Nematodenbekämpfungsmittels, abgeleitet von einem Bakterienstamm, natürlicherweise symbiotisch mit einem entomopathogenen Nematoden verbunden, **dadurch gekennzeichnet, daß** das Nematodenbekämpfungsmittel funktionelle Aktivität gegen den parasitischen Nematoden aufweist und ein Peptid ist.

3. Verwendung gemäß Anspruch 1 oder 2, wobei der Zielnematode nicht der gleiche ist wie der Nematode, mit dem der Bakterienstamm symbiotisch in der Natur gefunden wird.

4. Verwendung gemäß einem vorhergehenden Anspruch, wobei der zu bekämpfende Nematode einen oder mehrere von *Haemonchus, Trichostrongylus, Ostertagia, Nematodirus, Cooperia, Ascaris, Bunostomum, Oesopagosromuni, Chaberria, Trichuris, Strongylus, Trichonema, Dictyocaulus, Capillaria, Heterkis, Toxocara, Ascaridia, Oxyuris, Ancylostoma, Uncinaria, Toxascaris, Parascaris, Aphelenochoides, Anguina, Bursaphalenchus, Criconemella, Melodigyne, Ditylenchus, Globodera, Heliocotylenchus, Heterodera, Pratylenchus, Radopholus, Rotelynchus, Tylenchus, Trichodorus, Xiphenema* und *Caenorhabditis* umfaßt.

5. Zusammensetzung für die Bekämpfung von parasitischen Nematoden, welche als wirksames Mittel ein Nematodenbekämpfungsmittel umfaßt, wie definiert in einem vorhergehenden Anspruch, wobei die Bakterienspezies folgende ist:

   *Xenorhabdus bovienii* Stamm H31, hinterlegt bei der NCIMB unter der Zugangsnummer NCIMB 40985;
   *Xenorhabdus bovienii* Stamm I73, hinterlegt bei der NCIMB unter der Zugangsnummer NCIMB 40986;
   und/oder
   *Xenorhabdus* Stamm C42, hinterlegt bei der NCIMB unter der Zugangsnummer NCIMB 41004.

6. Wirtszelle, enthaltend einen Vektor, wobei der Vektor eine Nucleinsäure, codierend ein Peptid umfaßt, wie definiert in einem der Ansprüche 1 bis 4, und welche die gleiche Sequenz wie alles oder ein Teil des Cosmids cHRIM5, insbesondere p 13-1f oder p 14-2f, aufweist, und Varianten davon mit 70% oder mehr DNA-Sequenzidentität.

7. Wirtszelle, enthaltend einen Vektor gemäß Anspruch 6, fähig zur Replikation.

8. Wirtszelle gemäß Anspruch 6 oder 7, wobei die Nucleinsäure eine natürliche Nucleotidsequenz oder eine degenerativ äquivalente Sequenz oder eine funktionelle Variante davon umfaßt.

9. Wirtszelle gemäß Anspruch 6, 7 oder 8, wobei die Variante eine Sequenz hat, welche ein Derivat ist durch Addition, Insertion, Deletion oder Substitution von einem oder mehreren Nucleotiden.

10. Wirtszelle gemäß einem der Ansprüche 6 bis 9, wobei die DNA Teil einer längeren Sequenz ist und das Nematodenbekämpfungsmittel als Fusionsprotein exprimiert wird.

11. Verfahren zum Erzeugen einer transgenen Pflanze, welches den Schritt der Regenerierung einer Pflanze aus einer Pflanzenzelle gemäß einem der Ansprüche 6 bis 10 umfaßt.

12. Pflanze, erzeugt gemäß Anspruch 11, welche eine Kulturspezies ist, welche Mais, Baumwolle, Soja, Reis, *Brassica*-Spezies, Tomate, Kartoffel, Zuckerrübe, Gerste, Sojabohne, Erdnuß, Zwiebel, Roggen, Weizen, Getreide, Banane, Himbeere, Bohne oder eine Zier- oder andere Pflanze sein kann.

## Revendications

1. Utilisation d'un agent de lutte contre les nématodes dérivé d'une souche bactérienne associée naturellement par symbiose à un nématode entomopathogène, pour lutter contre un nématode parasite des plantes, **caractérisée en ce que** l'agent de lutte contre les nématodes a une activité fonctionnelle contre ledit nématode parasite et est un peptide.

2. Utilisation, dans la fabrication d'un médicament de lutte contre un nématode parasite, d'un agent de lutte contre les nématodes provenant d'une souche bactérienne associée naturellement par symbiose à un nématode entomopathogène, **caractérisée en ce que** l'agent de lutte contre les nématodes a une activité fonctionnelle contre ledit nématode parasite et est un peptide.

3. L'utilisation selon la revendication 1 ou 2, où le nématode cible n'est pas le même que le nématode avec lequel la souche bactérienne est en symbiose dans la nature.

4. L'utilisation selon l'une quelconque des revendications précédentes, où le nématode contre lequel doit être lutté comprend un ou plusieurs d'entre *Haemonchus, Trichostrongylus, Ostertagia, Nematodirus, Cooperia, Ascaris, Bunostomum. Oesophagosromuni, Chaberria, Trichuris, Strongylus, Trichonema, Dictyocaulus, Capillaria, Heterkis, Toxocara, Ascaridia, Oxyuris, Ancylostoma, Uncinaria, Toxascaris, Parascaris, Aphelenochoides, Anguina, Bursaphalenchus, Criconemella, Melodigyne, Ditylenchus, Globodera, Heliocotylenchus, Heterodera, Pratylenchus, Radopholus, Rotelynchus, Tylenchus, Trichodorus, Xiphenema et Caenorhabditis*.

5. Composition pour la lutte contre des nématodes parasites qui comprend en tant qu'agent efficace, un agent de lutte contre les nématodes tel que défini dans l'une quelconque des revendications précédentes, où l'espèce bactérienne est :

   La souche H31 de *Xenorhabdus bovienii,* déposée auprès de NCIMB sous le numéro d'ordre NCIMB 40985;
   La souche I73 de *Xenorhabdus bovienii,* déposée auprès de NCIMB sous le numéro d'ordre NCIMB 40986; et/ou
   La souche C42 de *Xenorhabdus,* déposée auprès de NCIMB sous le numéro d'ordre NCIMB 41004.

6. Cellule hôte contenant un vecteur, le vecteur comprenant un acide nucléique codant un peptide tel que défini dans l'une quelconque des revendications 1 à 4, et qui a la même séquence que tout ou une partie du cosmide cHRIM5, en particulier p 13-1f ou p 14-2f, et des variantes de celle-ci ayant une identité de séquence d'ADN de 70% ou plus.

7. Cellule hôte contenant un vecteur selon la revendication 6, capable de réplication.

8. Cellule hôte selon la revendication 6 ou 7, dans laquelle l'acide nucléique comprend une séquence de nucléotides naturelle ou une séquence équivalente par dégénérescence, ou une variante fonctionnelle de celle-ci.

9. Cellule hôte selon la revendication 6, 7 ou 8, dans laquelle la variante a une séquence qui est un dérivé par addition, insertion, délétion ou substitution d'un ou de plusieurs nucléotides.

10. Cellule hôte selon l'une quelconque des revendications 6 à 9, dans laquelle l'ADN fait partie d'une séquence plus longue et l'agent de lutte contre les nématodes est exprimé comme une protéine de fusion.

**11.** Procédé pour la production d'une plante transgénique qui comprend l'étape consistant à régénérer une plante à partir d'une cellule végétale selon l'une quelconque des revendications 6 à 10.

**12.** Plante produite selon la revendication 11, qui est une espèce de culture qui peut être du maïs, coton, soja, riz, des espèces *Brassica,* tomate, pomme de terre, betterave à sucre, orge, graine de soja, cacahouète, oignon, seigle, blé, céréale, banane, framboise, haricot ou une plante décorative ou autre.

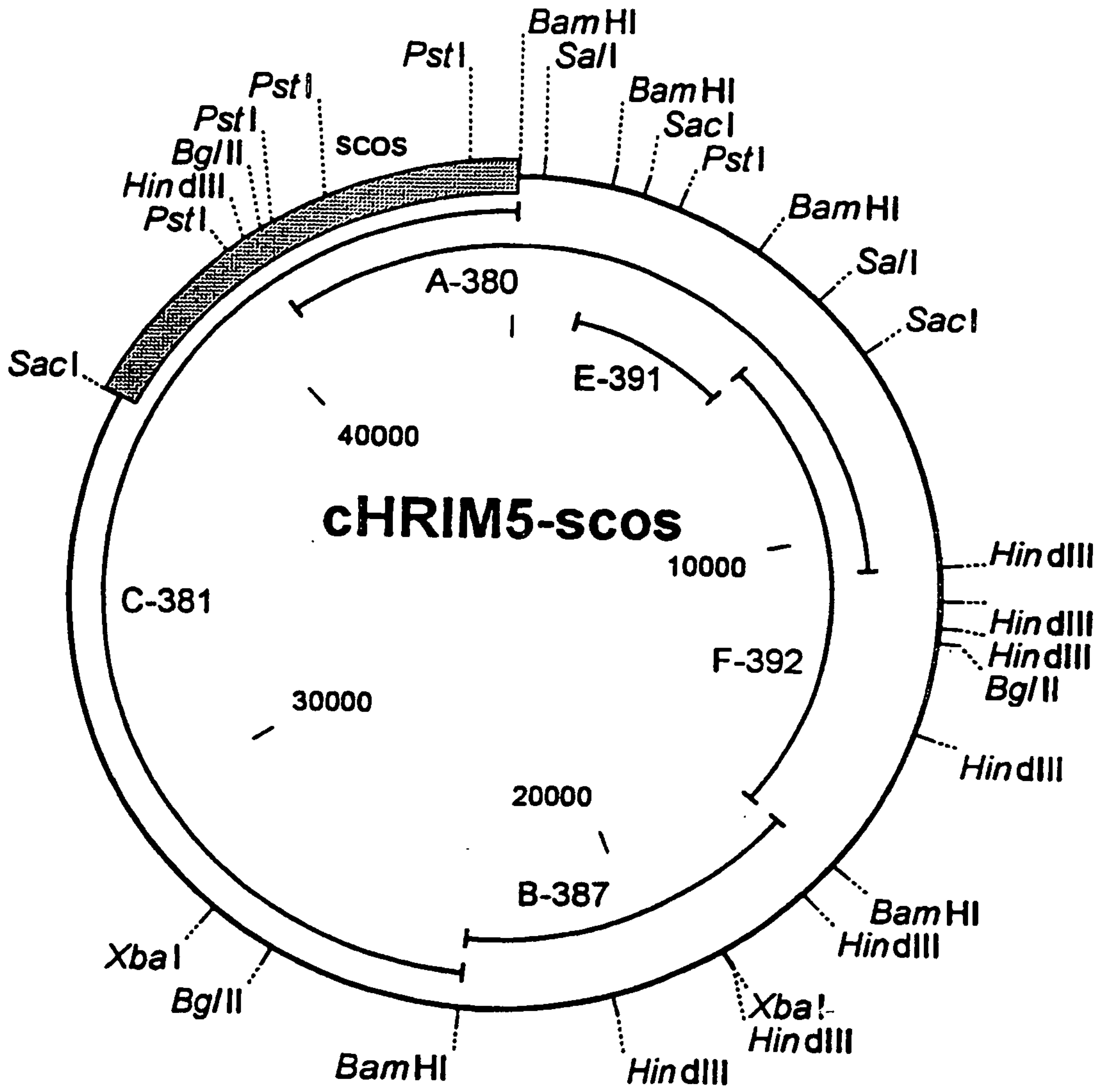
cHRIM5-scos
BamHI
SalI
BamHI
SacI
PstI
BamHI
SalI
SacI
HindIII
HindIII
HindIII
BglII
HindIII
BamHI
HindIII
XbaI
HindIII
HindIII
BamHI
BglII
XbaI
SacI
PstI
HindIII
BglII
PstI
PstI
scos
PstI
A-380
E-391
F-392
B-387
C-381
10000
20000
30000
40000

Fig. 1

Molecule: Sequence Data
Description: chrim5ed2.seq, 37544 bps DNA

1 ggatcagctg gtttgccacc gggatcccca ccgttgatgc cctgttagcg gaggaattct
61 ggcacggtga caaacaggct ttcccgccct ttacctgccg ttttacgcat tttgaccctg
121 ataaagaaca ggatgttact ctcgttccct cgacggaaga ggcttattgg ctgcaccggg
181 cgttgcaagg ccaaccgtta cacagtgagg tctatggcga cgatggcacc gcgcaggcgg
241 gtatccccta taccgttatg gacagtcggc cccaggttcg gcttctgacg ggtttaccgg
301 gtaactcacc gacagtctgg ccgagtgtga ttgaacagag aacctggcag tacgaacgga
361 ttgccgatga tccgcaatgc catcagcagg tggtgctgaa cagtgaccgc tacggttttc
421 cacgggagac cgtcgacatt gcttatccgc gccgccctaa gcctgcggtg tcaccttacc
481 cggatacgct gccggcgacg ttattcgaca gcagctatga tgagcagcaa cagcaattgc
541 ggcttacccg gcaacggcaa cattaccatc acctgactga cactgaacat caagtgctgg
601 gactgcctga tgtcatgcga agcgatgcct ggggctatcc ggcagcgcgg gtaccccgtg
661 aaggtttcac cctggaggac ttgctggcag agaacagtct gatagccccg ggcacgccat
721 tgacctattt agggcatcaa cgcgtggctt ataccggaac gaccggaacc gaagaaaaac
781 cgacccgaca ggcgctggtg gcttataccg aaaccgcggt tttgatgaa ttggccttgc
841 aggcctttaa tggcacattg agtcctgaag ccctggaaaa gaaattaatc gagtctggtt
901 atttgtctgt tccacgccca ttcaataccg gtgcggaatc ggcggtctgg gtcgcccgtc
961 agggatatac cgattacggc gggtctgagg cgttttaccg tccgttggct cagcggacga
1021 cggtgcagat tggcaaaaac accctccatt gggataccca ttactgtgcg gtcgtccgta
1081 tgcaggatgc ggcgggtctg tacacggatg ccgcctatga ttaccgcttc ctgacccccg
1141 ttcagataac cgatgccaat gacaaccagc aacatatcac actgaccgcg ctggggcagg
1201 tatcatccgg ccggttctgg ggcactgagg aagggactcc gcagggttat accccgcctg
1261 aagaccgccc atttacgcca ccgtcctcag tggcggaagc cctcgacttg aaaccggatc
1321 ttccggttgc caactgcatg gtttatgcgc cgctgagttg gatgccgttg gcgcacacct
1381 atcaggaata tatagccggc tttacgtggc aggcactgct tgacgcgggg gtagtgacgg
1441 aagataagcg ggtttgtgcg ctgggtttcc gtcgctgggt gcaacgtcag ggcattgtgc
1501 tgaatgggca ggcattggcc gattcacggg aacccgtcca tgtcctaacg ctggccactg
1561 accgttatga cacggatccc gatcagcaac tgcgcaagag cgtcacctac agcgacggct
1621 tcgggcgttt attgcaaagt gcagtctacc atgcgccagg agaagcctgg caacgcgcgg
1681 cagatggcag cctgatcacg gacgcgaaag gggcgcccct cgtagcccat acggcaaccc
1741 gctgggcggt ctcaggcagg acagagtatg acggtaaagg gcaacccgtc cgaacctacc
1801 cgccattctt cctgaatgcc tggcagtacc tcagtgatga cagtgcacgg caggatttaa
1861 atgccgatac acaccgttat gacccgctcg gccgggaata ccaggtgaga accgccaagg
1921 ggtatctgcg ccaaaatcgg ctgacccccct ggtttgtggt gaatgaggat gaaaacgaca
1981 cgctctctta attaacacga taacgttaaa taatcacacc ttcctgccag gtacgggggga
2041 aggttaacta ctctatcaag gaaagggttt atgactgtaa acagaggcga taacctgcat
2101 caaaaaacgc cggaagtgac ggttctggat aaccggggc tgaccgttcg cgagctccgt
2161 tatcaccgcc acccaaatac cccaccacc accgatgaac ggatcacccg ccatcggttt
2221 actctctcag gtcagttggc gcacagcatt gacccgcgtc tgtttgactt acagcagacg
2281 gataatacag tcaatcctaa catgatttat gatactgcac tgaccggtga ggttgtgcgc
2341 acaaggagtg tcgatgcggg taatgatctg atattgaatg acattaccgg ccggcctgtg
2401 ctggccatca atgcaaccga agtcactcgt acgtggcaat atgagaatga cactttaccc
2461 ggacgcccgc tcagtatcac agaacagcct gctggcgaag caggccgtat cacagagcgt
2521 tttgtctggg cagggaacag tcaggcggag aagaacagca acctggccgg acagtgcgtg
2581 cgtcactatg acaccgccgg actgaaccag acggacagta ttgcgcttaa cggcataccg
2641 ctgtccgtca cgcgccagct gctgccggat ggtacggacg cagactggca gggaaacaat
2701 gaacccgcct ggaacgaccg gctggcaccg gaaaacttca ccaccctgag cacggcggat
2761 gccaccggcg cggtactgac caccaccgat gcggccggta acctgcagcg tgtggcgtat
2821 gacgtagcag gcctgctgac tggcagttgg ctgcggcttg cgggcgggac agagcaggtt
2881 atcgtgaaat ccctgacgta ttccgccgcg ggtcagaaac tgcgcgaaga gcacggcaac
2941 ggcgtggtga ccacctacac ctacgagccg gagacccagc gccttgttgg cataaaaacc
3001 aaacgcccac agggacatgc acaggggacg aaggtgttgc aggacctgcg ctatgagtac
3061 gacccggtgg ggaacgtggt gaaagtgacg aacgatgcgg aggttacccg cttctggcgc
3121 aaccaaaaag tggtgccgga gaacacctat gtctatgaca gcctgtatca gctggtcagt
3181 gccaccgggc gcgaaatggc caatatcgtt caacaaagca cgctgttacc cactccttcc
3241 ctcattgata gcagtaccta cagcaactat tcccgcacct acaattatga ccgtggggac
3301 aatctgacgc agatacgtca cagtgctccg gccactggta acagttacac cacggacatc
3361 acggtctcag atcacagcaa ccgggcagtg ttggacacgc tgacggatga tccggcaaag
3421 gtggatgcac ttttcactgc gggcgggcac cagatcccac tgcaaccggg acagaacctc
3481 gtctggacgc cgcgcggtga gctgctgaaa gtggcacccg tggtacgtga cgggcagatt
3541 tccgaccagg aatcctatcg ttatgatgcc gccagtcagc gcatcatcaa aacccacgtt
3601 cagcagacgg ctaacagctc gcaggcgcag agcacgctgt acctgccagg gctggagcgg
3661 cacaccacaa taaatggcac gacggtgaaa gaggtgctac acgttatcac gataggcgag
3721 gcgggccgtg cgcaggtgcg ggtactgcac tgggagaacg gaaagccggg tgccatcagt
3781 aacaaccaga tgcgctacag ctatgataac cttatcggca gcagcggtct ggaggtggac
3841 ggtgacggac aaattatcag tatggaagaa tactacccgt acgggggcac tgcggtgtgg
3901 acggcgagga gtcagacaga ggctgattac aagactgtgc gttactcagg caaggagcgg

Fig. 2

chrim5ed2.seq

```
3961  gatgcaacgg ggctgtatta ttacggctac cggtattacc agccgtgggc ggggagctgg
4021  ctgagtgcgg acccggcggg cactatcgac gggctgaacc tgtaccgcat ggtcaggaat
4081  aacccggcga cactggatga taaaaacgga ctagcgcccg gaaatagata tgtatttttt
4141  ccatttattc atgaggacag gatttttcgt ctggcaagcg cgaatgttta cagaacggaa
4201  cataataaat ctgacatcat tgcggttgta gaagataaag cattagatag taaactattc
4261  accaatagta ttgagcagtt tttcaaaaaa cctaaaggaa aagcaatcct gaaaggatcc
4321  cctgatatta aagaaaggct actcaataat atagtacatg acctgagcaa tatgcaggta
4381  ggagatcagc tgtatgtaaa cgctcatggt cattctgcga aaccattttt ttactccgat
4441  tcgggatatt caaaaatcat catggaacag ctccaaagag ggcctaacta tgtagctaaa
4501  gatttagtaa ataagtttaa attaccagaa aatgcaacaa tcaagataag tacgtgtcat
4561  agtgctgaag gtaagggcgc tcatattacc gtcacatcca ctggaacaaa tgaaaaaatg
4621  agatacagtt ccattataga gaacaaaggg gaattttccc ggtctttagc aggtaccatg
4681  gaaaatgagt taattaaact acagccgggc agagttcgcg ggaatgtata tggttatctt
4741  ggcgcgacaa cgttctatgg tgctaaaaat gaaaaagtca tacacctcaa agatggcaat
4801  ctgacaactg gcgttcatga aggcaagtta tcaatgttta ctaaaaagaa ccgattttca
4861  gaaaacattt ttgggttaaa ggtaaaaaga agtctgacgc gaacaaactt taccggcagc
4921  ggcgtataaa aacaaatttc aaaccgcatt taaatacgga cagccagcgc gtgctcaaaa
4981  cgacctgacc tgtcacgtcg ttgcttcccc gttacaccgg caggtcggac gcctgctggt
5041  cttttacccg ttttcactgg atattgaccg gcataccatg agcatcacga ccaaatgtgg
5101  ttctgtcgca ttaacggcga gttaatcaga gatcccgcga gtggttgttt tttatactgc
5161  caatcgataa aattgttccg ccgccgataa acattcacct tcctcttgcg aatacgggtc
5221  tttccggagc atggcgacga gttctatccc tgccagtcac gtttgtgccc ggcgaaacga
5281  tttaaaacct aacattggtc gtatccgacg tttgacatgg cgatggtctt gctcaatgcg
5341  gttattcagg tatttatttt gccggatttc aatcccctgc tgaggctcac cttccgcatt
5401  gaggagggtg agcggcggcg tattggcccc acttttatcg atagtcacca cagtcggtct
5461  gcccgtcggt atcaaccgac cggtaaaggt atttccactg gcctttgact ttaatgtatg
5521  tttcatccat tcgccatcgg gagccaacag gctttttgcg tttccggaaa gccttgctga
5581  gcaggggtac caaacgtaac acccagcgag gtatcgtggc gtggtcgacg aagatccccc
5641  gttttgccat catttcctcc agattacgta agctcagcgc gtaggtcaga gaccaacgga
5701  cacattgggc catgatatcg acaggataat ggaggagttt gaatgcgttt cggatcaggg
5761  acatcactct ggactcactc aaaaacagga gagcttacct gatattacgc taatgcgaca
5821  gaacctattt tatcgattga ctgcataaat agtcatcatt cccacctccg tacaaacttt
5881  ctctgttaat gcgacagaac cctttttttcc atattgcctt gcattgcctg tagttgcctt
5941  aaattccttt aaatttccta tagttgctta aaatatccat aatattgcct gagccgattg
6001  gcgaagcgcg tcatttgagc cggtcggcga ggcgcgtcag caaatccatc cgctcgtgaa
6061  aaatcgccac aattaacgca ggcgcatttt cacgcggcag acaaaacaca tagtgacgtt
6121  cacaatgcgc catgcgcaat gccggaaaga gcgcgctcat gtccttaaaa gagccttggc
6181  cgacagcaag ccgcgcaatg ccctgttcca gcgcggtgat gtagcggcgc acctgcgcgt
6241  caccccattg cttgcgcgta tggcggatga ttgcgcgtaa atcggcctca gccgccttcg
6301  tgagtacata gtccatcagg cgcggtcgct cccggcaagt tcttcatcga gaatttcgcc
6361  gatacttttc ccgcacccct tcccctccat cccttcattg atgcgtgtat ccagcaaggc
6421  tttcagctcc tgccatgcct gatcgctatc gctcattccg ggaaacaggc gttcgagtgc
6481  gtattgcttg atcgtcttgc cttgcaaggc agccaatgcc ttcaggcttt ggtgctgccg
6541  gtcggtgatg tcaatggtca gacggctcat ggtttctcc ttatcggtta tacacaaacc
6601  cacattagca catataatgg tttgtggcta tttattacac aggggggtcag atatcgtttt
6661  gtccggcaaa cattcgtttg tcggtcattg tcattttcag aagacgactg caccaattaa
6721  tagggattag agcctggtgt gcatatgact ggcactgttg caaagtttga gtcactgatt
6781  atgctaagtt aaatgtttcg taacgggagc tcaccgtatg aatgtattca aaggccgtca
6841  ttttacaggc attatcattt tgtgggcagt tcgttggtac tgcaaatacg gcatcagcta
6901  tcgtgaactc caggaaatgc tcactgagcg tggcgtcaat gttgaccata cgactcttta
6961  tggttctgtt gcaataagta aagcctcggt aatatctcac ttatttgttg ataagagtgt
7021  cgtcaatgtc gttgatacga aaagccttca tgacacccat gcataagaga agcccatcat
7081  taccaagggc ggtattcctg ccgcctgagg gtttctgctg attatccctt tctgcctttt
7141  cccgcattct gttagaatgc ccacttctta attgtttcca aaactaatgt tgttatgtca
7201  aatcaagatc ctcataataa acgggacagt ctgttctctg ccccaatcgc caatttaggc
7261  gactggagtt tcgacgaacg tgttgccgaa gtctttcctg atatggtgaa acgttccata
7321  cccggttatt ccaatatcat ctccatgata ggtatgctgg ccagtcgctt cgtgacgcca
7381  ggtagccaaa tttatgatct cggttgctcc cttggggcgg caactctgtc catccgccgc
7441  agtatcaatg ctgataattg ccggattatc gctatcgata attcaccagc catgatcgaa
7501  cgctgccgcc gccatattga ttctttcaag gccagtacac ccgttgaggt gatcgaacag
7561  aatatccttg ataccgacat tcaaaatgcc tcgatggtag ttttgaattt cacattacaa
7621  ttcctgcacc ctgatgatcg ccagaaaata ttgaagaaaa tttacgcagg attaaaaccc
7681  ggagggggttt tggttctgtc tgaaaaattc aatttgaag accagaaaat tggcgagtta
7741  ctattcaata tgcaccatga tttcaagcga gccaatggtt atagtgagct ggaagtcagc
7801  caaaagcgca gtatgctgga aaatgtcatg cggacagatt ctgttgacac ccataagtca
7861  cgccttaaag aagtcggttt ccagcatgta gaagtctggt tccagtgttt caatttcggt
7921  tcattattgg caataaaagg aactgaacaa tgatcgattt cggtaatttt tatcaattga
7981  tcgccaagca cccactaaac cattggctgg atagcctgcc agcacaattg agccactggc
8041  aaaaaacatc acagcacggt cagttcagct catgggtaaa aattctggaa aatttgcctg
8101  agatcaagcc aagccacctt gacctgaaaa atggtgtcat tgcgattcat gagccggatc
8161  tgtcaaaagg tgaaaaagct cgcctccaca atatcctgaa aatattgatg ccatggcgaa
8221  aaggcccttt ttcattgtat gacgttgaaa ttgataccga atggcgctct gactggaaat
```

Fig. 2(i)

```
chrim5ed2.seq
8281   gggagcgagt gctgccccat atttctcctt tagaaggaaa aaccgtactt gatgtcggct
8341   gtggcagtgg ttatcacatg tggcgcatgg ttggcgaagg cgctcaattg gttgtgggta
8401   tcgatccaac ccaacttttt ctctgtcaat ttgaagcgat cagaaagttg ttggggaaca
8461   atcaacgagc ccaccttctg ccattgggca tcgaacaatt acccgaactg caagcctttg
8521   atacggtatt ttcaatggga gtgctctacc accgccgctc acctcttgat catctgtggc
8581   aactgaaaaa tcaactggtg tctgatggtg agttagtgct ggaaagttta gtgattgagg
8641   gtgatgaaaa tcagtgcctc attccgggtg aacgctatgc acaaatgcgg aatgtctact
8701   ttattccctc ggccaagatg ctgaaagtct ggctggaaaa atgtggtttt gtcgatgtca
8761   gaattgtcga tcatgcggct acaacacctg atgaacagcg ccggacagaa tggatgaaga
8821   ccgaatcact ggtagatttc cttgacccat cagatcacag taaaacaatt gaaggctacc
8881   ctgccccatt gcgtgctgtc ctcattgccc gcaaaccata atattgaata aatattaatg
8941   agtgaactgt ccaatatggc aattcactca ttaagttcta agatttcgct ttccttatga
9001   cgcaagcgat atcacatcta ccgcttaatc aggctcatca ctcccttcat cgactcaact
9061   aactcaccat caacactgta gtgagaatat tcatctacat cacgatcgtc cgaactcatc
9121   gccaccccta cctttcggta cttcatggta gaaggtacaa ttttacccgc cgagctatga
9181   acttccgtca ttccggcttc cagaaactta ctgatattac tcaccctgac acccgcgccg
9241   ggcataatta tcggcccacg gctggcttgc atcagctctt ttaacagcgt cagccccagt
9301   tcagcattct gctgttggcc tgatgttaaa atacgctgca ctccaagctc tgtcagttgt
9361   tccaacgcaa catgcggatt aaaacacata tcaaaagcgc gatgaaaagt aacagccata
9421   tttcccgaca gtgacatcaa ctgccgcata cggagtctgt caatatggcc gttttcgctc
9481   aaaatgccaa aaacaatgcc ggggaacccc atatcacgga tacgagcaac gtcatttttc
9541   atggcttcaa aatccatgtt gttataacag aagtctcccc ctcttggccg cacaatggga
9601   tgcacaggaa tagataaccg ctgtaacgac tgttgtaatg ccccaaaact gggtgtcaat
9661   ccgccttcca acgggcttgc gcttaattcg attcggtcag cgcccgcatt ttgtgcaacc
9721   agcgcacagc ttatgctata acagcaaatt tccagcttta tcattaaaaa gccctccgaa
9781   tacgaattcg tcaacaagtc atcatttaac ctaaaactac tttattagtg aacttattaa
9841   ctatgacaac taacttatct taatgacatg ttggaaatca caaggtcaga atttttttact
9901   ggaacagcat gataaaaacg tcatttttgc cggctatacc tcacttttga caatttctct
9961   gatacaaaaa ggatgatatt tgatcgtgat ttcaccatca gtgacagcca aaatcgggtt
10021  cggcaaccgt tcatttttttc cttttggatg actctccttc atccgaaaat tatctggtaa
10081  aggttgaggc aataactttt tagcttcaca aataagttgc tctggtggta caggcagcac
10141  cagttcgaca tgttcccaac cttggtggac atagtgtttt tggctgggat aaggcaattc
10201  cacgcaatca attttccagt caagtagtac tattggctgg ttcagatcaa ataagcagat
10261  cggacggcca ttaatgatac tttcagatat taactgacca cattgcagaa agcccttacg
10321  ccaacgatcg gctattttac tttcattaca acgcagggaa atatggtctg ctgaatattg
10381  ttcgaagtgt aagccaagat gcccttcaaa ttgcttaagt ttttgctcaa atccggctaa
10441  atcagccact aaatcctgta actcagaaat ttttgaaaaa tgagacattt cttccccatt
10501  aaataaaccg taaaaattgc gttccattaa tgtgacataa atcacaaatc gtctattttt
10561  gccgaaatat cactcagtaa gcgactaatt gaagttggca taacgacgaa tcgcctgaaa
10621  gacaggctaa aaacaaaaag taacaccacc agaggtggct gatggtagca tgcaggaccc
10681  cgaatatggt ataaaccccg ttattttctc ataacccaca cgcttaaaag tattgcattt
10741  ccaaaatgca taagctttcg tgcgtaactt aaggtaacac ggtgaatata caggttattc
10801  tttcagaaaa aatcagcaat gcgctgattg aagctggcgc tccaaccgac agtgaagctc
10861  acgtccgtca atctgccaaa gcacaatttg gtgactatca agcgaatggt gtgatggctg
10921  ccgctaaaaa ggtgggaata cctcctcgac aattggcaga aaaagtcgtc agccaactgg
10981  atctgcaagg aattgccagc aaagttgaaa ttgcaggccc aggttttatc aatatttttc
11041  ttgataaagc gtgggttgca gcaaatatag aaactaccct gaaagatgaa aagctcggta
11101  tcaccccagt ggaaccgcaa accatcgtta tcgattattc cgcaccgaat gtcgccaagc
11161  agatgcatgt tggacacctg cgctcaacca tcattggcga tgctgcggcg cgtacccttg
11221  agtttcttgg gcataaagtt attcgagcca accacgttgg tgattgggga acccagttcg
11281  ggatgctgat cgcctatctg gaaaagatcc agaacgaaaa tgccaatgac atggcattag
11341  cggatttaga agctttctat cgcgaagcaa agaaacacta cgatgaagat gaagagtttg
11401  ctattcgcgc tcgtaactac gtcgtcaaac tgcaaggcgg tgatgaatat tgccgtaaga
11461  tgtggcgtaa gctggtagat atcaccatgt cccagaatca ggaaacttat aaccgcctga
11521  atgtcacatt gacagaaaaa gacgttatgg gtgaaagcct gtataacgat atgctaccgg
11581  gtatcgttgc agatttaaaa caacgtggaa ttgccgttaa gagtgatggc gcgacagtgg
11641  tttaccttga tgaattcaag aataaagaag gcgaacccat gggcgttatt atccagaaaa
11701  aagatggtgg ctatctttac accacgacgg atatcgcctg cgccaaatac cgtcatgaaa
11761  ccctaaatgc cagccgtgtg ctttactaca tcgattcacg ccagcaccag cacctgatgc
11821  aagcttgggc aattgtacgg aaaacgggtt atattccaga atccatgtca ctcgaacacc
11881  acatgtttgg catgatgctg ggcaaagatg gtaaaccatt caaaacccgt gccggcggca
11941  cagtaagact gtccgatttg ctggatgaag cgattgagcg tgcggatacc ctcattcgtg
12001  agaaaaaccc agatatgcca gaagacgaac tgaaaaaagt cgtggaagcg gtagggattg
12061  gcgcggtgaa atatgcagat ctttccaaga gccgtactac agactatgtt ttcgactggg
12121  ataatatgct ggcctttgaa ggcaacacgg caccttatat gcaatacgcc tacacgcgcg
12181  tgtcatctat ctttaaacgt gcggatatcg atgaaaacag cctgacactg ccggtgatgc
12241  tgaatgaaga acgcgagcag gcattggcaa cccgcctgtt gcagtttgaa gaaacgatca
12301  ctaccgtcgc ccgtgaaggt acgccacatg ttatgtgtgc atacctgtac gatctggccg
12361  gtctgttctc tggtttctat gagcactgcc ctatcctgaa tgccgatagc gaagaactgc
12421  gccagagccg cctgaagttg gctctgctga cagcgaaaac tttgaagcaa ggtcttgata
12481  ctctgggtat tcagactgta gaacgtatgt aataatcttc tacaaagctg aaaactggcg
12541  tgatattatt ttattacgcc agtttttcc tttcttctat tctgtcaaaa attaccaatc
```

Fig. 2(ii)

chrim5ed2.seq

```
12601   tgacatataa ttaatcttga gctaacattt tgatatttaa tcatagaaat atatgaacac
12661   agaagcagtt gttatgaaaa aattatttat ttctaactgc tagccctacc aatctccata
12721   aaaaaacctg atttttattc actattaata attaatgata atttctattt taattaacct
12781   tgttataaaa aatagtattt taaaaaaaca ttttacatta tataaaatat atcaatcgac
12841   tctttatttc tttatccatt tataaaatat attttttacc aaaataatat ttaaatcata
12901   tattatattt acatcacgtt agatcaaaat aacaattttt tagtcgttaa cccagattca
12961   gaacggcacg atgatatata agtcacatgg gttgtaaata aaggaaaaag ataaaaaaga
13021   ggagataaat cttcgcattt cttcaatgaa gatggatata gatactgtaa ggtagtaatt
13081   taaatgaaat ccattaacaa attaatattt aatttacatt aagagaggat tctatgagtg
13141   attggactgg tgtttcaaca tttaatgtta ttcttgaaac aggattagat aactgcaata
13201   tctacgctaa tgggcttaac atgattgggg taattattaa tatcacaccc actgatgatg
13261   aagggaactt cgtagatatt gacgatgtta cactaaatga taacatcaag attgttgatt
13321   atatcgatgg aagcgacatt gatggcagtg acggatggtt ttatacagga aatcctaatg
13381   aatacaacac tattccaaat agtcagtctt attctttatt aaagagtgaa aattctcaaa
13441   ttacgcaaat taaacgatat gtttcttgtt caaatacatc caggctaaga accaagtctt
13501   tttctgcgaa ggtaaccact accagtggaa aagttatttc aataactcaa aatagcatta
13561   attcatctcg ggtagtaatt aatgcaatag atgcaactaa ttttactgat gatgaacttc
13621   gaacaacaaa agaaacaagg tttgaaaatc aatcctatac gtcacataaa tcatctacaa
13681   actctttata tgtgcatacg tggacaatac caagaagctt aaaactacaa aattggcgtt
13741   gggaagatta caataatggc tggacttggg cacaaagttg ctactataaa acaggagccg
13801   atggaggatc agagtcaacc cgctggttgg ccgctggttc aatctttcca ccaggaaatt
13861   atgatggcct gtggctagat aatgatatcg cactaagtgg tatggcacac aaaagctaca
13921   atgttgatac tggtatcaat caattgagtt ttacccgtat tataggtaaa ggtttcagct
13981   gggtttataa tatatccgga cttgatagag ggcatgccgt tattattatc gaccagtatg
14041   gtaacaaata tagaatatta ttccatgccg ggtatgaaaa ctcagatcct tacttgtctt
14101   catcaatagt atattaaaaa tagtgttacc aatggtgtgg tagcatttcc ccaatacgaa
14161   acttgaccta gtggctgtaa ttataattta ttttcacagc cactttttaa gtatacataa
14221   aattccttaa gttattcagg agaatcacca tgagtgacaa taatgagttt tttactcaag
14281   ctaataattt caccagcgct gtcagtggtg gcgttgaccc tcgcacagga ttatacaata
14341   tacaaattac tttaggtcac attgttggta acggtaatct tggacctact ctgcctctta
14401   ccttaagcta ttctcctctt aacaaaacag atattggatt tggcattggt tttaattttg
14461   gattatcagt ctacgacaga aaaaattctt tattgtctct ttctaccggt gaaaattata
14521   aagtcatcga aaccgataaa acagtaaaac ttcagcaaaa aaaactcgac aatttacgct
14581   ttgaaaaaga cctaaaagaa aattgttatc gtattataca taaatccggt gatattgaag
14641   tgttaactgg tttcaataac aatgcctttg acctgaaagt ccctaaaaaa ctattaaacc
14701   ctgctggaca tgctatctat attgattgga attttgaggc aactcaacct aggctaaatc
14761   gtatttatga tgatctggat gggcatgata taccattatt aaacctagaa tatcaaggac
14821   taattaaaac gatattaacg cttttccctg ggcaaaagga aggctaccgt accgagctac
14881   gctttctaaa cagacaattg aacagcatcc acaactttag cttgggtaat gaaaaccctc
14941   tcacttggtc cttcggttat acccctatag gaaaaaatgg tattttgggg caatggataa
15001   caagtatgac cgctcctgga ggattaaaag aaacggttaa ttatagtaat aataatcagg
15061   ggcatcattt cccccaatca gccaatctac cggtgttgcc ctatgtcaca ttaatgaagc
15121   aggttcctgg agcaggacaa cccgctatac aagcagaata ttcgtatacc tctcataatt
15181   atgtcggtgg gggatctaat ggtatatgga ataataaatt agataatctg tatggattga
15241   tgacagaata taattatggc tctactgaat cccgcagata taaagataaa gaaggccatg
15301   atcaaatagt ccgtatagaa cgcacataca ataattacca tctgttaact tccgaatgta
15361   agcaacaaaa tggatatata cagacaactg agacagcata ttatgctatt attggccata
15421   attttgattc tcagccctca caattccagt tgccaaaaac caaaacagaa acttggcgta
15481   gtgcagataa cagctatcga agtgaaatta ctgaaaccac atttgatgaa agcggaaacc
15541   ccctaaccaa agtaatcaaa gataagaaaa cacaaaaaat aatctccccc tcaacgcatt
15601   gggaatacta ccctccggct ggggaggtcg ataattgccc accagaaccg tatggattta
15661   ctcgtttcgt aaaaaaaatc atacaaactc cctatgactc cgaatttaaa gatgatccgg
15721   agaaatttat ccagtatcgt tatagcctca ttggcagtca gagtcatgtg actttaaaaa
15781   tagaagagcg ccactacagt gcaactcaac ttctgaatag tactctattt caatataata
15841   cggataaaag tgaacttggt cgtttattaa aacaaactga atgtaccaaa ggagaaaatg
15901   gaaaaactta ttctgtcgtg cataaattta cctatacaaa acaggacgac acgctgcaac
15961   agagccattc cataaccacc catgataatt tcacaattca ccgcagtcag gttcgttccc
16021   gttataccgg gcgtctgttt tctgacacag atactaaaga cattgtaact caaatgtcct
16081   atgacaaatt gggtcgatta ctcacacgca cccttaattc cggtacacca tatgccaaca
16141   ctctgacata tgattatgaa ctaaataatc ttcaggatga caatcgccct ccgtttgtta
16201   ttaccaccac ggatgtaaat ggcaatcagc ttcgcaatga attcgacggt gccggacggc
16261   atgtcagcca atgcctgaaa gactccgatg gtgatggaaa attctatacg atacatacgc
16321   aacaatatga tgaacaaggg cgtcatcata catctacata ctccgactat ctcacaaatg
16381   gaagacaaca gacggatcct gataaggtgc atctgtctat gtcaaaatcc tatgataatt
16441   gggggcaaat tgcgaacaca cactggagtt atggggtttc agaaaaaata actgtagatc
16501   cgataacatt gacggccacc aaacagttac aaagcaatag caataatgtg caaacgggta
16561   aagaggttac aacttatacg ccaagtcaac aacctataca gattacgtta tttgacgaag
16621   caggccattt acagagttgt catacccctga ctcgggatgg ctgggatagg gttcgcaaag
16681   aaaccgatgc aataggccaa tgcactattt accaatatga taactataac cgagtcattc
16741   aaataacgct tcctgatggc accatcgtaa atcgcaaata tgcacccttt agtactgata
16801   cgctgataac agatattcga gtgaatggaa tttccttggg acagcaaacg tttgacgggt
16861   tgagtcgatt aacacaaagt caagatgggg gacgagtatg ggcttatact tattcggcag
```

Fig. 2(iii)

chrim5ed2.seq

```
16921   gtaatgacca atgcccatca acagtaataa caccagatgg tcagtttatc cattatcaat
16981   atcagccaga attagatgat gcagtattac aagtagcatc aaatgaaatt actcagcagt
17041   tcagctataa cccagtcact ggggcattat taaaggcggt ggcagaggga caaagcttga
17101   cccctatcta ttatccatcg ggaagactta agatggaaaa tatcaatgat atgaaaaaaa
17161   tgagttacct atggacactt aggggtctgg agaacggtta cactgatctg actggaacaa
17221   tacagaaaat ttcgcgtgat acccatggca gggtgacaca aattaaagat tcgtcaataa
17281   agactactct aaattacgat gacctgaatc gccatattgg tagtcaagta acagatttag
17341   cgactggtca tatgttgaca acaacagtgg aatttgatgg cttaaaccga gaaattggac
17401   ggaaattgtg tgatagctca ggccatacgt tagatatcca gcagagctgg ctgaaaacac
17461   agcaattagc aaatagaata gtgaaactga atggagtatt gcagcgtaca gaacagtact
17521   cttacgattc ccgtaatagg ttgaaccaat ataaatgtga cggtgcggaa tgcccgacag
17581   acaaatatgg ccatagcata gtcacacaaa attttactta tgatatctat ggcaatatca
17641   ccgcctgtca caccacattc gcagatggga cagaagacca tgctaccttc aaatttgcca
17701   acccaactga cccatgccaa ctgacagagg tacaccacac tcatccagat atgccggata
17761   atatcaggct gaaatatgat aaggctggta gagtaataaa tatcactgat aaccatggaa
17821   atacggaaaa ctttacctac gatacattgg gcagattaca aaacggtcaa ggtagtgttt
17881   atggttatga tccattaaat cgcttagtga gtcagaaaac agatacccta gattgtgagc
17941   tgtactatcg ggaaaccatg ttggtcaatg aagtacgcaa tggagaaatg atccgtttat
18001   tacggacggg tgaaacaata atcgcacagc aacgcgcatc aaaagtcttg ctaacaggaa
18061   cagatagcca acagagcgtg atattaacga gtgataaaca aaacttgtct caagaagcat
18121   atagtgcata tggaaagcat aaatctacag caaatgacgc ttctatcctg ggctataatg
18181   gtgaacgcgc tgacccagtt agtggagtaa cacatttagg taatggttac cgctcctatg
18241   atccaacatt aatgcgcttc catactccag atagcttaag cccctttggt gctggaggga
18301   ttaatcccta ttcctattgc ttaggagacc caattaatcg ctcagaccct tctggtcatt
18361   tgagttggca agcatggaca gggattggca tggggatcgc tggattactg ctgaccatag
18421   cgacaggtgg aatggcaatt gcagcagcgg gaggtattgc ggcggcaatt gcttccacct
18481   ccacaactgc actggcattt ggggcactga gtgttacatc ggatataacg tctattgtta
18541   gcggtgcact ggaagatgct tcaccgaagg catcttctat actcggatgg gtttcaatgg
18601   gaatgggtgc tgccgggtta gctgaatcgg ccattaaagg tggcaccaaa cttgcgacac
18661   atctaggagc attcgctgag gacggggaaa acgccttact taaatcgact tccgaaagtt
18721   ctagaataaa gtggggagtg acaagaagct tagatagaga aattgttcgc aatgaagaag
18781   gtcaggtgat aaaagatcat agccgaggtt ataccgataa ctttatgggg aaaggagagc
18841   aggctatatt agttcatgga gataaagatg gatttttgta tcatacagaa ggaaacaaac
18901   ataatggaaa agggccatac actcgacata ctcctgaaca actcgttgat tatttgaaag
18961   acaataacat cgttgatctt acacaaggag gagacaaacc tgttcattta ttatcctgct
19021   atggaaaaag cagcggtgca gcagataaaa tggcaaaata tatcaacagg ccagttatcg
19081   cttattctaa taaaccaaca atatcacaag gattagccag aatagaaaga aaggactttt
19141   tcttaaaaag tacttaccat tcgtatgatc cacggaagat catactggga agaacagaaa
19201   aaacagtgaa accaaaaact tttcgcccct aataaccttg caaaattcaa aggtactggc
19261   agaagtcata taaataactc tatgactggg taaatgaaat cagtattcaa acattaaaca
19321   ggatggaagt ggtcatctta tcaaccgccc aataaaaaat tgggcggttg tattgaataa
19381   aattatttat taatcaggga atcactgcaa tccccgatga gcaaaatctt tcagtctaaa
19441   tcccatcaag gccagaactg caaaataact gcctgctccc gcaatcacta cgcccattaa
19501   gcgcagtagg cgcattgcca tattgccctg ttcccatgct ggcataaccc acaatactgc
19561   caacagcacc ccgaccatca cagcaattgc caccaccaat ttaaacagga atatccccca
19621   tcccgccaaa ggcgtgaaaa tatcacgctt acgcagttgc caataaagca tactggcatt
19681   gaagcaggca gccagaccaa tagaaagcgc cagacctgca tgtttcaacg ggccaacgaa
19741   agcaaggttc atcaattggg tcagaatcaa ggtcgcgatc gcaattttta ctggtgtttt
19801   gatatcttga cgtgaataaa agcccggagc gagaacttta accacaatca accccatcaa
19861   gccaaaacag taggcaatta acgcccgctg agtcatctca gcatcaaaag cagaaaagtg
19921   accatattga aataatgata ccgtcagagg ctccgcgaga ataccgagag caactgcaca
19981   aggcaacgcc agcaagaaac agagacgtag cccccaatcc atcagttttc gatattcttc
20041   gtgattacca ctggaaaaac ttttcgccag tgaaggcagc aaaatcgtcc ctaacgccac
20101   acccagtaca ccagaaggca attccattaa acgatcagcg taatacatcc atgaaacaga
20161   gcctgaaacc agaaatgagg caaaaattgt attaatgatc aaggaaatct gcccgaccga
20221   tacacccaga attgcaggcc ccatttgacg gataacccgc catacggcac tgtcacggaa
20281   agaaatccgc ggcaatacca gcatgccgat ctttttcaga tgaggaagct gataggccaa
20341   ttgcaaaacc cctccggcaa caacggccca acccagcgcc agcactggcg gattgcaata
20401   aggagccaca aacaatgcaa aaatgatcat actgacattg agcagtgtcg gagcaaaagc
20461   aggcaccgaa aagcggttcc atgtattaag aattgcgcca gccaaagaag ccagcgaaat
20521   caaaaagata taaggaaacg taattctaag taaatcacgg gttaagacaa acttatccgg
20581   tgtatccgta aatcccggcg cagtcacata gatgatccaa ggtgcagcaa ttacacctat
20641   cactgagacg atagccagaa tcaatgtcaa catacctgag atatatgcaa taaaggtacg
20701   tgttgcttca tccccttgtt gatttttgta ttcggcaaga ataggaacaa aagcttgcga
20761   aaaagcgccc tctgcaaaga tacggcgtaa caggttaggt aatttaaagg caacaaaaaa
20821   ggcatccgtc gccattcctg caccaaatat acgagcaata atggcatcac gaataaagcc
20881   cagcacgcga gaaaacatcg tcattgaact gaccgctgcc agtgatttca ataagttcat
20941   ggtattgttc taaagttgta ttcttatgga attaagcata aaaatgtaaa gctatcccat
21001   caggcatcat aaaaatggca tataaagcaa tctggcggga tagcagccgg tgtttaaagt
21061   ctaacagaca aaaaccctgt ctacattttc tatattacgc cccattagcc ttaccccgag
21121   attcataaac ccactttgcc ccatgcatcg tcagtcaatg ccgtcagcac atgatcttgc
21181   caaacaccat ctatcattaa ataattcttg gcataacctt cctgctcaaa tcccaacttt
```

Fig. 2(iv)

```
chrim5ed2.seq
21241  ttgagcaggt tcccactacg atggttatgt ggcatgtaat tagccataat ccggtgcatt
21301  ctctgatagc gctgcatata gcggatcgca ggttgcagcg cttcatacat caatccttgc
21361  ccttgcaatt tctcagctaa agaataacca agataacaag agtggaacgc gccgcgtaca
21421  acattagtaa aattcgccac acccataatt tcacgctcat cagagtccaa taatacaaaa
21481  ttaaatgtcg cattctgccg ttgtaactca gcgatatagt tcaaccgatt tgtccatcca
21541  gagggttgat aaaaactgcc gtcccttgtt ggctcccatg gcttcaggaa atctttattt
21601  tctgaataat actcagccaa tcgataagca tcacgttcat ataccagacg aacaaccatc
21661  ctatccgtaa taaatcgaat tttgggcgat gcagaacgat aaccaaacat gccttctcct
21721  gatatttgtc ttgaaaaaat tctggataat ttatctctta ttaaaattat tgcctattac
21781  cctacgataa aaaaatatca tctataaccc tctaccttaa agatgagatt agggtcagaa
21841  taataagaac ttcatattta attctctcat atttagtgg tgcgcaatgt cgttggtttc
21901  acaagcacgt agcttgggta aatacttcct gctatttgat aatttattag ttgttttagg
21961  ttttttgtc gtttttcccc taatttcaat tcgtttcgtc gaacaacttg gctgggcggc
22021  attgattgtt ggtttcgctc tggggcttcg tcagcttgtt cagcaaggct tggggatctt
22081  cggtggcgcc atcgcagacc gatttggtgc caagccgatg attgttactg gcatgttatt
22141  gcgagcactg ggttttgctc tgatggcaat ggcacatgag ccatggatat tgctgctttc
22201  ctgcgttcta tcaggattgg gaggaacatt gtttgatccc cccagagcgg ctttggtcat
22261  taagttaacc cgtccccatg agcgagggcg tttttattca atcctgatga tgcaggacag
22321  cgcaggtgcc gtggttggcg cactcatcgg aagctggttg ctgcaatatg atttcaatat
22381  cgtctgctgg attggtgcat ccatttttgt gctggccgca ttatttaacg cctggctact
22441  gcctgcatac cgtatttcaa caatccgtac tcctatcaaa gaaggcatga tgcgggttat
22501  tagagatcgt cggttccttt actatgtgct gacattgacg ggttattttg tcctgtcagt
22561  acaagtgatg ctgatgtttc cgattattat ccatgaaatt accggcactc ctactgccgt
22621  caaatggatg tatgccattg aaaccgctat ctccctgaca ttgctctatc ccatcgcacg
22681  ctggagtgaa aaacatttcc gactggagca gcgcttgatg gcgggcttat ttttgatgag
22741  catctgcatg tttccgatcg gctgggtcaa tcaattacat acactgtttg gcctgctttg
22801  cctgttttat ttaggtttgg taacagccga tcctgctcgt gaaacgctga gtgcttcact
22861  gtctgatcca cgggcgcgtg gcagttatat gggatttagc cgtctgggtt tagctctagg
22921  tggtgcgata ggttacaccg gtggagggtg gctctatgat actggccgtg acttgaatat
22981  gccgcaatta ccctggattt tgctaggatt gtctggtttg attaccattt atgctcttca
23041  tcgccaattc aatcagaaga aaattgatcc tgtgatgctt ggtagacatt aatcctatta
23101  gaaatagtgt aaaatctgcc cattgaaaat aaaaaggagc caatatgaaa agatttttct
23161  taggggcagc attagtgctg gtaggattag tcagcggctg tgatcaattt aaagacttca
23221  gcatcaacga aggtctgatg aatgattatt tgctcaaaaa agtgcattat cagaaaaaaa
23281  tcagcattcc aggtatcgcg aatgccaata tcacgctggg ggatttatcc agccagatag
23341  gccgccagga tcctgaaaaa attgaactat ccacgcaagc aaaagttcaa cttgcaacac
23401  tactgggtac gattcaggct gatatgaaac tcactatcaa ggctaaaccc gtatttgatg
23461  cagaaaaagg cgctattttc gtgaaagggc tggaaatcgt agactaccag acaacaccgg
23521  aaaaagcagc ggctccggtt aaggcattga ttccttatct gaatacctct ttgagtgagt
23581  ttttcgatac tcatccggtt tatgttctga atccagaaaa aagcaaggcc gaggcagcag
23641  cctcacaatt cgctaaaagg ttggaaatta aacccgggaa gttagttatt gggttgaccg
23701  ataaataatt tttatcgtca ttgaaataat aagggcaaat tattaatgga taatttgccc
23761  ttttatttta tcaattagtg accgatacac tcgcctgttc atggccttcg gctgaactat
23821  tatcgtgaca ctgtcactca gattctaatt catcacgcaa tgcctgcaac gcttcacgag
23881  tgacaatcgc cagcgttcga taaaaaccac tggttgcatg ggcttcaact ttaccgagga
23941  actgcccaca ccaaggcaaa agatactcat caaacaacgt aatttgtgct tggatttcat
24001  cttcagctgc ctgatcttcc agccatgaca cagccagcaa tagagagccg aactgatcag
24061  caggagtatc agacagtggc attccccgtt ctgtcaaaaa ctgacgtact tcactttctt
24121  ctccgtccgt ataatcagat cgacaaacag caacactcgc cgattctcca gtaaacagag
24181  cgtgataatc ggctgccatc actgacaatt cactgttttg ctgtaaacga tccagccatt
24241  catcctgttc caaaggccat gcttgtttta gtttcccctc agcaatcatg gtgattaaag
24301  gttgcaaaac gggatcttgc ggtgcgcggt taaacaatgt gcccaatatg cggcagacaa
24361  tagaaaattc gttcataact tgattcaatc ccaataaatt atcaaatagc gttattgtcc
24421  ctaatcgcct gccatgctgt aaagcaactt gttaatagcc acgtacccta tcgacgaggc
24481  cagttggcat ttcaccctgt accatccgcc ggatattttc acagatgaca tccatcgcag
24541  cctcaggaat agtgttagca gcaatatggg gagtcacatt aatacgaggg tgagtccaga
24601  atgggtgcat atttgacaat ggttcttcgg caaacacatc aagggttgct cctgcgatat
24661  aacctttatc aatagcaacg agcagatcct gttcgacaag ctgcgctcct cgcgccagat
24721  taatcacata tgaacccgat tttagttggc tgaataatga cagattaagg ataccacgag
24781  tgtcgggagt atcagggaga aggttaatca gcaccttgca gccagaaaga aaatcaccca
24841  attgctcttt accgtaaaaa ctttcgacac tatcgagttg ttttgacgtc ctgctccaac
24901  aacgcacatt aaaatcaaat tccattaatt tcccaatcac acttcgccct aatattccgg
24961  cccctaaaac gccgataaca aattcttttc ggttatgcgg agcaataggg ttccaaaggc
25021  gctgttcttg gtatcgttta tattcatcca tgcggcggaa ataatgcagc actgaggtaa
25081  tggcatattc ctgcatctgc cttcccattc ctgtatcttc gaggcggatc aatggaacat
25141  cagccagtaa cgtaccggga tttttcgatt cttgtttgaa aatcgcatcg acaccggcac
25201  ccagtgcgaa tatgcctttt agcccctgac gattggctaa catttcataa ggtggctgcc
25261  aaactaatgc atagtctgcg ggctcttgat caccactgac ccattgcctc actttggcat
25321  caggcagacg tgcctgaatt ccttgaatcc actcatcggt attaaaggaa gggtgaaaaa
25381  aaataatgtt catacaatct tattccttat tgttttttg atagggttaa cctattgcta
25441  aaatggttgc aagcctctgc tggaaagcga ggaatgaaaa tattaataat gttacccagt
25501  taacatattt accactgcat attacaaaaa gcgcatcggc tttagtaaat gactatcgaa
```

Fig. 2(v)

```
chrim5ed2.seq
25561   tattcaatt gttttttatt tgtgtaatca gtcaaaagc ctgaaaaat cgtcataagc
25621   ctgttgacgc ctgccctgct tttccctata gtagcgcccc gttgcagcga cgaactcaag
25681   tgatatcgct acaacaacaa aatacggtga ggtgtccgag aggctgaagg agcacgcctg
25741   gaaagtgtgt atacgtgaaa acgtatcgag ggttcgaacc cctctctcac cgccatattc
25801   taagaaagag cctgaacaca atactaaggc tttttgtgg ttactcttga tagagcattg
25861   aatctataat ttagtaaccc ttgcggaaaa tccctgacag gacgaccgca gaaacaaaac
25921   acggtgaggt gtccgagagg ctgaaggagc acgcctggaa agtgtgtata cgtgaaaacg
25981   tatcgagggt tcgaacccct ctctcaccgc catctttcaa gagaaagcct gaacttatgt
26041   tcaggctttt tcgcatttat actccccaaa gtataggtga aaaacctcgc aagggttcac
26101   ctcaacaacc tgctctaatg ggaatgtctt aaagatttgt ggcttaatta caccttgttc
26161   tagcctaatc caaaggacat acccgaataa tatgaccatc gggatcttca gcaaggaaag
26221   tacggccaaa aacttcggta tggggttcct gtactatttt gatctcaggg ttctttcgcc
26281   attcttcaaa gcaccgatca acatctttac cggatggcag cataatacca atttcagaaa
26341   atcgcggaat agcacgatca ggctttgctc ctccactcca aatagcaaac agtacttcac
26401   caccagcagg aaatgccaca taacgggagc tggcaaatat cggttcagca ttgaaaattg
26461   ttttataaaa agcggttgaa ctctcgatat cagagacgta aacaagctga agattgggtt
26521   tgggagatac tgtctcaacg acagatttca gccttaatac gctttcaacc agatcttgcg
26581   taccttgctt aaagaaactt tcggcctgaa cagcgggaat atttgcatca ggttcaaatg
26641   aaacggtcac ctgaacacga cacgccatat tgttttcatc tgtaataatt tgatgtcgga
26701   atgttaatat tcccatttga ggaaggttaa cctgatcggt gaattcttta ttcacttcga
26761   tatttgaaag ataaaaacga atttccggca ttccacttaa tatcattctg ccgtattgac
26821   ccgttttgac ctccccttca aattgaaaat attcaagatc gacttcccat tttttccgca
26881   aatcaaaatc gacatagtgc gcccagatat gggatggttt agcgtttacc gatgtagaaa
26941   aattgagtgt taacatgaca aaactctcac agttaaatgt atcacattga agattaagcg
27001   agctttttaa tacagtagaa cagcccgcag agtatcaatg gtgagtgaca atttctgtca
27061   gtagtcttta tttggctaac gagaaatttt ttccattgct ctccattctt tgagcaatac
27121   ttgccttgaa cgggggtaac attggttttc aattttcaaa cgcatgattc tatctgatct
27181   gaaatgacga aattcctcgc gtaattcaca ccatccaatc acaatgctga tattttcaaa
27241   atagcctaag gcaaatggcc atattgttct ttctgatggg atgtctttta tatccaaata
27301   agcgagggtg attttatgcc gggtattgat cgcctgacgt atctgctgaa tctcgacaac
27361   aggctgaaca gctgtcgcag caggcccgat cagtaaggag cttgcctcca acatttgttt
27421   caattctgct gggatcacag ccgcaatttt gcttattgca ttatttgcag attcttttag
27481   ttgggggtct gcacgtttag ccacccaatt cgcgcccaat gccaacgcct ctatttcatt
27541   ttgtgtaaac atgagcggtg gtaacacaaa tccaggcctc aaaacgtatc ctattcccgg
27601   ctcaccttcg ataatcgcgc cttgagcctg caacgatgca atatcccgat acagtgttct
27661   taagctgata ttcaatttct gcgccaacac ttttccctga accggaaagt gataacggcg
27721   caatatttcc atgagaaata acaaacgctg tgctctagac aattcgtccc atccattcaa
27781   gttaaccgac tcatcaacct ctaataatac gcaactatca taatttaatt gattaaaaag
27841   atagtttttt gatcccttgt acaagatcat tgttatctga ttgccctttt agattttta
27901   ttttattaat aatgctgata aattgacctc taaaggactt agagaaaaat gaccatatac
27961   gatttaaaac cccgtttcca aaacttactg cgtcctatcg taatttatct gtataaacaa
28021   gggatcaccg caaatcaggt cactttaacc gcgctgttcc tgtcaatctt tgccggttca
28081   ctattgagcc tatttccctc gccccacctc tattggttgc tgcctgtttt tcttttcatt
28141   cgcatggctc tgaatgccat tgatggcatg ctggcacggg aacataacca gaagtctcat
28201   ctgggcgcta tttataatga attgggggat gtcatttctg atgttgccct ctacctcccc
28261   ttctgccttt tacctgatgt gaacagcctc agcctgttga ttattttatt cctcactatc
28321   ttgaccgaat tcatcggcgt actggcacaa acgattggtg catcacggcg ctatgacggc
28381   ccgataggaa aaagtgaccg tgcttttatc ttcggagctt atggattgat tattgcgatt
28441   ttccctttgg ccttgggctg gagtatctct ttgtttgctt tcatgatcat tttactcttg
28501   gtgacttgct atcagcgcgt tgttaaagcc ttacgtgaaa tccggctggc tgaacagtca
28561   cactccaaat gaggcgttaa catgacacca caactcgatc aacgtattgc tgaagaacat
28621   tatttcacca catcagataa tgcttctctg ttttaccgtt actggccaca acaacaggcc
28681   aatccagaca gagcgatcat tatttttcac cgtggtcatg agcactcagg acgtatccag
28741   catgtcgttg acggactcga tctgcctgat gttcctatgt tcgcgtggga tgcccgtgga
28801   cacggtaaga cagaagggcc gcgcggttac agcccatcca tgggaacgtc gattcgtgat
28861   gttgatgaat ttgtcagatt tattgccact cagtacggca tcgccatgga aaatatcgtg
28921   gttatcggcc agagtgtcgg agcggtatta gtctctgctt gggtacacga ctatgcgcca
28981   aaaatccgcg ccatgatcct cgcagcaccc gcatttgata ttaaattgta tatccctttt
29041   gccacgcagg gactgcaatt gatgcaaaaa gcacgaggta ttttcttcgt gaattcctat
29101   gtgaaagcca gatatctgac tcacgatgaa acccgaattg cctcttataa tagcgatccg
29161   ttgattaccc gggaaatcgc cgtcaatatt ctcttggatc tttaccaaac cgccgagcga
29221   gtagttaaag atgccgccgc cattacacta cctaccctgt tgtttatttc aggcagcgat
29281   tatgtagtga acaaaaaacc acagcatcag ttttatcagc agctaaatac ccctatcaaa
29341   gaaaaacatg tgatggatgg cttctaccac gatacgttgg gtgaaaaaga tcgccatctg
29401   gtttttgaca aaatccgggt ctttattgag cgcatttttg cacttccgcg ttatcagcac
29461   gattacagcc aagaagatac ctggagtcac tctgccgatg aatttcgaac attaagcaca
29521   tcattaccgt gtctgtgtcc taagaaactc agctatcaat tgatgcgtaa ggtaatgagt
29581   actcactggg gcagaacttc cgagggtgtc tgcatcggtc tcaaaacggg gtttgattcc
29641   ggctccacat tagattatgt ctaccgcaac caaccgcagg gtaagggcat tttggggcga
29701   atactcgata agcattattt gaacagcatt ggttggcgcg gtatacgcca gcgcaagatc
29761   catattgaaa tgttgatccg ccatgctatt cgcagtctac gtgaacagaa tatgcctgtg
29821   catatggttg atatcgccgc cggacacgga cgctatattc ttgacgcaat caacgatttc
```

Fig. 2(vi)

```
chrim5ed2.seq
29881   agcaaagtcg attctatttt gttaagggac tatagcgaaa tcaatgttaa tcaagggcag
29941   gcttatattg aggagcgcga tctgacggac aaaattcgtt ttattatcgg tgatgccttt
30001   aatgctgaaa gcatctcatc cattacgcca gcgccgacac tgggtattgt atccggtctc
30061   tatgaattgt tccctgataa taatttactc agaaattcgc tacgcggctt tgctgatgtt
30121   atgacagaaa atggttatct ggtgtacacc ggccaaccgt ggcatccaca aattgaggtc
30181   atcgcccgtg ttctttccag ccatcgtgac agtcaaccgt ggatcatgcg gcgccgtact
30241   caaggggaaa tggacgcatt agtggaagcc gccgggtttg aaaaactgta ccaactgaca
30301   gataactggg gcattttcac tgtttcgatt gccaagcgtg ttcatcgctg atgaataaat
30361   aaatataaga tggaacacca catgcactct tctctcgata gtcgtcggcg cctatggctg
30421   acaggtgtta tctggctatt gtttctggct ccgtttttct ttcttactta tggccaggtc
30481   aatcagttca cggcacaaag aagcgatgtc ggcactgtga tgttcggttg ggaacataac
30541   atcccttttt ggtcatggtc gattatccct tactggagta tcgatctgtt ctacggaata
30601   tcgttattta tctgtaccca tcgccgtgaa cagtggcttc acggctggcg attaatgacc
30661   gcatcactga ttgcctgtgt tggattctta ctgttccctc tgaaattttc gttctcccgc
30721   cccaccacag aaggcctatt tggctggtta tttaatcaac tggagttatt tgatctgccc
30781   tataatcaag ccccttccct gcacattatt ctgctgtggt tgctctggct gcgctattca
30841   gcctacgtga gtggttactg gcgtgggttg ctgcacattt ggtcagtgct gattgcactc
30901   tcggttctga cgacttggca gcaccatttt atcgatgtac taacgggttt tgccgttggt
30961   gtcatcctca gttacctact gccggtttca taccgctggc gctggcaacc taatcaagat
31021   cgctatgcac ggaagttatt cggctattat ctgacaggca gcgctttgtt cgcgcttata
31081   gcgagtctgc tggggggag tttctggata ctgctgtggc ctgctgtatc gttactgatg
31141   atcgcactgg gctacgcagg attaggcagc tccgtgtttc aaaaacagcc agatggccgg
31201   atgtcactgt ctgcacgctg gctactggcg ccataccaac tgggagcatg gctctcttat
31261   ctctggttcc ggcgtaaaag cgcacctttc aaccatataa ctgaagggat tattctcggc
31321   agcctgcctt gccagcccgt tacggcggtc agtgtccttg atataaccgc tgagtggcac
31381   aggcgatcgg atgcccgcac agtaaattat gtttgccagc cgcaaatcga cttactgccg
31441   ctggcacctg aagctctaca atcggcagtt tgtacgctgg ataaactacg ccagcaggga
31501   gatgttttcg ttcattgtac gcttggactg tcacgcagtg cgatggtggt agcagcatgg
31561   ctactgaaac agcatcctga atatgatatc aacactgtcg tagcaatcct gcgtaaagcc
31621   agaccgcatg tcacgttcag acaaacacat ctggatgccc tgtctcaatg ggcaaaaggc
31681   tacctataac ggggaacata acatgcagcc ggaaaatctg atcagcaaag tgattatcgc
31741   aacgttaaaa agctggcgct ttatatccac actatctgct ttttctatcc tgatcgcgac
31801   tgcaatgctg attgctgtct tcaacactac cgccttaaac aacattgcac tctatgccgt
31861   actattattc acaacgctgt actgccaata ctattgttgg cgcacttggc ttgactgcca
31921   ctattttcag atcctcaatt catcccctga aaaaagcgcc gagttcgatc aaacgttatt
31981   gctgatattt aacaagttac cccaatcacg gacacagaat gatcgcttta acggagcaat
32041   caaactgtta aaaaaggcta cgattggtct gatcctgcaa tggatactgt ttttcctgtt
32101   tctgcttact ttgaaatatt cagcctgaat tcagctttaa tctgacaaag tcagaacgta
32161   acgatgctac attttctcga ctgtactact attaactaac ttttagacct gtaacacctt
32221   tattgggtga taaaagagat actcttacat attctttttc agcccgtcat gcggacgcct
32281   tattttttgg ttttagatta aatgaatact cgtaaaatta atgggatacg cccatttagt
32341   gcgtttattg actcatgtct gaaggaatct tactctttcc cccgttttat cagagatatc
32401   atcgctggga ttaccgtggg tgttatcgct atcccattgg caatggctct ggcgatagga
32461   agcggagttg caccacagta tggcctgtat actgccgcta tcgccggcat tgtcatcgcc
32521   atgaccggag gctcacgtta tagcgtttcc ggcccaacgg cggcttttgt ggtgatcctt
32581   tatcctgttt cacagcagtt tggtttgagc ggcctgctca ttgcgacgct gatgtcaggt
32641   gtgattttga ttgtgatggg attggcacgt tttggccggc ttatcgaata tattcctatg
32701   tctgttaccc ttggatttac ttccggtatt gccatcacta tcgctaccat gcaggtgcaa
32761   aatttctttg gcctgaaact ggcacatata ccggaaaatt atattgataa ggtagttgca
32821   ctttatcaag ccctgccttc attacaattg agtgatacgc ttatcgggct gactacgctt
32881   ctggtactga ttttctggcc gaaactggga gtaaagttac cgggtcactt acccgctttg
32941   atcgcgggta cagctgttat gggcgcaatg catctgctga accatgatgt cgccaccatt
33001   ggttcatcgt ttagttacac actggcggat ggtacgcagg gtcaaggcat tcccccccatt
33061   cttccccaat ttgtcctgcc gtggaatttg cccgatacac attctctcga tattagctgg
33121   aataccgtat cggcattgct gcccgctgcg ttttcgatgg ccatgctggg tgcgattgaa
33181   tcgttactgt gtgcagtgat tctggatggt atgacaggga aaaaacacca ttctaacggt
33241   gagctgcttg gccaggggtt gggcaatatt gccgcccctt tctttggtgg cattaccgcc
33301   actgcggcta tcgcccgttc agccgccaat gtgcgggcag gtgcaacttc cccgatagcc
33361   gctgtggtgc attccctgct ggtattatta acattgctgg ttttggctcc gatgctctct
33421   tatttaccac tggcggcaat gtcagccatt ctgctgattg tcgcgtggaa tatgagcgag
33481   gcacataagg tagtggattt aatacgccat gcgcctaaag atgacattat tgtcatgctt
33541   ttgtgtctgt cattgactgt cctattcgat atggtccgtc gcgatcacta tcggcattgt
33601   gctggcatca ctcctgttta tgcgcaaaat tgccaatatg actcgaatca gcacgtcatc
33661   tttaacaagc gcggagaaag ggttattggt cgtacgaatt aacggccctt tattcttcgc
33721   tgccgccgaa cgtatttttg ccgaactgag agaaaaaagt gctgattatc aaaccatcat
33781   catgcagtgg gatgccgtac ccgttttgga cgctggcgga ttacacgctt ttcagggttt
33841   tgtgcgcgaa cttggcaaag aaaaacatat cgtcgtatgt gatattccct tccagccatt
33901   gaaaacgctg gcaagagcca aagttatgcc gattgaagga gagctgagtt tttatgctac
33961   cttacccaag gcactaaaag agatggcagt tgattacacg cctgaggtgt gtgcttcttc
34021   agaaaagatt caaggtcagt aacaacaagt caccctctgc cagttatgct ggcagagggt
34081   gactaataga agactagcgt aggaatttat ttgctggtat ccagttccgg gaaatttttc
34141   accacgtcat caatcgcttt aatttgcatc aggaatgact ccagttttgc caatggcaaa
```

Fig. 2(vii)

chrim5ed2.seq

```
34201   gcagacgggc catcacattt ggcattttct ggatctgggt gcgcttcaag gaacagacct
34261   gcgatcccca cagccatacc cgcacgagcc agttcagcaa cctgcgcgcg acggccaccg
34321   gaagctgcac ctaatgggtc gcggcactgt aatgcatgag tgacgtcgaa aatgaccggc
34381   gcaccttggg tagcttgttg catgacaccg aagcccagca tgtcaaccac cagattgtca
34441   taaccaaagt tgctgccacg gtcacacagg atcacctggt cattgccgcc ttctttgaat
34501   ttttcaacaa tattgcccat ttgcccaggg ctaacaaact gtggtttttt aacattaatc
34561   acagcaccgg ttttcgccat cgcttcaacc agatcggtct ggcgggcaag gaaggctgga
34621   agctgaatca catccaccac atccgcgact ggttgagcct gtgcaggctc atgcacgtca
34681   gtgataattt tcacgccgaa ggtttgtttc agttcctgaa agattttcat cccctcttcc
34741   agccctggcc cacgataaga gcggatagaa gaacggttag ccttatcaaa agacgcttta
34801   aaaacataag gaatgcccag tttttgtgtc acggtgacat agtgctcaca aatccgcatg
34861   gccaaatccc gtgattcaag gacgttcatt ccaccaaaca atacaaatgg cagatcattt
34921   gcgaccttga tatcaccaat attaaccact ttctgttgca tgcttatacc ttctcttgtt
34981   aagggcaaat aaatttgctg tatcaataaa aaaataaacc taatgcagga caatcaaccg
35041   ctgttctatg gtgttaattt gcatttttat catttctgag attgggtcct cagggcaatg
35101   ttcgacaaaa taactcaaat ccgaaacagc aatatgattg cagtcgagct gggcatagat
35161   aagcccccga tcgcggattt catagggatc atcgggatca aacgtcagca ccacttcgct
35221   ggccttaagc gccagttcca tctttttctc ttccatcaga gaaactttga tggtgtcagt
35281   gattttgcgc acgaggctga cattatcggc ttcctgcaaa tcctgttttt tcagacgaac
35341   agttgggcca atattacctt ttaaccagac atccagcgta tgttcattca gcgtatcccc
35401   attcaaggga ttaatgaacc aagtgggctg atcaagcaaa tcaatccgca atatcagttg
35461   gattggaaat atgacaggct gtaccgacag ccccagcgcc tgagcaatat gcgtaaacac
35521   cgtacccaat gatacaggtg agccttgacg tgaatggagt aagcgatcca gccacagggt
35581   atctgataga caatacaccc cattagctcc accaaacttc cattcccgat aaaaaagtgt
35641   tagcagcgaa tccaatttca ctttggaatc ggtaatggag gaaagcctct gccgggcttc
35701   ttcaaccaat gcagttagct gctcactcac cagagtctga ggaaaatcag ggcggataat
35761   ttttgatacc agaataatac cgtcaatcag ggagagtatta ttgaattcat aattaactat
35821   gggtttcatt tttattccat cgacctatcg tgatgcgttc attaccgcca taatcctgaa
35881   aagtcgctat ctgttgataa cccttctcta aaaataggtt tctgacaacg gttccctgtt
35941   tccagccatg ttccagcaat agccatccat ttggtgacag gaaatggcgc gcctgtccca
36001   caattgcctg caaatccgcc atgccatttt gtgcagcgat caatgcagtg gctggttcaa
36061   acctgatatc cccttcttgt agatgaggat cacgctcatc tatatacgga ggattgctga
36121   caatcatatc aaattgttgg ttacccactg ctgcaaacca ctcactttgc aaaaaattca
36181   cattgtgaat ggccagtttt ccggcgtttt tttcagcatt gtgttgtgcc agcatcacgg
36241   catcagagtt gatatcgacc cctgtcacat aacaatcatt ccgctcactt gccaatgcca
36301   gtgcaatcgc ccccgtcccc gtccccagat ccagaatccg ggctggagaa tcaggcaata
36361   attccaatgc cttctccacc agacattcag tatcagggcg cgggatcaac gtcgctggcg
36421   atacggcaaa cggcagtgac cagaattccc gttcaccaat aatataagct accggctctc
36481   cctgaatgcg gcgcaccagc aggctatcaa gctgatgcaa ttcttccgat gagattagcg
36541   tttcatcgaa agcaatcaga taagtacggg aacgccctgt cacgtatccc aacaagattt
36601   ccgcatcacg cttagggctg tcactttcag acaactgggt agccgcatgt tgtagccagc
36661   attggtaatt cattaatcct gctctgacag tgcagacagt tgatctgcct gatattcgat
36721   aatgatcggc tgaataagca tatccagttt gccttctatc acttcatcaa ggcggtataa
36781   cgtcagattg attcggtgat cagtcacacg cccctgtggg aagttatagg tgcggttgcg
36841   atctgagcgg tcaccagaac ccagcaaatt tcggcgttca gaggcttcca cttcttggcg
36901   cttccgcatc tcagcagcac ggatacgcgc tgccaatacg gacatcgctt ttgctttgtt
36961   tttgtgctgg gaacgctcat cctgacattc cactacgatc cccgttggga gatgggtaat
37021   tcgaatcgca gaatcggtgg tattgacgtg ctgcccaccc gcaccggaag agcgaaacgt
37081   atctattttc aaatcacccg ggctgatgtc cggtaattca gcttctggaa tttctggcat
37141   gacagccaca gtacaggcag aagtgtgaat gcgcccctga gattccgttt ccggtacacg
37201   ctggacacga tgaccgcctg attcaaattt caagtgacca taaacctgat cacccgaaac
37261   tttggcaatc acttctttgt agccaccatg ctcgccttcg ttggcgctta taatctctac
37321   tctccagcgg cgggcttccg catagcggct atacatgcgg aacaaatctc ccgcaaatat
37381   cgcggcttca tcgccaccgg ttcctgcccg gacttcaagg aaacagttgc gctcatcatc
37441   cggatctttc ggcaacagca gtagctgtag ctgctgttcc agctcttcat tacgaatttt
37501   tgcctccttg agctcttcct gcgccatttc ccgcatttcg acca
```

Fig. 2(viii)

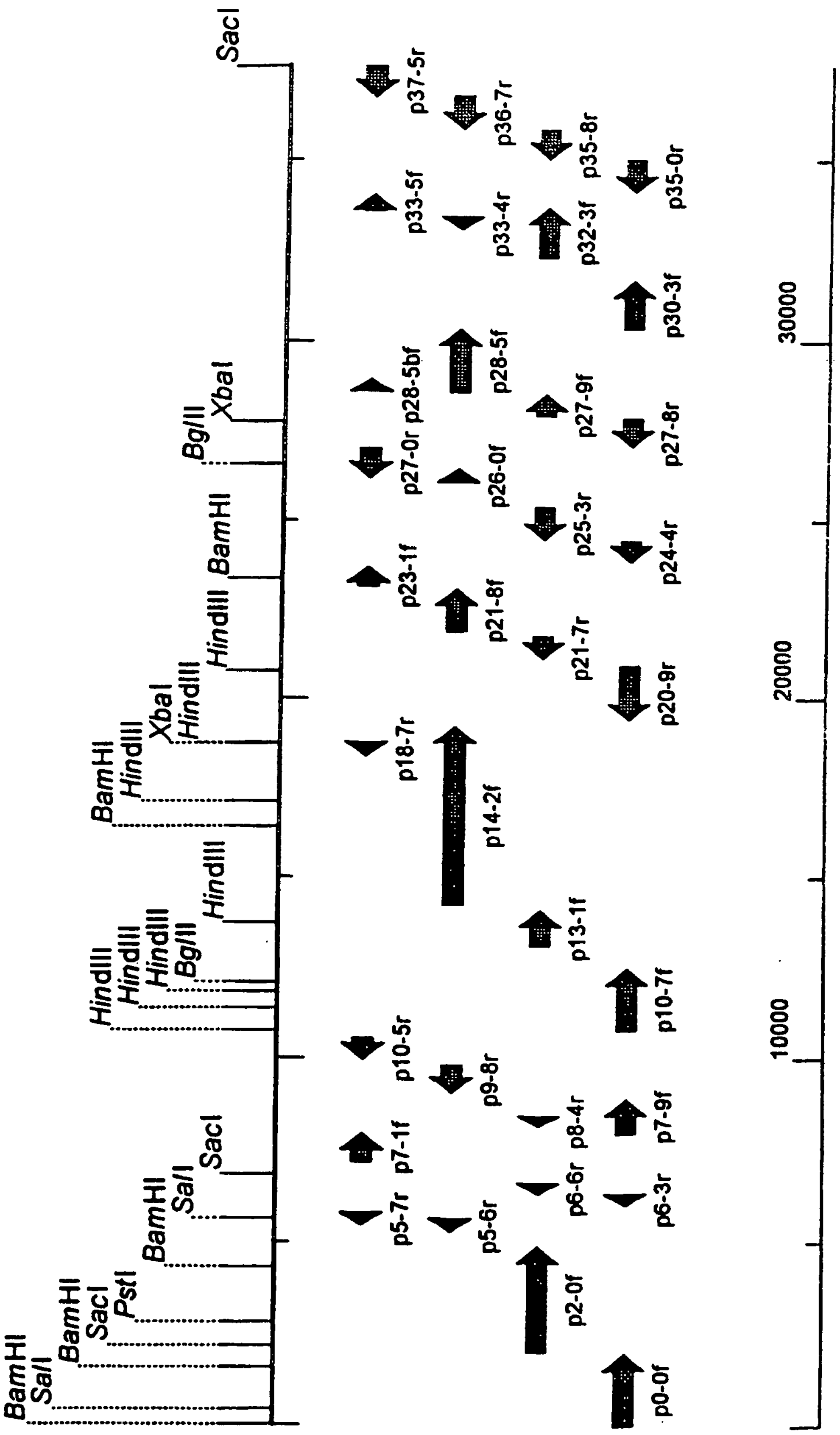


chrim5ed2.seq (37544 bps)

Fig. 3

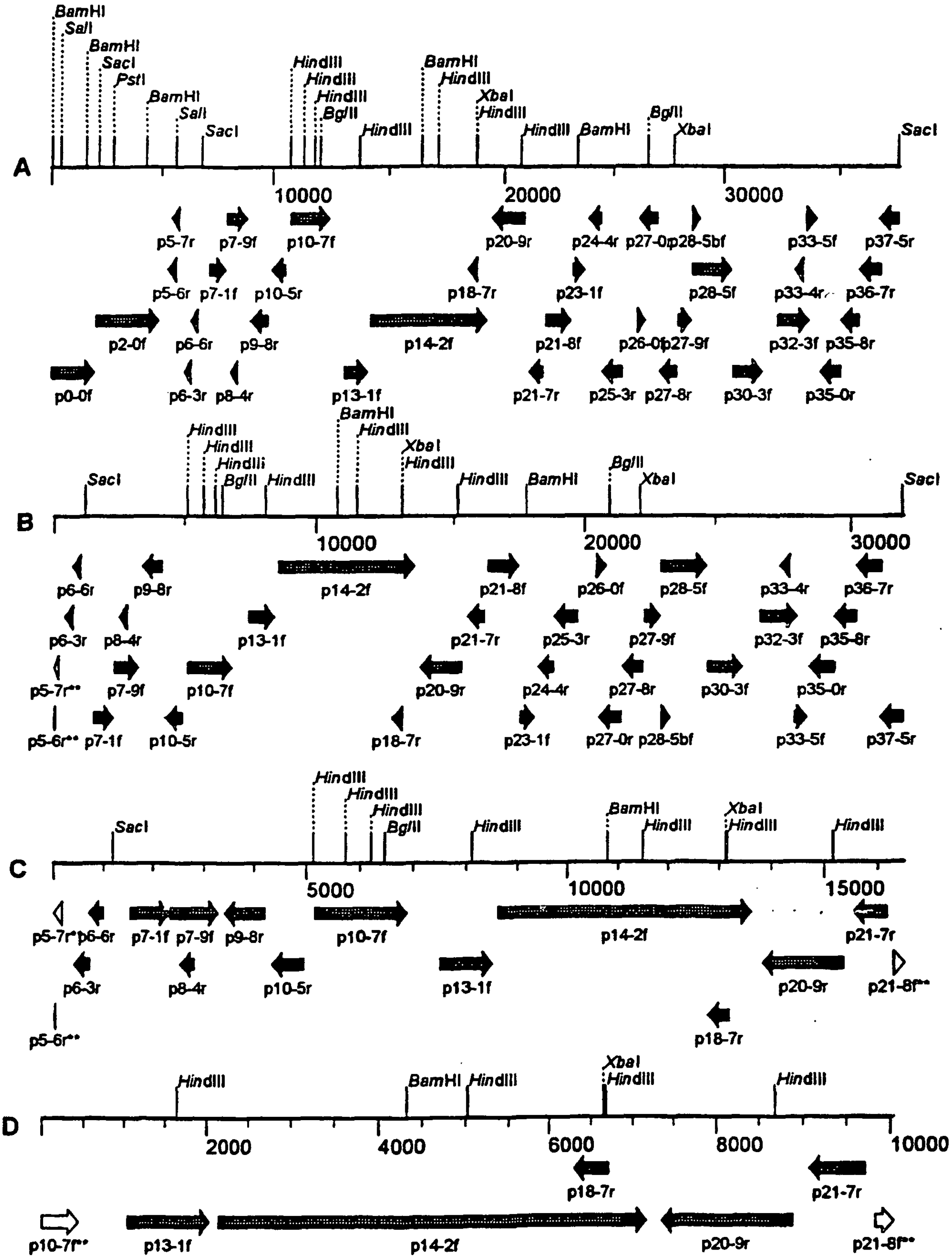
A
BamHI
SalI
BamHI
SacI
PstI
BamHI
SalI
SacI
HindIII
HindIII
HindIII
BglII
HindIII
BamHI
HindIII
XbaI
HindIII
HindIII
BamHI
BglII
XbaI
SacI
10000
20000
30000
p5-7r p7-9f p10-7f p20-9r p24-4r p27-0r p28-5bf p33-5f p37-5r
p5-6r p7-1f p10-5r p18-7r p23-1f p28-5f p33-4r p36-7r
p2-0f p6-6r p9-8r p14-2f p21-8f p26-0f p27-9f p32-3f p35-8r
p0-0f p6-3r p8-4r p13-1f p21-7r p25-3r p27-8r p30-3f p35-0r
B
SacI
HindIII
HindIII
HindIII
BglII
HindIII
BamHI
HindIII
XbaI
HindIII
HindIII
BamHI
BglII
XbaI
SacI
10000
20000
30000
p6-6r p9-8r p14-2f p21-8f p26-0f p28-5f p33-4r p36-7r
p6-3r p8-4r p13-1f p21-7r p25-3r p27-9f p32-3f p35-8r
p5-7r** p7-9f p10-7f p20-9r p24-4r p27-8r p30-3f p35-0r
p5-6r** p7-1f p10-5r p18-7r p23-1f p27-0r p28-5bf p33-5f p37-5r
C
SacI
HindIII
HindIII
HindIII
BglII
HindIII
BamHI
HindIII
XbaI
HindIII
HindIII
5000
10000
15000
p5-7r** p6-6r p7-1f p7-9f p9-8r p10-7f p14-2f p21-7r
p6-3r p8-4r p10-5r p13-1f p20-9r p21-8f**
p5-6r**
p18-7r
D
HindIII
BamHI
HindIII
XbaI
HindIII
HindIII
2000
4000
6000
8000
10000
p18-7r p21-7r
p10-7f** p13-1f p14-2f p20-9r p21-8f**

Fig. 4

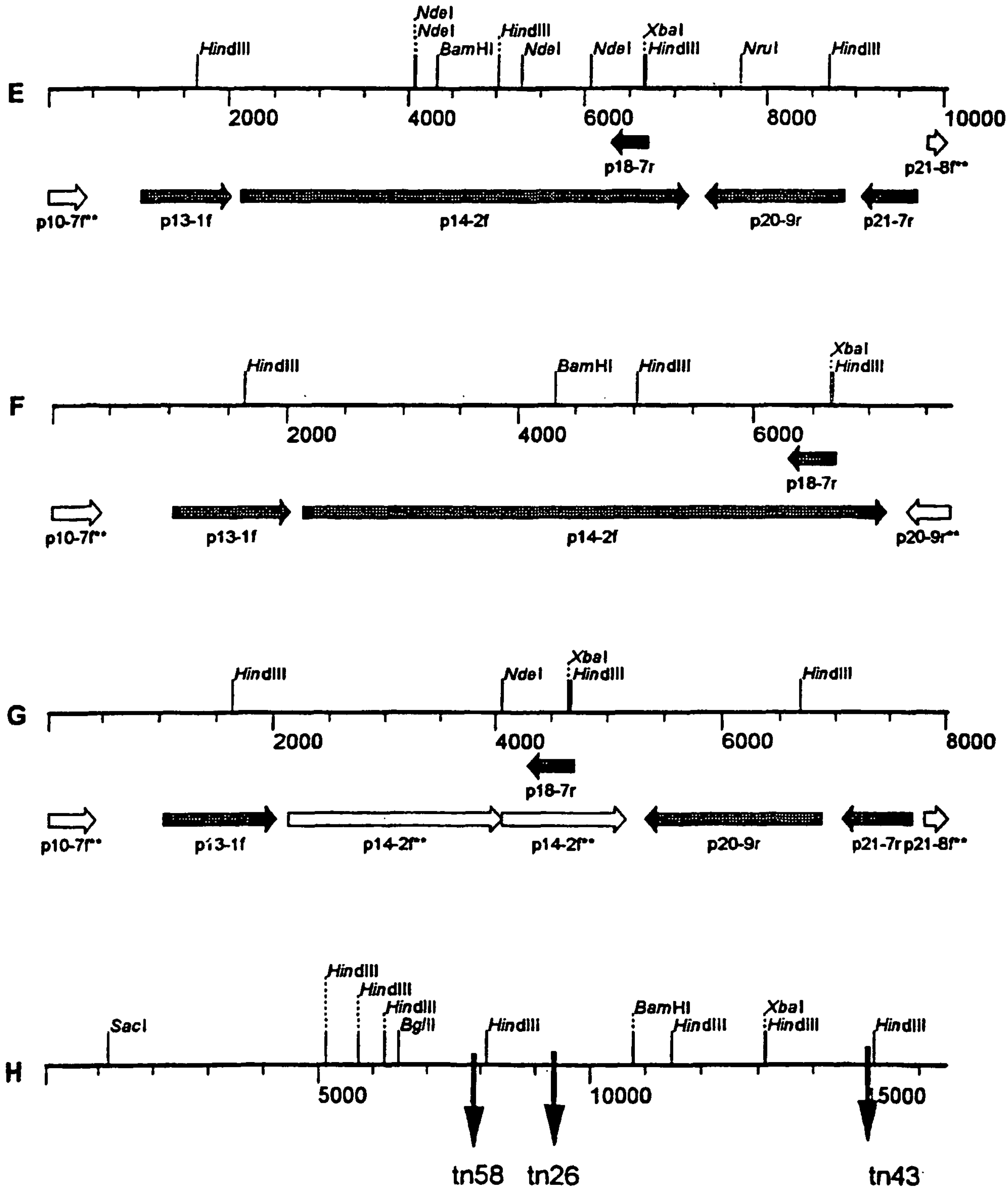
E
HindIII
NdeI
NdeI
BamHI
HindIII
NdeI
NdeI
XbaI
HindIII
NruI
HindIII
2000
4000
6000
8000
10000
p18-7r
p21-8f**
p10-7f**
p13-1f
p14-2f
p20-9r
p21-7r
F
HindIII
BamHI
HindIII
XbaI
HindIII
2000
4000
6000
p18-7r
p10-7f**
p13-1f
p14-2f
p20-9r**
G
HindIII
NdeI
XbaI
HindIII
HindIII
2000
4000
6000
8000
p18-7r
p10-7f**
p13-1f
p14-2f**
p14-2f**
p20-9r
p21-7r
p21-8f**
H
SacI
HindIII
HindIII
HindIII
BglII
HindIII
BamHI
HindIII
XbaI
HindIII
HindIII
5000
10000
15000
tn58
tn26
tn43

Fig. 4(cont'd)

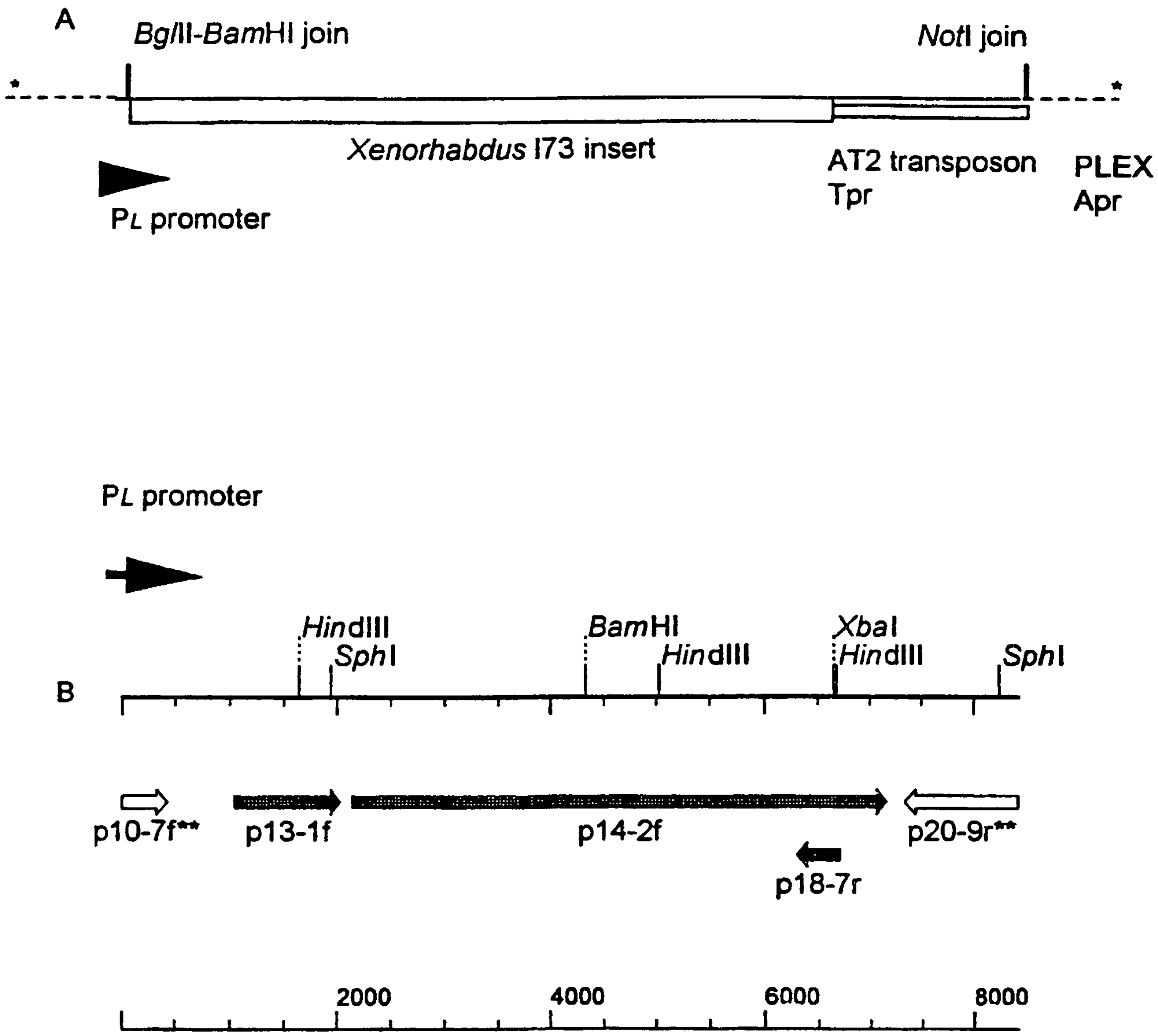
A
BglII-BamHI join
NotI join
*
*
Xenorhabdus I73 insert
AT2 transposon
Tpr
PLEX
Apr
PL promoter
PL promoter
HindIII
SphI
BamHI
HindIII
XbaI
HindIII
SphI
B
p10-7f**
p13-1f
p14-2f
p18-7r
p20-9r**
2000
4000
6000
8000

Fig. 5